(19)

Europäisches
Patentamt
European
Patent Office
Office européen
des brevets

(11) **EP 4 483 951 A1**

(12) **EUROPEAN PATENT APPLICATION**

(43) Date of publication:
**01.01.2025 Bulletin 2025/01**

(21) Application number: **23182874.0**

(22) Date of filing: **30.06.2023**

(51) International Patent Classification (IPC):
*A61P 11/00* [(2006.01)]     *C07K 16/40* [(2006.01)]

(52) Cooperative Patent Classification (CPC):
**C07K 16/40; A61K 39/39591; A61P 11/00;**
A61K 2039/505; A61K 2039/54; C07K 2299/00;
C07K 2317/22; C07K 2317/34; C07K 2317/569;
C07K 2317/76; C07K 2317/92; C07K 2317/94

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université de Liège**
  **4000 Liège (BE)**
• **Université Catholique de Louvain**
  **1348 Louvain-la-Neuve (BE)**
• **Vrije Universiteit Brussel**
  **1050 Brussel (BE)**

(72) Inventors:
• **DUMOULIN, Mireille**
  **4000 Liège (BE)**
• **MORALES YÀNEZ, Francisco**
  **4000 Liège (BE)**

• **REDEGHIERI, Paola**
  **4000 Liège (BE)**
• **VANBEVER, Rita**
  **1200 Woluwe-Saint-Lamberg (BE)**
• **VANDERSTRAETEN, Jessica**
  **1200 Woluwe-Saint-Lambert (BE)**
• **LEAL, Teresinha**
  **1200 Woluwe-Saint-Lambert (BE)**
• **MOTTAIS, Angélique**
  **1200 Woluwe-Saint-Lambert (BE)**
• **MUYLDERMANS, Serge**
  **1050 Brussel (BE)**
• **VINCKE, Cécile**
  **1050 Brussel (BE)**

(74) Representative: **De Clercq & Partners**
**Edgard Gevaertdreef 10a**
**9830 Sint-Martens-Latem (BE)**

Remarks:
The complete document including Reference
Table(s) and the Sequence Listing(s) can be
downloaded from the EPO website

(54) **SINGLE-DOMAIN ANTIBODY FOR INHIBITION OF NEUTROPHIL ELASTASE ACTIVITY**

(57)    The present invention relates to a binding agent capable of specifically binding to and inhibiting competitively a neutrophil elastase, which binding agent comprises an immunoglobulin single variable domain (ISVD), as well as related products, methods, and uses, such as in particular methods and uses aimed at the prevention, treatment, or diagnostics of inflammatory diseases.

EP 4 483 951 A1

**Description**

**FIELD**

The invention is broadly situated in the medical field and particularly relates to agents, more specifically binding agents, and pharmaceutical formulations suitable for inhibition of neutrophil elastase (NE) activity or for the detection/-quantification of active NE.

**BACKGROUND**

Neutrophil elastase (NE) is a protease that plays an important role in the immune response and host defence mechanisms in both physiological and disease-associated conditions. NE is stored in azurophilic granules of polymorphonuclear nucleophiles and can be released during neutrophil degranulation and neutrophil extracellular traps release (NETosis). Under physiological conditions, the amount of extracellular NE is tightly regulated, mainly by the antiproteases AAT (alpha1-antitrypsin) and Elafin. However, in a number of pathological conditions, this balance is displaced towards exceeding extracellular NE levels, which are associated with progressive destruction of tissues and loss of their associated functions and impaired immune system regulation. Multiple types of inhibitors, including protein-based inhibitors and small molecule inhibitors, are known to inhibit NE activity. Regarding protein-based inhibitors, an inhaled version of AAT, an endogenous glycoprotein inhibitor of neutrophil elastase, has been tested for the treatment of respiratory pathologies. However, the effectiveness of inhaled AAT in reducing inflammatory parameters and improving pulmonary function is significantly limited. While it does not decrease the frequency of infectious exacerbations, it mitigates their severity. The need for administering high doses of inhaled AAT reflects the inadequate potency of the protein (Amin and Ratjen, Expert Opin Emerg Drugs 2014, vol. 19(1), 143-55). This inefficiency may be attributed to the rapid degradation of AAT in pulmonary tissue, as the glycoprotein is highly unstable in biological fluids due to its susceptibility to proteolysis and oxidation (Suter et al., Eur Respir J. 1991, vol. 4(1), 40-9). Moreover, the utilization of nebulizers for pulmonary administration to accommodate the high AAT doses necessitates extended periods of manipulation and inhalation, further adding to the therapy burden for patients. Additional drawbacks associated with AAT are its elevated cost and limited production capacity, as it is derived from human plasma through purification processes. Furthermore, small molecule inhibitors of NE have been studied extensively. However, only few of these molecules, such as Sivelestat, have resulted in an approved drug in the market for a limited number of conditions, partially due to significant organ toxicity. Challenges in the development of small molecules include inefficient efficacy in inhibiting elastase, a lack of specificity towards elastase, and inadequate metabolic stability *in vivo.* Therefore, there is a compelling demand from patients with significant unmet clinical needs for the development of new, potent agents that effectively inhibit NE.

**SUMMARY**

The present invention is at least in part based on the identification of immunoglobulin single variable domain (ISVD) binding agents which bind to and efficiently inhibit neutrophil elastase (NE). In particular, as evidenced in the experimental section of this specification the NbE201 ISVD, which illustrates an embodiment of the present invention, is a competitive inhibitor of NE and demonstrates a high affinity, a high specificity and a high inhibitory capacity of NE. Furthermore, the ISVD displays a high stability and long halftime in the lung, especially when PEGylated. This opens an avenue for diagnostic, prognostic, and therapeutic applications with the ISVDs disclosed herein.

In view of these advantages, an aspect of the invention provides a binding agent capable of specifically binding to and inhibiting a neutrophil elastase (NE), wherein the binding agent comprises an immunoglobulin single variable domain (ISVD) and the binding agent competes with Elastase Inhibitor 3 (EI3) for binding to NE.

Another aspect of the invention provides a binding agent capable of specifically binding to and inhibiting a neutrophil elastase (NE), wherein the binding agent comprises an immunoglobulin single variable domain (ISVD) binding to at least one of the residues R36, A60, N61,P96, V97, S195, G218 and G219 of the NE, preferably two or more of the residues R36, A60, N61,P96, V97, S195, G218 and G219, more preferably three or more of the residues R36, A60, N61, P96, V97, S195, G218 and G219, more preferably all of the residues R36, A60, N61, P96, V97, S195, G218 and G219 of the NE, wherein the residues are annotated according to the chymotrypsinogen scheme. In certain embodiments, the ISVD binds to at least the residues R36, A60, N61,P96, V97 and S195 of the NE.

Another aspect of the invention provides a binding agent capable of specifically binding to and inhibiting a neutrophil elastase (NE), wherein the binding agent comprises an ISVD comprising a complementarity determining region 1 (CDR1) having the sequence set forth in SEQ ID NO:1 (GRTISLYR), a CDR2 having the sequence set forth in SEQ ID NO:2 (INWSGDMT) and a CDR3 having the sequence set forth in SEQ ID NO:3 (TADPKLLPLADSSYGY).

A further aspect provides a binding agent capable of specifically binding to and inhibiting a NE, wherein the binding agent comprises an ISVD comprising a CDR1, CDR2 and CDR3, each as present in SEQ ID NO: 4 wherein the CDR1, CDR2 and CDR3 are annotated according to any one of the IMGT, Kabat, Chlotia, Martin or AHo numbering systems.

A related aspect provides a nucleic acid molecule comprising a polynucleotide sequence encoding the binding agent mentioned above, or a vector comprising the nucleic acid molecule.

A related aspect provides a cell or either a virus comprising the nucleic acid molecule or a vector optionally wherein the cell is capable of expressing or it expresses the binding agent or the virus is configured to induce expression of the binding agent in a recipient cell infected with the virus.

A related aspect provides a pharmaceutical composition comprising the binding agent or the nucleic acid molecule or vector or the cell or virus, and a pharmaceutically acceptable carrier and/or diluent and/or excipient, optionally wherein the pharmaceutical composition comprises a further inhibitor of the NE.

An additional aspect provides a kit such as a diagnostic kit comprising the binding agent or the nucleic acid molecule or vector or the cell or virus. Another additional aspect provides a method for determining the level of active neutrophil elastase in a specimen, comprising contacting the specimen with the binding agent, and detecting at least the neutrophil elastase bound to the binding agent.

A further aspect provides the binding agent, nucleic acid molecule, vector, cell, virus, or pharmaceutical composition as taught above for use in medicine.

Another aspect provides the binding agent, nucleic acid molecule, vector, cell, virus, or pharmaceutical composition as taught above for use in the prevention or treatment of an inflammatory disease.

A related aspect provides a method for treating an inflammatory disease in a subject in need thereof, comprising administering to the subject an effective amount of the binding agent, nucleic acid molecule, vector, cell, virus, or pharmaceutical composition as taught above.

The present binding agents can also be employed for the detection of active NE, and a further aspect provides a method for determining the level of active neutrophil elastase in a specimen, comprising contacting the specimen with the binding agent as taught above and detecting at least the neutrophil elastase bound to the binding agent.

A further aspect provides a method for the diagnosis, prognosis and/or monitoring of an inflammatory disease in a subject, comprising contacting a biological sample obtained from the subject with the binding agent as taught above, and determining the level and/or activity of neutrophil elastase in the sample by detecting at least the neutrophil elastase bound to the binding agent.

These and further aspects and preferred embodiments of the invention are described in the following sections and in the appended claims. The subject-matter of the appended claims is hereby specifically incorporated in this specification.

**BRIEF DESCRIPTION OF DRAWINGS**

The following description of the figures of specific embodiments of the invention is merely exemplary in nature and is not intended to limit the present teachings, their application or uses.

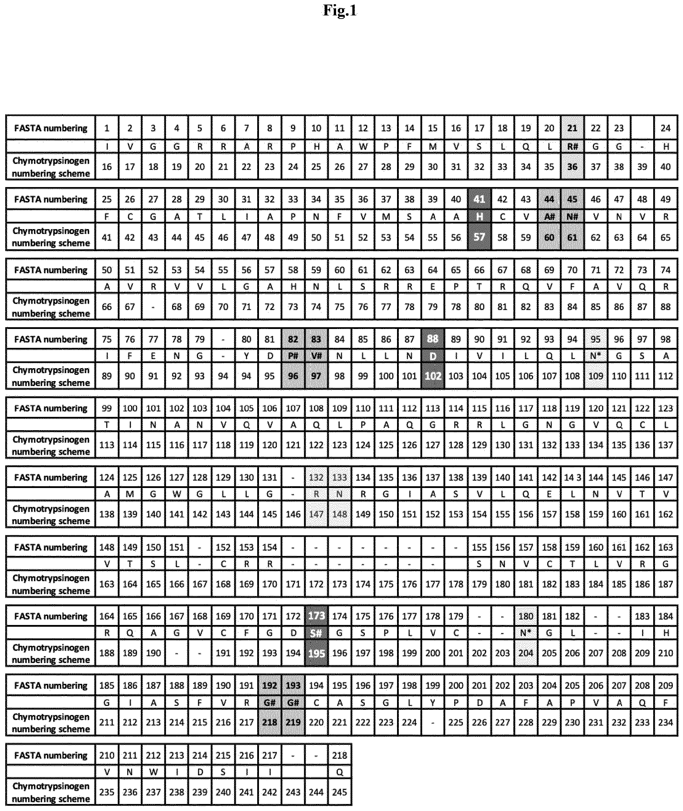

**Figure 1.** Sequence of mature hNE in a FASTA scheme matched to the chymotrypsinogen numbering scheme. The residues from the catalytic triad (H57, D102 and S195) are shown in white letters with dark grey background. Asterisks demonstrate the sites of glycosylation (N* 109 and N*204). The residues labelled in light grey letters and background correspond to the gap in the crystal structure due to low resolution (R147-N148). The amino acids implicated in the binding to the Nb E201 are R36, A60, N61, P96, V97, S195, G218 and G219 are marked with a hash symbol. The binding to S195 occurs via a molecule of water.

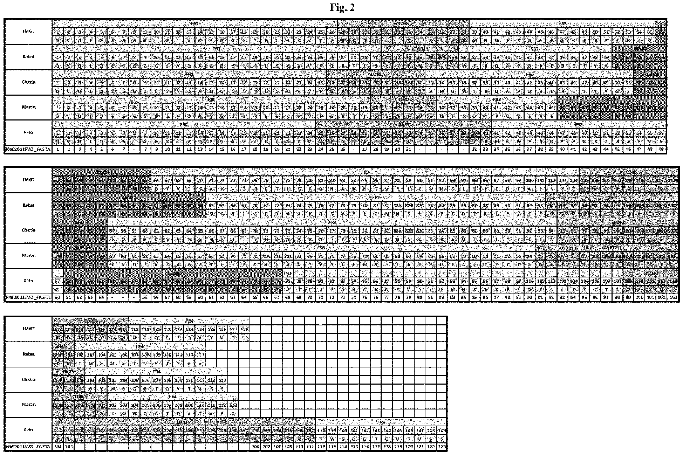

**Figure 2.** Sequence of ISVD NbE201 (SEQ ID NO: 4) showing CDR1, CDR2 and CDR3 sequences annotated according to IMGT, Kabat, Chlotia, Martin or AHo numbering systems.

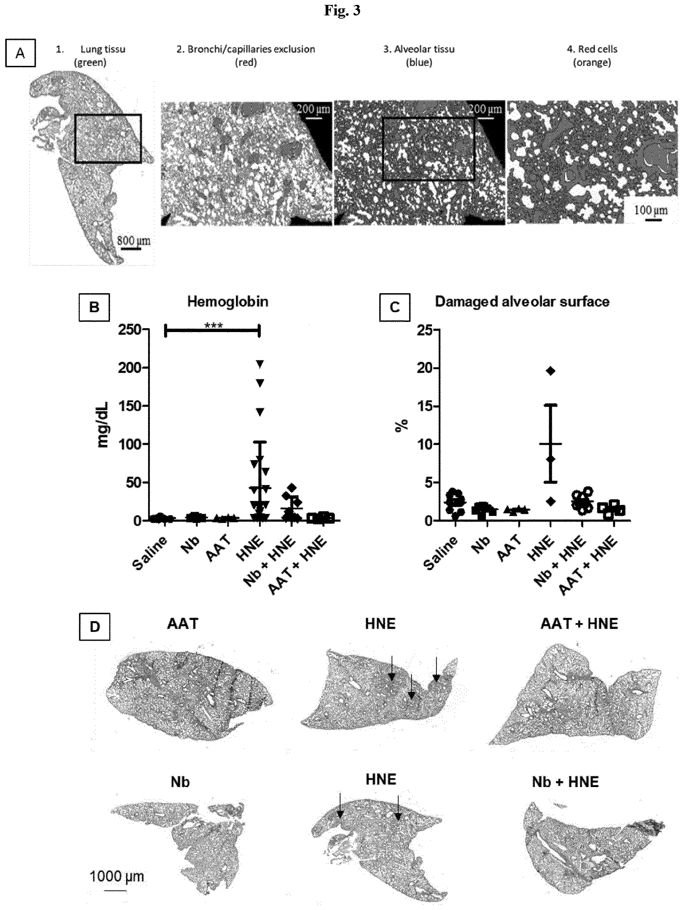

Figure 3. A) Quantification of the percentage of surface with alveolar-capillary lesions after intranasal instillation of hNE. Digitization of all histological sections of lung stained with hematoxylin/eosin, the images were analyzed with Visiopharm® software. Four steps were performed: 1) delineation of lung tissue (black); 2) exclusion of the bronchial and capillary analysis area; 3) delineation of alveolar tissue (grey) and alveoli (white); and 4) delineation of injured areas (arrow). Protective activity of nanobodies against the development of HNE-induced acute lung injury syndrome in mouse model. Acute lung injury syndrome is identified by B) the presence of hemoglobin in bronchoalveolar lavage, C and D) and the presence of red blood cells in alveolar surface (black arrow). Data are the mean ±standard deviation (SD), n=3-12 mouse/group. AAT: alpha anti-trypsin; HNE: human neutrophil elastase; Nb: nanobody

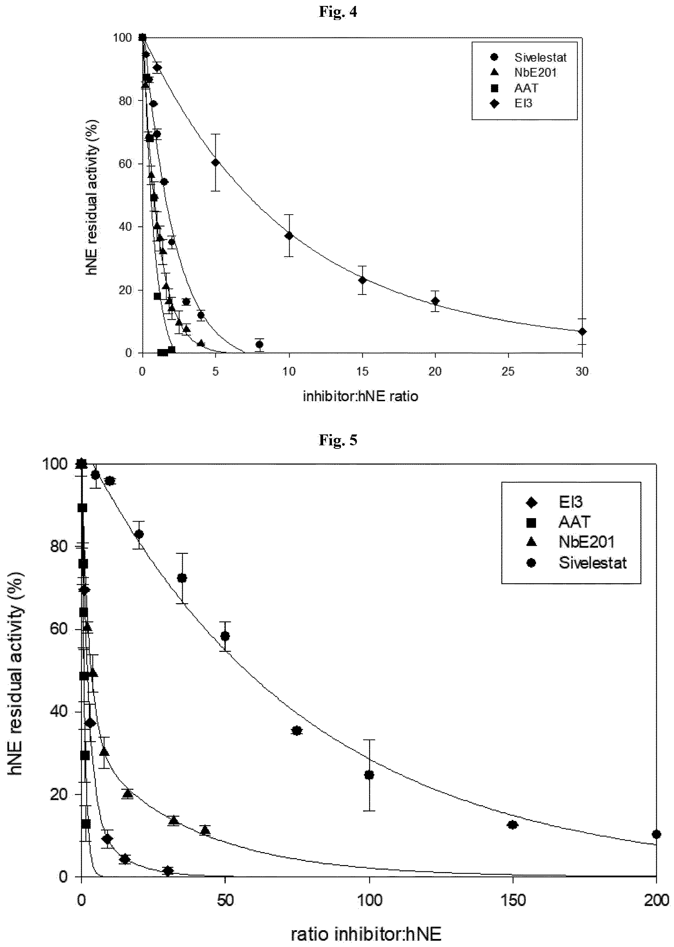

**Figure 4.** Curve of residual hNE activity (in percentage) at different [inhibitor]/[hNE] ratios for the hydrolysis of the small chromogenic substrate N-Succinyl-Ala-Ala-Ala-pNA (1 mM). The following inhibitors are tested: (▲) hNE inhibition by NbE201 (N=3), (■) hNE inhibition by AAT (N=1), (♦) hNE inhibition by EI3 (= elastase inhibitor 3 or MeOSuc-AAPV-CMK) (N=2) and (●) hNE inhibition by Sivelestat (N=2). The concentration of hNE is 50 nM. The displayed points are the average of the independent measurements and the error bars show the SD. The data are fitted using a single exponential equation using SigmaPlot for visual purpose.

**Figure 5.** Curve of residual hNE activity (in percentage) at different [inhibitor]/[hNE] ratios for elastin hydrolysis. (■) hNE inhibition by AAT (N=3), (♦) hNE inhibition by EI3 (N=2), (▲) hNE inhibition by NbE201 (N=3) and (●) hNE inhibition by Sivelestat (N=2). The displayed points are the average of the independent measurements and the error bars correspond to the standard deviation.

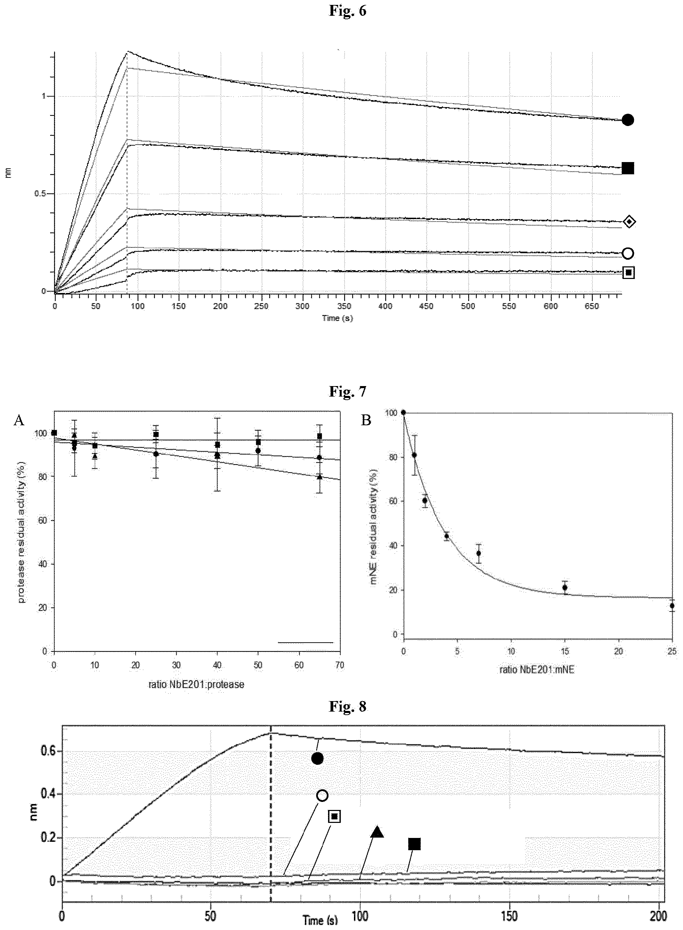

**Figure 6.** Representative sensorgrams of the measurement of affinity by BLI. From 0 to 90 seconds: association step; above 90 seconds: dissociation step. The kinetics of association and dissociation between hNE at different concentrations (analyte), and NbE201 immobilized on the sensor (ligand) are shown. (●) hNE 40 nM, (■) hNE 20 nM, (⬥) hNE 10 nM, (O) hNE 5 nM and (▣), hNE 2.5 nM. The black line represents the measured data ($\Delta\lambda$). The grey line represents the fitting of the data used to calculate the $K_D$ values. Data were fitted accordingly to a 1:1 ligand model by using the Data Analysis Octet Software Version 10.0 (Sartorius). Independent replicates of the experiment were done (N=5) and the mean ($\pm$ st. dev.) of the kinetics parameters measured and indicated in the text.

**Figure 7.** Residual activity of several serine proteases in the presence of different concentrations of NbE201. A) The proteases tested are human proteinase 3 (hPR3, ▲), human cathepsin G (hCG, ■) and porcine pancreatic elastase (pPE, ●). B) Murine elastase (mNE, ●). The experiments were carried out in triplicates (N=3). The displayed points are the average of the independent measurements and the error bars show the SD.

**Figure 8.** Sensorgrams of the association and dissociation steps of the interaction between NbE201 and different serine proteases. From 0 to 70 s: association step; above 70 s: dissociation step. hNE ((●)), hCG (■, 36.7% sequence identity with hNE), hPR3 (▲, 54.7% sequence identity with hNE), mPE (▣, 36.8 % sequence identity) and pPE (O, 39.2% sequence identity with hNE).

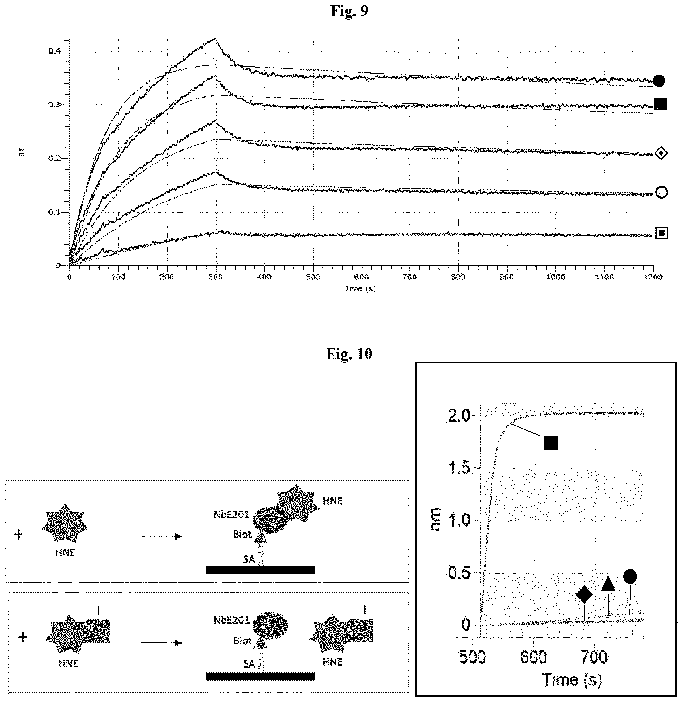

**Figure 9.** Representative sensorgrams of the association and dissociation steps of the interaction between NbE201 and different concentrations of mNE. From 0 to 300 s: association step; above 300 s: dissociation step. In black, the experimental signal. In grey, the fitting using a 1:1 model. (●) mNE 100 nM, (■) mNE 70 nM, (⬥) mNE 50 nM, (O) mNE 30 nM and (▣), mNE 10 nM. Data were fitted accordingly to a 1:1 ligand model by using the Data Analysis Octet Software Version 10.0 (Sartorius). Independent replicates of the experiment were done (N=3) and the mean ($\pm$ SD.) of the kinetics parameters measured and indicated in the text.

**Figure 10.** Competitive binding between NbE201 and hNE complexed with a series of known inhibitors monitored by BLI. Left upper panel: free hNE is able to bind to NbE201 immobilised on the BLI sensor; left lower panel: hNE in complex with a competitive inhibitor is not able to bind to NbE201 immobilised on the BLI sensor. The competitive inhibitors tested are (i) Nb-H7S-Nter-P, hNE inhibitor III (MeOSuc-Ala-Ala-Pro-Val-CMK) and (ii) AAT. A control experiment with only hNE was carried out. After a hydration step, the biotinylated ISVD NbE201 (10 $\mu$g·mL$^{-1}$) was first immobilized onto streptavidin (SA) sensors. In the association step (Left panel), the sensors were immersed into a solution of hNE (250 nM) or hNE complexed with AAT, with Nb-H7S-Nter-P1 or with hNE inhibitor III (I). Right panel, sensorgrams recorded with hNE alone (■) and in the presence of either AAT(▲), hNE inhibitor III (♦) or Nb-H7S-Nter-P1 (●).

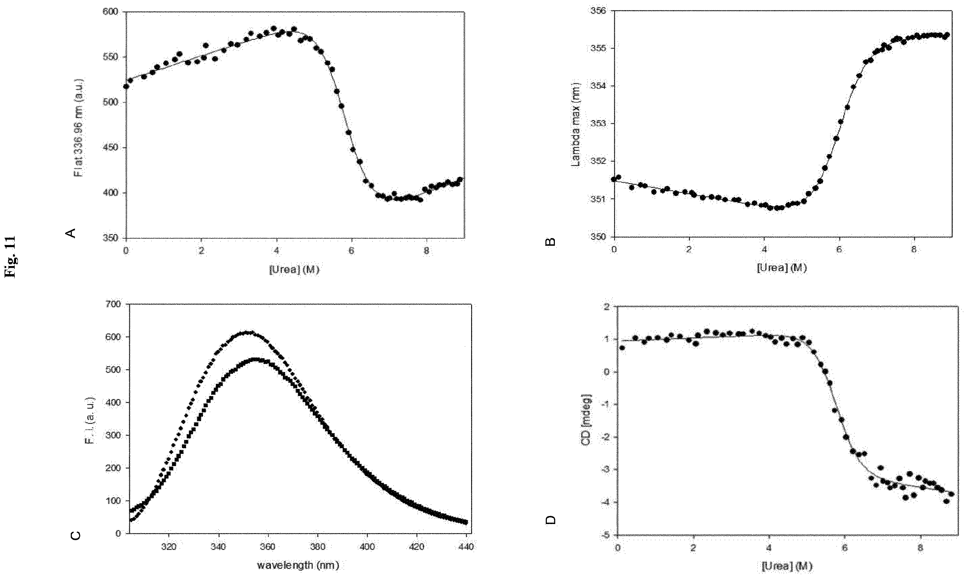

**Figure 11.** Urea-induced unfolding transition of NbE201 monitored by intrinsic fluorescence and far Ultra Violet-circular dichroism (far UV-CD). A) Transition monitored the change in intensity at 337 nm. B) Transition monitored by the change in the wavelength of maximal intensity ($\lambda_{max}$). The solid line represents the best fit calculated using equation 1. C) Intrinsic fluorescence spectrum of the native (♦) and denatured (■) state of hNE. D) Transition monitored by far UV-CD.

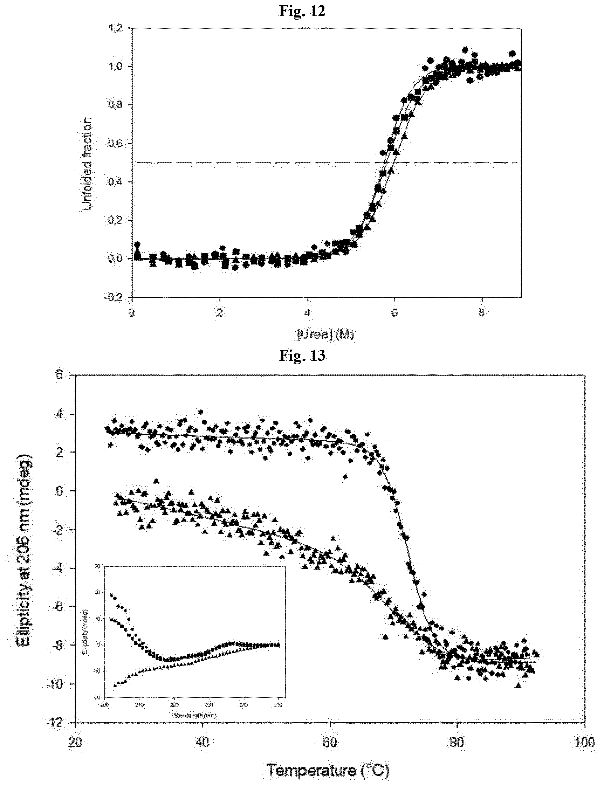

**Figure 12.** Urea-induced unfolding transition of NbE201 normalised using equation 2. (●) unfolded fraction curve relative to the CD signal, (▲) the unfolded fraction curve relative to the lambda max signal, and (■) the unfolded fraction curve relative to the 337 nm signal.

**Figure 13.** Example of the denaturation and renaturation curve for NbE201 monitored by far UV-CD at 206 nm. (■) curve for the denaturation transition from 25 to 97°C, (▲) curve for the renaturation transition from 97°C to 25°C. In the insert there are the spectra of NbE201 in far-UV CD (190-250 nm). (●) the spectrum of NbE201 at 25°C (▲) the spectrum of NbE201 at 90°C and (■) the spectrum ofNbE201 at 25°C after denaturation/renaturation.

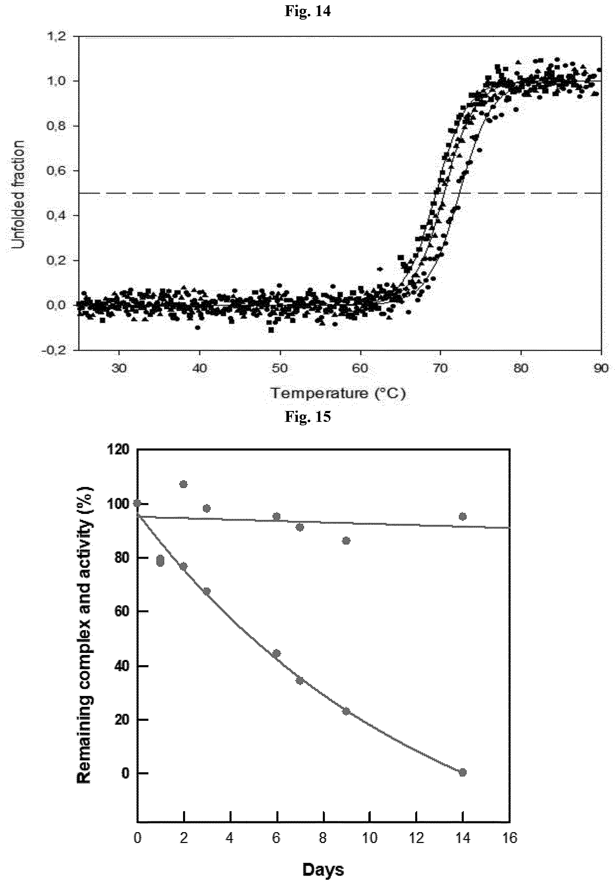

**Figure 14.** Heat-induced unfolding transition of NbE201 monitored by the changes far UV-CD at 206 nm and normalised using the equation 2. Each symbol indicates the transition for a different production lot: (■) production lot 18032021, (▲) production lot 07052021 and (●) production lot 26022021, for a total of N=3 replicates.

**Figure 15.** Stability of the complexNbE201+hNE overtime. The integrity ofNbE201 was monitored during 14 days and it was analyzed by SDS-PAGE (upper curve). The lower curve shows the remaining activity of hNE monitored over the 14 days.

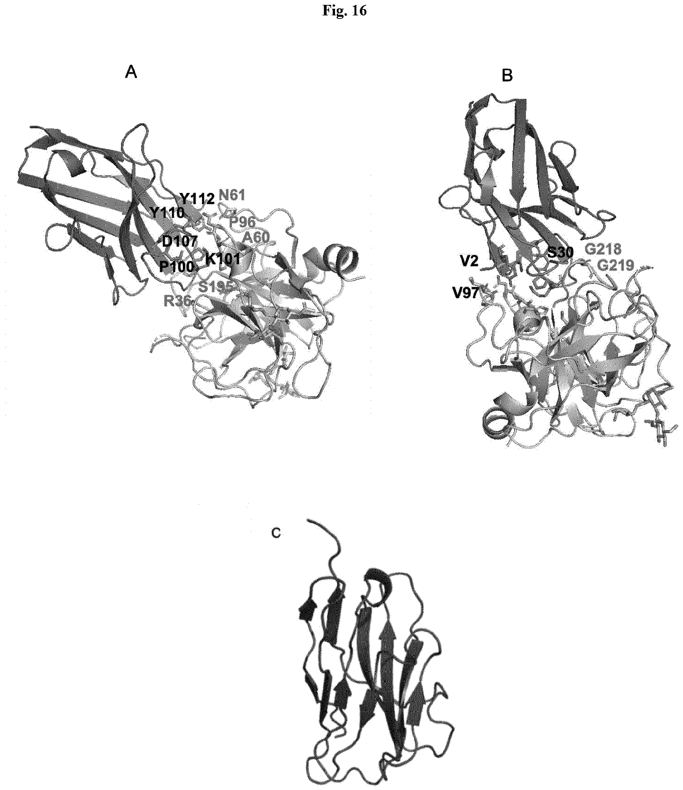

**Figure 16.** 3D structure of the complex NbE201+hNE solved by X-rays crystallography. A) Representation of the NbE201/hNE complex showing the amino acids P100, K101, D107, Y110 and Y112 from CDR3 of NbE201 (black letters) in contact with the hNE (A60, R36, N61 and P96 in light grey letters). B) polar interactions via the CDR1 (S30 in black) with the hNE (G218 and G219 in light grey letters). The hydrophobic interactions of V2 of NbE201 and V97 of the hNE are represented in black. The figures are made with PyMol software. Residues of hNE are labelled following the chymotrypsinogen scheme (Hartley, B.S. (1970), doi: 10.1098/rstb.1970.0010. C) Ribbon representation of the structure ofNbE201 alone. The figures are made with PyMol software.

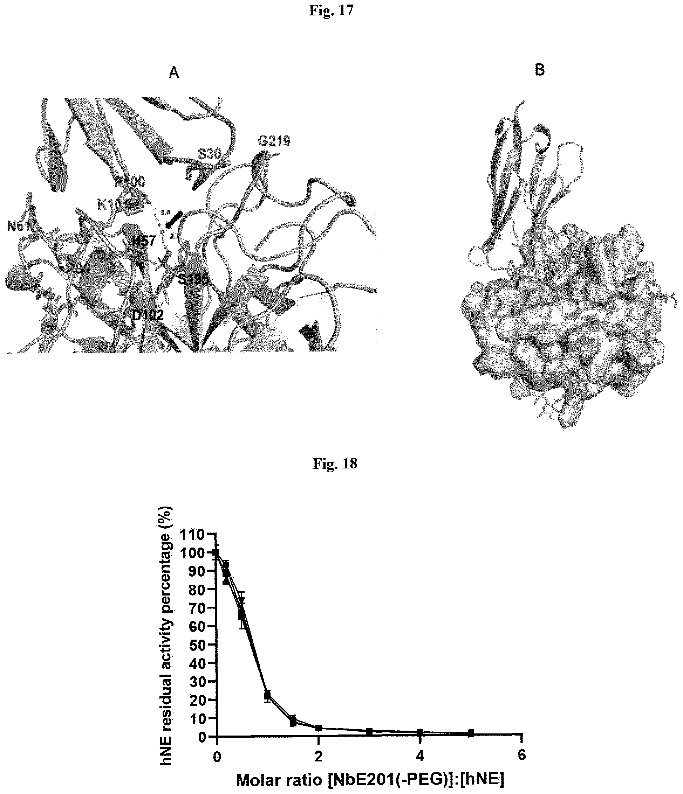

**Figure 17.** 3D structure of the complex NbE201+hNE solved by X-rays. A) Representation of the site of interaction between hNE and NbE201 CDRs. CDR1, CDR2, and CDR3 are shown in dark grey. The catalytic triad (H57, D102, S195) is shown in dark grey. B) Surface representation of the hNE showing that the CDR3 of NbE201 penetrates the cleft where the active site of hNE is located. The residue S195 from the catalytic triad is in contact with the residue P100 of the CDR3 of NbE201 via a water molecule (indicated by an arrow).

**Figure 18.** hNE inhibition by PEGylated and non-PEGylated NbE201. Suc-Ala-Ala-Pro-Val-pNA degradation by purified hNE (0.169μM) was followed by absorbance measurements during one hour in the presence or in the absence of PEGylated (linear PEG10 and PEG20 or branched PEG40) or non-PEGylated NbE201. hNE: Nb molar ratio = 1:0.25, 1:0.5, 1:1, 1:1.5, 1:2, 1:3, 1:4, 1:5. N=3, n=3. Mean ± SD.
(●) NbE201, NbE201-PEG10 (■), NbE201-PEG20 (▲) and NbE201-PEG40 (▼).

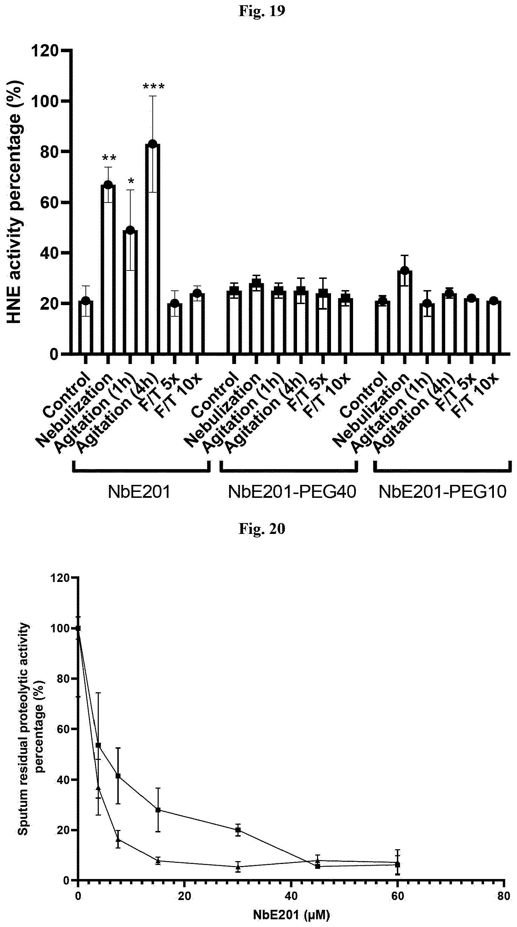

**Figure 19.** hNE residual activity in the presence ofNbE201(-PEG10 or 40) before (control) or after exposure of the ISVD to nebulization, one or four hours of magnetic agitation or 5 or 10 freeze/thaw (F/T) cycles. Suc-Ala-Ala-Pro-Val-pNA degradation by purified hNE was followed by absorbance measurements during one hour in presence of PEGylated (PEG10 or 40) or non-PEGylated NbE201. hNE: Nb molar ratio = 1:1. N=3, n=3 (NbE201 wt and NbE201-PEG40). N=2, n=3 (NbE201-PEG10). Mean ± SD. Statistical test: one-way ANOVA followed by a Dunnett post-hoc test, compared to the control group. *Pvalue <0.05, **p value<0.01, ****p value <0.001. The experiment was conducted twice with the NbE201-PEG10 and three times with the NbE201 and the NbE201-PEG40, with 3 technical replicates.

**Figure 20.** Sputum proteolytic activity inhibition by NbE201: inhibition curve. Suc-Ala-Ala-Pro-Val-pNA degradation by 20mg of a purulent (■) and a muco-purulent (▲) expectoration from patients with cystic fibrosis was followed by absorbance measurements during one hour in the presence or in the absence of non-PEGylated NbE201. N=3, n=3. One representative experiment from a serie of 3 Mean of 3 technical replicates ± SD.

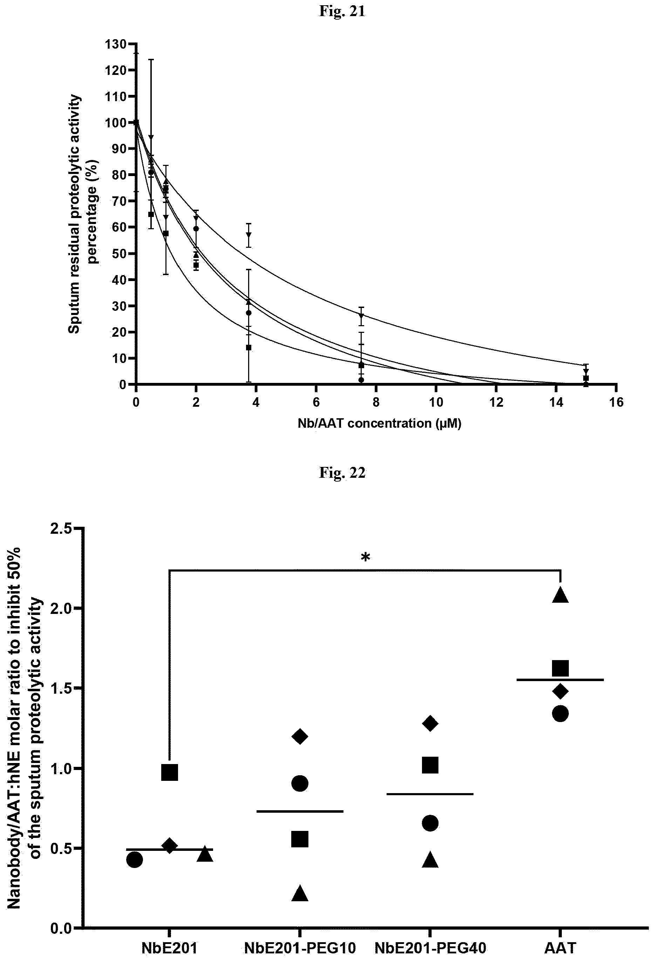

**Figure 21.** Sputum proteolytic activity inhibition by NbE201-(PEG10 or 40) or AAT: inhibition curve. Suc-Ala-Ala-Pro-Val-pNA degradation by 15 mg of an expectoration from a patient with cystic fibrosis was followed by absorbance measurements during one hour in presence or absence of increasing concentration of non-PEGylated NbE201(■), NbE201-PEG10 (■), NbE201-PEG40(▲) or AAT(▼. N=3, n=3. One representative experiment from a series of 3. Mean of 3 technical replicates ± SD. Statistical test: one-way ANOVA followed by a Tukey post-hoc test.

**Figure 22.** Nanobody/AAT : hNE molar ratio to inhibit 50% of the sputum proteolytic activity. Nonlinear regression curves were fitted via the use of GraphPad Prism 9.1.2 to interpolate concentrations of Nb/AAT to inhibit 50% of the activity of 10 to 15mg of cystic fibrosis sputa. Elastase activity in the different sputa was estimated via a standard curve with purified hNE to assess the Nb/AAThNE molar ratio in the sputa. N=4, n=3, where N represents the number of experiments and n represents the number of replicates. The different symbols of the individual data in the graph correspond to different sputa (one symbol by sputum). Statistical test: one-way ANOVA on paired data followed by a Dunnett post-hoc test, compared to the NbE201. *P value <0.05

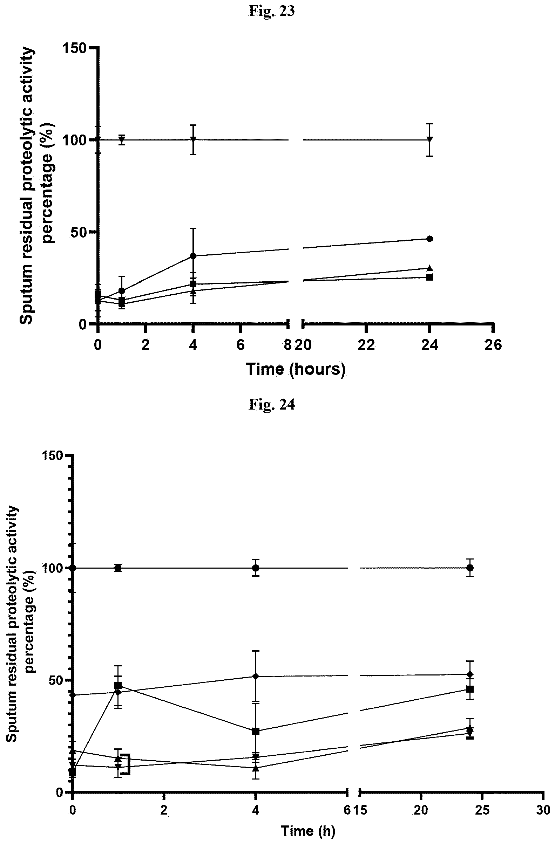

**Figure 23.** Sputum proteolytic activity inhibition by PEGylated and non-PEGylated NbE201 from 0 to 24h. Suc-Ala-Ala-Pro-Val-pNA degradation by 20 mg of expectoration from a patient with cystic fibrosis was followed by absorbance measurements during one hour after exposure to $7.5\mu g$ (0.52 nmol) of PEGylated (linear PEG10 or branched PEG40) or non-PEGylated NbE201 during 0, 1, 4 or 24 hours. Control condition: unrestricted elastase activity in the sputum. N=3, n=3. Mean of 3 technical replicates $\pm$ SD. One representative experiment. N represents the number of experiments; n represents the number of replicates. (▼) control, (■) NbE201-PEG10, (■) NbE201, and (▲) NbE201-PEG40.

**Figure 24:** Sputum proteolytic activity inhibition by AAT, PEGylated and non-PEGylated NbE201 from 0 to 24h. Suc-Ala-Ala-Pro-Val-pNA degradation 15 mg of expectoration from a cystic fibrosis (CF) patient was followed by absorbance measurements during one hour after exposure to 0.56 nmol of PEGylated (linear PEG10 or branched PEG40), wild type NbE201 or AAT during 0, 1, 4, or 24 hours. N=2, n=3. Mean of 3 technical replicates $\pm$ SD. One representative experiment. (●)control, (▲)NbE201-PEG10 (■)NbE201, (▼)NbE201-PEG40, and (♦)AAT. N represents the number of experiments; n represents the number of replicates.

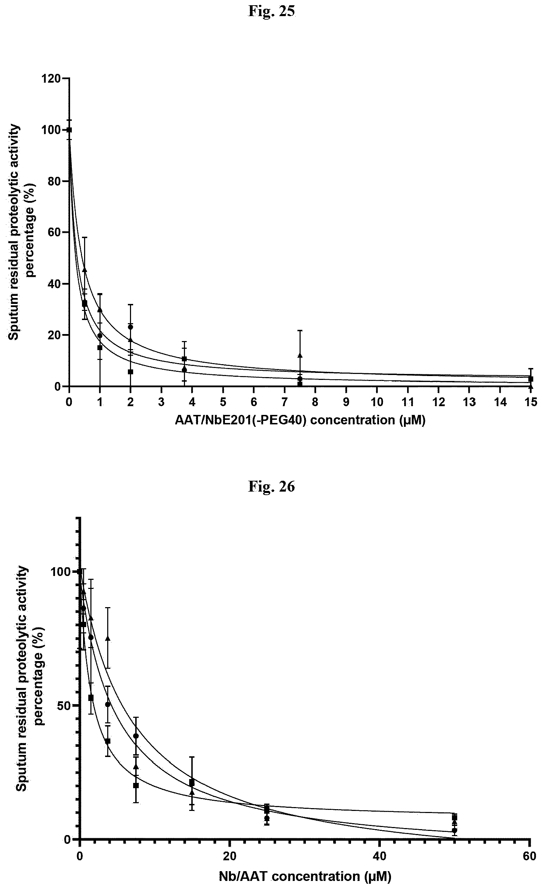

**Figure 25.** COPD (Chronic obstructive pulmonary disease) sputum proteolytic activity inhibition by NbE201(-PEG40) or AAT: inhibition curve. Suc-Ala-Ala-Pro-Val-pNA degradation by 15mg of an expectoration from a patient with COPD followed by absorbance measurements during one hour in presence or absence of increasing concentration of wild type NbE201, NbE201-PEG40 or AAT. N=1, n=2. Mean of 2 technical replicates $\pm$ SD.( ●) NbE201, (■) NbE201-PEG40, (▲) AAT . N represents the number of experiments; n represents the number of replicates.

**Figure 26.** Ciliary Dyskinesia sputum proteolytic activity inhibition by NbE201(-PEG40) or AAT: inhibition curve. Suc-Ala-Ala-Pro-Val-pNA degradation by 8mg of an expectoration was followed by absorbance measurements during one hour in presence or absence of increasing concentration of NbE201, NbE201-PEG40 or AAT. N=1, n=3. Mean of 3 technical replicates $\pm$ SD.( ●) NbE201, (■) NbE201-PEG40, (▲) AAT.

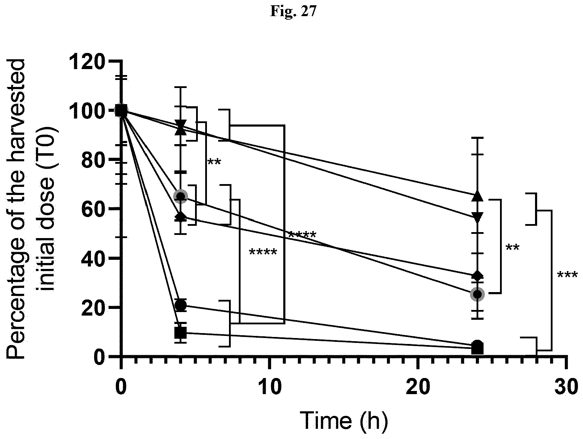

**Figure 27.** Percentage of the initial dose of NbE201, NbE201-PEG40 or AAT harvested at time 0h after intratracheal instillation in Swiss or $\beta$-ENaC mice. N=5. Mean $\pm$ SD. Statistical test:One-way Anova, pot-hoc test: Tukey. **p value<0.01, ***p value <0.001, ****p value <0.0001. (●)NbE201 Swiss mice, (▲)NbE201-PEG40 Swiss mice (■) NbE201 $\beta$-ENaC mice, (▼)NbE201-PEG40 $\beta$-ENaC mice, (♦) AAT Swiss mice, and ☀ AAT $\beta$-ENaC mice.

## DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "having", "have", "including", "includes", "containing", or "contains", and are inclusive or openended and do not exclude additional, non-recited members, elements or method steps. The terms also encompass "constituted of", "consists in", "consisting of", and "consists of", and also the terms "consisting essentially of", "consisting essentially in" and "consists essentially of', which enjoy well-established meanings in patent terminology.

The recitation of numerical ranges by endpoints includes all integer numbers and, where appropriate, fractions subsumed within the respective ranges, as well as the recited endpoints. This applies to numerical ranges irrespective of whether they are introduced by the expression "from... to..." or the expression "between... and..." or another expression. Any numerical range recited herein is intended to include all sub-ranges subsumed therein.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone, B alone, C alone, A and B in combination, A and C in combination, B and C in combination, or A, B, and C in combination.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As corroborated by the experimental section, which illustrates certain representative embodiments of the present invention, the inventors provide advantageous applications of certain neutrophil elastase (NE) binding agents, such as in particular human NE (hNE) binding agents and nucleic acid molecules, vectors, viruses, cells comprising such binding agents, methods to manufacture these and their use in medicine. The present anti-NE immunoglobulin single variable domain agents display one or more advantages, and may for example serve as a competitive inhibitor, exhibit strong affinity, specificity, and inhibitory capacity towards NE. Additionally, the anti-NE ISVD can exhibit exceptional stability *in vivo* and an extended half-life in the lung, in particular when PEGylated, thereby paving the way for therapeutic interventions and the potential for combined therapy applications involving the anti-NE ISVD.

An aspect of the invention thus provides a binding agent capable of specifically binding to and inhibiting a neutrophil elastase (NE), wherein the binding agent comprises an immunoglobulin single variable domain (ISVD) comprising a complementarity determining region 1 (CDR1) having the sequence set forth in SEQ ID NO: 1 (GRTISLYR), a CDR2 having the sequence set forth in SEQ ID NO: 2 (INWSGDMT) and a CDR3 having the sequence set forth in SEQ ID NO: 3 (TADPKLLPLADSSYGY).

NE as intended herein may particularly concern human NE (hNE). The qualifier "human" as used herein in connection with a NE protein may in a certain interpretation refer to the amino acid sequence of the NE protein. For example, a NE protein having the amino acid sequence as a NE protein found in humans may also be obtained by technical means, e.g., by recombinant expression, cell-free translation, or non-biological peptide synthesis. Because the present ISVDs are intended to therapeutically target NE in humans, in a certain other interpretation the qualifier "human" may more particularly refer to a NE protein as found in or present in humans, regardless of whether the NE protein forms a part of or has been at least partly isolated from human subjects, organs, cells, or tissues. A skilled person understands that the amino acid sequence of a given native protein such as a NE protein may differ between or within different individuals of the same species due to normal genetic diversity (allelic variation, polymorphism) within that species and/or due to differences in post-transcriptional or post-translational modifications. Any such variants or isoforms of the native protein are subsumed by the reference to or designation of the protein.

By means of further guidance and without limitation, human NE is annotated under U.S. government's National Center for Biotechnology Information (NCBI) Genbank (http://www.ncbi.nlm.nih.gov/) Gene ID no. 1991. A human wild-type NE preprotein amino acid sequence may be as annotated under Genbank accession no: NP_001963.1, reproduced here below:

MTLGRRLACLFLACVLPALLLGGTALASEIVGGRRARPHAWPFMVSLQLRGGHFCGATLI

APNFVMSAAHCVANVNRAVRVVLGAHNLSRREPTRQVFAVQRIFENGYDPVNLLNDIVI

LQLNGSATINANVQVAQLPAQGRRLGNGVQCLAMGWGLLGRNRGIASVLQELNVTVVTS

LCRRSNVCTLVRGRQAGVCFGDSGSPLVCNGLIHGIASFVRGGCASGLYPDAFAPVAQFV

NWIDSIIQRSEDNPCPHPRDPDPASRTH (SEQ ID NO: 13)

A skilled person can appreciate that any sequences represented in sequence databases or in the present specification may be of precursors and may include parts which are processed away from mature molecules. Without limitation, the sequence of mature human NE after processing away of a signal peptide, the dipeptide SE and the c-terminal propeptide may be as shown below:

IVGGRRARPHAWPFMVSLQLRGGHFCGATLIAPNFVMSAAHCVANVNRAVRVVLGAH

NLSRREPTRQVFAVQRIFENGYDPVNLLNDIVILQLNGSATINANVQVAQLPAQGRRLGNG

VQCLAMGWGLLGRNRGIASVLQELNVTVVTSLCRRSNVCTLVRGRQAGVCFGDSGSPLV

CNGLIHGIASFVRGGCASGLYPDAFAPVAQFVNWIDSIIQ (SEQ ID NO: 14)

By means of further elucidation of scientific knowledge regarding the biological relevance of NE, and without limitation, NE, a serine endopeptidase, possesses a catalytic site consisting of the amino acids Asp, His, and Ser. Among serine proteases found in neutrophils, NE is the most abundant. It is predominantly stored at high concentrations within the azurophilic granules of neutrophils. Upon neutrophil activation, NE can bind to chondroitin sulfate and heparan sulfate proteoglycans, leading to its localization on the cell surface. NE exhibits proteolytic bursts at the neutrophil surface, attributed to its high local concentration, which temporarily overwhelms endogenous antiprotease levels. While NE's protease activity is crucial for innate immune function, its release into the airway environment contributes to the progression of lung diseases.

On a deeper level, NE upregulates the gene expression of Mucin 5AC (MUC5AC), a major gel-forming mucin secreted in the respiratory tract. NE can also activate intracellular signals, including reactive oxygen species (ROS), nicotinamide adenine dinucleotide phosphate (NADPH) quinone oxidoreductase 1 (NQO1), and epidermal growth factor receptor (EGFR), which contribute to the upregulation of MUC5AC expression. By releasing cell surface transforming growth factor $\alpha$ (TGF$\alpha$) and enhancing epithelial permeability through the degradation junctional proteins like Zona occludins-1 and E-cadherin, NE facilitates the ligation of TGF$\alpha$-induced EGFR. In other words, NE permits the basolateral ligand, TGF$\alpha$ access to activate EGFR on the apical site of the cell-membrane. Moreover, NE induces the secretion of mucins from bronchial epithelial cells and contributes to airway mucus obstruction. Additionally, NE triggers goblet cell metaplasia, altering the epithelial composition in the airway and perpetuating increased mucin production and secretion in the airways.

Within the airways, effective mucociliary clearance relies on the presence of adequate mucin levels, proper hydration of the airway surface liquid, and a healthy ciliated epithelium. NE, however, disrupts each of these essential components necessary for preserved mucociliary clearance. In addition to increasing mucin abundance in the airway, NE hinders airway surface liquid hydration by degrading the cystic fibrosis transmembrane conductance regulator (CFTR), an apical chloride channel, and activating ENaC, an apical epithelial sodium channel. Consequently, the ionic regulation of airway hydration becomes disrupted. Furthermore, NE reduces ciliary motility and causes damage to ciliary structures, further impairing the effectiveness of mucociliary clearance within the airways. Considering the effect of NE in the airways, NE emerged as a suitable drug target for inflammatory pulmonary diseases, such as CF and COPD.

The anti-NE ISVD mentioned herein inhibits NE by binding to at least a portion of the active site of NE. By binding into the active site, the anti-NE ISVD can block the access of substrates to the serine protease, preventing proteolytic activity and interfering with the normal function of NE.

It is generally known that hNE is a serine protease, which means its catalytic activity is dependent on the presence of a serine residue within its active site. The catalytic triad of residues in hNE consists of histidine 57 (H57), aspartate 102 (D102), and serine 195 (S195). The numbering is according to the chymotrypsinogen scheme, a correlation between this scheme and FASTA numbering is shown in Figure 1.

The serine residue is an important component of the catalytic site and is responsible for nucleophilic attack during the proteolysis process. The histidine residue acts as a general base, deprotonating the serine hydroxyl group to enhance its nucleophilicity. The aspartate residue (D102) stabilizes the histidine residue and helps in maintaining the proper conformation of the active site.

The binding of a substrate or inhibitor to the active site of an enzyme can be demonstrated with X-ray crystallography as shown in example 10, in which the enzyme-substrate or enzyme-inhibitor complex is crystallizing and then subjected to X-ray diffraction analysis. The resulting diffraction pattern can be used to determine the three-dimensional structure of the complex, including the binding interactions at the active site. Some other methods include without limitation Nuclear Magnetic Resonance (NMR) spectroscopy, active site-directed mutagenesis, fluorescence spectroscopy, isothermal titration calorimetry (ITC), mass spectrometry and enzyme kinetics experiments.

In a certain embodiment, the anti-NE ISVD is capable of inhibiting NE activity with a 50% inhibitory concentration ($IC_{50}$) of 60 nM or less, preferably 50 nM or less, more preferably 42 nM or less, for instance as carried out in the examples with an *in vitro* assay with N-Sucinyl-Ala-Ala-Ala-paraNitroAnilide substrate or with an $IC_{50}$ of 20 to 70 nM, preferably 25 to 60 nM, more preferably 30 to 55 nM, even more preferably 35 to 50 nM, even more preferably 30 to 45 nM, even more preferably 35 to 40 nM and most preferably 38.3 nM as determined in an *in vitro* assay with elastin substrate.

In another embodiment the anti-NE ISVD is capable of binding the NE with a dissociation constant ($K_D$) of 4 nM or less, preferably 3 nM or less, as determined by bio-layer interferometry (BLI).

Yet in another embodiment the anti-NE ISVD has a Gibbs energy change ($\Delta G(H_2O)$ of at least 25 kJ mole (-1), more preferably at least 30 kJ mole (-1), even more preferably at least 35 kJ mole (-1) as measured by urea-induced unfolding at 25°C and pH 7.

Yet in another embodiment the anti-NE ISVD comprises a melting temperature ($T_m$) of at least 65°C, preferably at least 68°C, more preferably at least 70°C.

In yet another embodiment the anti-NE ISVD can compete with alphal-anti-trypsin (AAT) for binding to the NE.

In another embodiment the anti-NE ISVD can bind to or within a region of the NE comprising the active site of NE. Yet in another embodiment the anti-NE ISVD can bind to amino acid residues R36, A60, N61, P96, V97, S195, G218 and G219 ofNE as shown in Figure 1.

In certain embodiments CDR3 of the anti-NE ISVD can interact with the amino acid residues R36, A60, N61, P96, S195, V97 of NE according to the chymotrypsinogen numbering scheme. More specifically, the amino acids P100, K101, D107, Y110 and Y112, according to sequential FASTA numbering, from CDR3 of the anti-NE ISVD can interact with the amino acid residues R36, A60, N61, P96, V97, S195, G218 and G219 of NE according to the chymotrypsinogen numbering scheme. The residue P100 binds via a water molecule to the S195 of the catalytic triad of the enzyme.

As used herein, the term "antibody" is used in the broadest sense and generally refers to an immunologic binding agent. The term encompasses whole immunoglobulin molecules, immunologically effective fragments of immunoglobulins, i.e., fragments displaying the ability to specifically bind the antigen recognised by the whole immunoglobulin molecule, as well as constructs comprising an antigen-binding portion comprised within a modified immunoglobulin-like framework, and constructs comprising an antigen-binding portion comprised within a non-immunoglobulin-like framework or scaffold. Antibody fragments comprise a portion of an intact antibody comprising the antigen-binding or variable region thereof. Examples of antibody fragments include Fab, Fab', F(ab')2, Fv, and single-domain sdFv (sdFv) antibodies, such as VL, VH or VHH single-domain antibodies. Fusions proteins of the heavy (VH) and light (VL) chain variable regions, commonly known as single chain Fv (scFv), are also included in antibody fragments. The term "antibody" thus includes without limitation intact monoclonal antibodies, intact polyclonal antibodies, multivalent (e.g., 2-, 3- or more-valent) antibodies and/or multi-specific (e.g., bi- or more-specific) antibodies formed from at least two intact antibodies, and further immunologically effective fragments of any of such antibodies as well as multivalent and/or multi-specific composites of such fragments (e.g., diabodies, triabodies, tetrabodies, multibodies). The term further encompasses without limitation intact antibodies and antibody fragments of non-human animal origin, as well as chimeric, humanised or chimeric/humanised forms of such antibodies or antibody fragments, and further encompasses fully human antibodies or antibody fragments. More broadly, grafting of at least one complementarity-determining region (CDR) from an antibody of one origin onto a framework of another origin is contemplated. The term "antibody" also encompasses any fusion proteins, protein conjugates or protein complexes comprising an immunoglobulin molecule or an immunologically effective fragment thereof, as well as chemically and/or enzymatically modified or derivatised immunoglobulin molecules or immunologically effective fragments thereof. The term "antibody" is not only inclusive of antibodies generated by methods comprising immunisation, but also includes any polypeptide which is made to encompass at least one CDR capable of specifically binding to an epitope on a cognate antigen, regardless whether such molecules are produced *in vitro*, in cell culture, or *in vivo*. For example, antibodies produced by recombinant DNA techniques in cultured host cells (e.g., bacterial, yeast or fungal, plant or animal cells) or in non-human host organisms (e.g., in transgenic plants or transgenic animals) are also encompassed.

**EP 4 483 951 A1**

The aspects disclosed herein employ an anti-NE immunoglobulin single variable domain (ISVD), more preferably an anti-hNE ISVD. The term "domain" (of a polypeptide or protein) as used herein refers to a folded protein structure which has the ability to retain its tertiary structure independently of the rest of the protein. Generally, domains are responsible for discrete functional properties of proteins, and in many cases may be added, removed or transferred to other proteins without loss of function of the remainder of the protein and/or of the domain. The term as contemplated here is particularly used to denote an "immunoglobulin domain", a globular region of an antibody chain (such as for example a chain of a conventional 4-chain antibody or of a heavy chain antibody), or to a polypeptide that essentially consists of or consists of such a globular region. Structurally, immunoglobulin domains have been described as retaining the immunoglobulin fold characteristic of antibody molecules, which in particular involves a two-layer sandwich of about seven antiparallel beta-strands arranged in two beta-sheets, optionally stabilised by a conserved disulphide bond. Particularly intended by reference to a "domain" is the immunoglobulin variable domain.

The terms "single-domain" or "single variable domain" or "immunoglobulin single variable domain" defines molecules wherein the antigen-binding site is present on, and formed by, a single immunoglobulin domain. This sets immunoglobulin single variable domains apart from "conventional" immunoglobulins or their fragments, wherein two immunoglobulin domains, in particular two variable domains, interact to form an antigen binding site. Typically, in conventional immunoglobulins, a heavy chain variable domain (VH) and a light chain variable domain (VL) interact to form an antigen binding site. In this case, the complementarity determining regions (CDRs) of both VH and VL will contribute to the antigen binding site, i.e., a total of 6 CDRs will be involved in antigen binding site formation. Hence, the antigen-binding domain of a conventional 4-chain antibody (such as an IgG, IgM, IgA, IgD or IgE molecule) or of a Fab fragment, a F(ab')2 fragment, an Fv fragment such as a disulphide linked Fv or a scFv fragment, or a diabody derived from such conventional 4-chain antibody, would normally not be regarded as an immunoglobulin single variable domain, since in these cases binding to the respective epitope of an antigen would normally not occur by one (single) immunoglobulin domain but by a pair of (associated) immunoglobulin domains such as light and heavy chain variable domains, i.e., by a VH-VL pair of immunoglobulin domains, which jointly bind to an epitope of the respective antigen.

In contrast, immunoglobulin single variable domains are capable of specifically binding to an epitope of the antigen without pairing with an additional immunoglobulin variable domain. The binding site of an immunoglobulin single variable domain is formed by a single VH, VHH or VL domain. Hence, the antigen binding site of an immunoglobulin single variable domain is formed by no more than three CDRs. An immunoglobulin single variable domain may be a light chain variable domain (a VL-sequence) or a suitable fragment thereof, or a heavy chain variable domain (a VH-sequence or a VHH-sequence) or a suitable fragment thereof, insofar capable of forming a single antigen binding unit (i.e., a functional antigen binding unit that essentially consists of the single variable domain, such that the single antigen binding domain does not need to interact with another variable domain to form a functional antigen binding unit).

ISVDs in their broadest sense are not limited to a specific biological source or to a specific method of preparation. The term "immunoglobulin single variable domain" encompasses variable domains of different origin, comprising mouse, rat, rabbit, donkey, human, shark (for example, the so-called "IgNAR domains", see for example WO 05/18629), and camelid variable domains.

Hence, as contemplated herein, the operative, antigen-binding principle of a single-domain antibody is an immunoglobulin single variable domain. In certain preferred embodiments, this single-domain may be a "heavy chain variable domain" which, as used herein, denotes (i) the variable domain derived from the heavy chain of a heavy-chain antibody, which is naturally devoid of light chains, including but not limited to the variable domain of the heavy chain of heavy-chain antibodies of camelids or sharks or (ii) the variable domain derived from the heavy chain of a conventional four-chain antibody (also indicated hereafter as $V_H$), including but not limited to a camelised (as further defined herein) variable domain of the heavy chain of a conventional four-chain antibody (also indicated hereafter as camelised $V_H$), or any functional fragments thereof. In certain preferred embodiments, the single-domain may be as stated in (i). Hence, in certain embodiments, the single-domain antibody as intended herein is a heavy chain variable domain derived from a heavy-chain antibody ($V_{HH}$) or a functional fragment thereof, i.e., an hNE-binding fragment thereof. Such fragment may for example bind hNE with $K_D$ not higher than $10\times$ the $K_D$ of the full-length reference, preferably not higher than 5x the $K_D$ of the full-length reference, more preferably not higher than 2x the $K_D$ of the full-length reference, such as with $K_D$ substantially the same as the $K_D$ of the full-length reference (e.g., +/- 1.5x or +/- 1.2x).

"VHH domains", also known as VHHs, VHH domains, VHH antibody fragments, and VHH antibodies, have originally been described as the antigen binding immunoglobulin (variable) domain of "heavy-chain antibodies", i.e., of antibodies devoid of light chains (Hamers-Casterman et al. Naturally occurring antibodies devoid of light chains. Nature 1993, vol. 363, 446-448). The term "VHH domain" has been chosen in order to distinguish these variable domains from the heavy chain variable domains that are present in conventional 4-chain antibodies (which are referred to conventionally and herein as "VH domains") and from the light chain variable domains that are present in conventional 4-chain antibodies (which are referred to conventionally and herein as "VL domains"). Hence, in certain preferred embodiments, the single-domain may be a VHH domain, or in other words the anti-NE antibody may be a VHH single-domain antibody, or VHH antibody in short.

In certain preferred embodiments, the anti-NE antibody may be a nanobody. The terms "nanobody" (Nb) as used herein ("Nanobody®", "Nanobodies®" and "Nanoclone®" are registered trademarks of Ablynx N.V, Ghent, Belgium) refers to a single variable domain derived from naturally occurring heavy-chain antibodies (devoid of light chains), in particular those found in camelids (Hamers-Casterman et al. 1993, *supra*; Desmyter et al. Crystal structure of a camel single-domain VH antibody fragment in complex with lysozyme. Nat Struct Biol. 1996, vol. 3, 803-811), and consequently often referred to as VHH antibody or VHH. Camelids comprise old world camelids (*Camelus bactrianus* and *Camelus dromedarius*) and new world camelids (for example *Vicugna pacos, Lama glama, Lama guanicoe* and *Vicugna vicugna*). The term nanobody as used herein in its broadest sense is not limited to a specific biological source or to a specific method of preparation. For example, nanobodies in the broadest sense may encompass an immunological binding agent obtained: (1) by isolating the VHH domain of a naturally occurring heavy-chain antibody; (2) by expression of a nucleotide sequence encoding a naturally occurring VHH domain; (3) by "humanisation" of a naturally occurring VHH domain or by expression of a nucleic acid encoding such a humanised VHH domain; (4) by "camelisation" of a naturally occurring VH domain from any animal species, and in particular from a mammalian species, such as from a human being, or by expression of a nucleic acid encoding such a camelised VH domain; (5) by "camelisation" of a "domain antibody" or "Dab" as described in the art or by expression of a nucleic acid encoding such a camelised VH domain; (6) by using synthetic or semi-synthetic techniques for preparing proteins, polypeptides or other amino acid sequences known *per se*; (7) by preparing a nucleic acid encoding a nanobody using techniques for nucleic acid synthesis known *per se,* followed by expression of the nucleic acid thus obtained; and/or (8) by any combination of one or more of the foregoing.

For a further description of VHHs and nanobodies, reference is made to the review article by Muyldermans (Reviews in Molecular Biotechnology 74: 277-302, 2001), as well as to the following patent applications, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134231 and WO 02/48193 of Unilever; WO 97/49805, WO 01/21817, WO 03/035694, WO 03/054016 and WO 03/055527 of the Vlaams Instituut voor Biotechnologie (VIB); WO 03/050531 ofAlgonomics N.V. and Ablynx N.V; WO 01/90190 by the National Research Council of Canada; WO 03/025020 (= EP 1433793) by the Institute of Antibodies; as well as WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V and the further published patent applications by Ablynx N.V.

The reference to single-domain antibodies including VHH and domain antibodies also encompasses functional fragments thereof that retain at least part of or the entirety of the functional activity and/or retain at least part of or the entirety of the binding specificity of the original immunoglobulin single variable domain such as VHH domain from which the fragments are derived. Functional fragments are not particularly limited as to their length and/or size, and may without limitation denote N-terminally and/or C-terminally deleted or truncated forms of the original immunoglobulin single variable domain which may for example represent at least about 50% (by amino acid number), e.g., at least about 60%, or at least about 70%, or at least about 80%, or at least about 90%, or at least about 91%, or at least about 92%, or at least about 93%, or at least about 94%, or at least about 95%, or at least about 96%, or at least about 97%, or at least about 98%, or at least about 99% of the contiguous amino acid sequence of said original immunoglobulin single variable domain. The term encompasses fragments arising by any mechanism, such as, without limitation, by heterologous expression of a truncated form of the immunoglobulin single variable domain, or by physical, chemical or enzymatic proteolysis thereof. Usually, a functional fragment of an immunoglobulin single variable domain such as a VHH domain as disclosed herein contains at least some of the amino acid residues that form at least one of the complementarity determining regions of the original immunoglobulin single variable domain such as VHH domain from which they are derived from.

The antibodies contemplated herein are anti-NE antibodies, i.e., the antibodies specifically bind to NE. The term "specifically bind" as used throughout this specification means that an agent binds to one or more desired molecules or analytes substantially to the exclusion of other molecules which are random or unrelated, and optionally substantially to the exclusion of other molecules that are structurally related. Put differently, an antibody is said to specifically bind an antigen when it preferentially recognises its target antigen in a complex mixture of proteins and/or macromolecules.

The binding of an antibody would in particular be to an epitope on the NE protein. The term "epitope" includes any polypeptide determinant capable of specifically binding to an immunoglobulin or T-cell receptor. Epitope determinants may include chemically active surface groupings of molecules such as amino acids, sugar side chains, phosphoryl, or sulfonyl, and may have specific three-dimensional structural characteristics, and/or specific charge characteristics. An epitope is a region of an antigen that is bound by an antibody. An antibody is said to specifically bind an antigen when it preferentially recognises its target antigen in a complex mixture of proteins and/or macromolecules.

The term "specificity" refers to the number of different types of antigens or antigenic determinants to which a particular antigen-binding molecule or antigen-binding protein (such as an antibody) molecule can bind. The specificity of an antigen-binding protein can be determined based on affinity and/or avidity. The affinity, represented by the equilibrium constant for the dissociation of an antigen with an antigen-binding protein ($K_D$), is a measure for the binding strength between an antigenic determinant and an antigen-binding site on the antigen-binding protein: the lesser the value of the

$K_D$, the stronger the binding strength between an antigenic determinant and the antigen-binding molecule (alternatively, the affinity can also be expressed as the affinity constant ($K_A$), which is $1/K_D$). As will be clear to the skilled person, affinity can be determined in a manner known *per se,* depending on the specific antigen of interest. Avidity is the measure of the strength of binding between an antigen-binding molecule (such as an antibody) and the pertinent antigen. Avidity is related to both the affinity between an antigenic determinant and its antigen binding site on the antigen-binding molecule and the number of pertinent binding sites present on the antigen-binding molecule. Typically, antigen-binding proteins (such as antibodies) will bind with a dissociation constant ($K_D$) of $1 \times 10^{-5}$ to $1 \times 10^{-12}$ moles/liter (M) or less, and preferably $1 \times 10^{-7}$ to $1 \times 10^{-12}$ M or less, and more preferably $1 \times 10^{-8}$ to $1 \times 10^{-12}$ M or less and even more preferably $1 \times 10^{-9}$ to $1 \times 10^{-12}$ M or less, such as between $1 \times 10^{-9}$ and $1 \times 10^{-10}$ M, or between $1 \times 10^{-10}$ and $1 \times 10^{-11}$ M, wherein $K_D$ = [AB][AG]/[AB-AG], AB denotes the antibody, AG denotes the antigen, and AB-AG denotes the antibody-antigen complex. Any $K_D$ value greater than $10^{-4}$ M is generally considered to indicate nonspecific binding. Preferably, an antibody will bind to the desired antigen with a $K_D$ less than 500 nM, preferably less than 200 nM, more preferably less than 10 nM, such as less than 5nM, less than 4 nM, less than 3 nM, less than 2 nM, or less than 1 nM, e.g., about 500 pM, about 600 pM, about 700 pM, about 800 pM, or about 900 pM. In certain preferred examples, an antibody will bind to the desired antigen with a $K_D$ of between 500 pM and 3 nM. Specific binding of an antigen-binding protein to an antigen or antigenic determinant can be determined in any suitable manner known *per se,* including, for example, Scatchard plot analysis and/or competitive binding assays, such as immunoassays, enzyme immunoassays (EIA) and sandwich competition assays, and the different variants thereof known *per se* in the art.

In certain particularly preferred embodiments, the anti-NE ISVD may comprise, consist essentially of or consists of an amino acid sequence having a sequence identity of at least 90% to SEQ ID NO: 4 or a functional fragment thereof:

QVQLQESGGGGLVQAGGSLRLSCVVP<u>GRTISLYR</u>MGWFRQAPGKEREFVAG**INWSGDMTD** YVDSVKGRFTISRDNAKNTVYLEMNSLKPEDTAIYYC<u>**TADPKLLPLADSSYGY**</u>WGQGTQ VTVSS (SEQ ID NO: 4)

SEQ ID NO: 4 comprises CDR1, CDR2 and CDR3 as set forth in SEQ ID NO: 1, 2 and 3, indicated above in underlined, bold, and underlined bold fonts, respectively. The CDR sequences shown above have been annotated in SEQ ID NO: 4 in accordance with the IMGT numbering. In certain embodiments, the anti-NE ISVD may comprise, consist essentially of or consists of an amino acid sequence having a sequence identity of preferably at least 91%, at least 92%, at least 93% or at least 94% to SEQ ID NO: 4, more preferably a sequence identity of at least 95%, such as at least 96%, at least 97%, at least 98% or at least 99% to SEQ ID NO: 4, or a functional fragment thereof.

In particular embodiments the anti-NE ISVD comprises CDR1, CDR2 and CDR3 sequences, each as present in SEQ ID NO: 4, the CDR1, CDR2 and CDR3 sequences being annotated according to any one of IMGT, Kabat, Chlotia, Martin or Aho numbering systems. These respective CDR1-3 annotations, and the corresponding framework regions (FR1-4) are shown on the SEQ ID NO: 4 sequence in Figure 2.

In certain particularly preferred embodiments, the anti-NE ISVD comprises, consists essentially of or consists of the amino acid sequence set forth in SEQ ID NO:4, or a functional fragment thereof. In certain particularly preferred embodiments, the anti-NE ISVD is NbE201 as described in the examples.

SEQ ID NO: 4 comprises FR1, FR2, FR3 and FR4 as set forth in SEQ ID NO: 17, 18, 19 and 20, indicated below in consecutive order in bold font in SEQ ID NO: 4:

**QVQLQESGGGGLVQAGGSLRLSCVVP**GRTISLYR**MGWFRQAPGKEREFVAG**INWSGDM TD**YVDSVKGRFTISRDNAKNTVYLEMNSLKPEDTAIYYC**TADPKLLPLADSSYGY**WGQ GTQVTVSS** (SEQ ID NO: 4).

The FRs sequences shown above have been annotated in SEQ ID NO: 4 in accordance with the IMGT numbering. In certain embodiments, the anti-NE ISVD may comprise, each independently, a framework region 1 (FR1) having a sequence at least 90%, preferably at least 91%, at least 92%, at least 93% or at least 94%, more preferably a sequence identity of at least 95%, such as at least 96%, at least 97%, at least 98% or at least 99% identical to the sequence set forth in SEQ ID NO: 17 (QVQLQESGGGGLVQAGGSLRLSCVVP) and framework region 2 (FR2) having a sequence at least 90%, preferably at least 91%, at least 92%, at least 93% or at least 94%, more preferably a sequence identity of at least 95%, such as at least 96%, at least 97%, at least 98% or at least 99% identical to the sequence set forth in SEQ ID NO: 18 (MGWFRQAPGKEREFVAG), framework region 3 (FR3) having a sequence at least 90%, preferably at least 91%, at least 92%, at least 93% or at least 94%, more preferably a sequence identity of at least 95%, such as at least 96%, at least 97%, at least 98% or at least 99% identical to the sequence set forth in SEQ ID NO: 19 (DYVDSVKGRFTISRDNAKNTVYLEMNSLKPEDTAIYYC) and framework region (FR4) having a sequence at least 90%, preferably at least 91%, at least 92%, at least 93% or at least 94%, more preferably a sequence identity of at least 95%, such as at least 96%, at least 97%, at least 98% or at least 99% identical to the sequence set forth in SEQ ID NO: 20 (WGQGTQVTVSS).

In certain embodiments, the anti-NE ISVD may comprise FR 1-4 sequences each independently having a sequence identity of at least 90%, preferably at least 91%, at least 92%, at least 93% or at least 94%, more preferably at least 95%, such as at least 96%, at least 97%, at least 98% or at least 99% to the respective FR1-4 sequences as present in SEQ ID NO: 4, in accordance with any one of IMGT, Kabat, Chlotia, Martin or Aho numbering systems.

The term "protein" generally encompasses macromolecules comprising one or more polypeptide chains. The term "polypeptide" generally encompasses linear polymeric chains of amino acid residues linked by peptide bonds. A "peptide bond", "peptide link" or "amide bond" is a covalent bond formed between two amino acids when the carboxyl group of one amino acid reacts with the amino group of the other amino acid, thereby releasing a molecule of water. Especially when a protein is only composed of a single polypeptide chain, the terms "protein" and "polypeptide" may be used interchangeably to denote such a protein. The terms are not limited to any minimum length of the polypeptide chain. Polypeptide chains consisting essentially of or consisting of 50 or less ($\leq$ 50) amino acids, such as $\leq$ 45, $\leq$ 40, $\leq$ 35, $\leq$ 30, $\leq$ 25, $\leq$ 20, $\leq$ 15, $\leq$ 10 or $\leq$ 5 amino acids may be commonly denoted as a "peptide". In the context of proteins, polypeptides or peptides, a "sequence" is the order of amino acids in the chain in an amino to carboxyl terminal direction in which residues that neighbour each other in the sequence are contiguous in the primary structure of the protein, polypeptide or peptide. The terms may encompass naturally, recombinantly, semi-synthetically or synthetically produced proteins, polypeptides or peptides. Hence, for example, a protein, polypeptide or peptide can be present in or isolated from nature, e.g., produced or expressed natively or endogenously by a cell or tissue and optionally isolated therefrom; or a protein, polypeptide or peptide can be recombinant, i.e., produced by recombinant DNA technology, and/or can be, partly or entirely, chemically or biochemically synthesised. Without limitation, a protein, polypeptide or peptide can be produced recombinantly by a suitable host or host cell expression system and optionally isolated therefrom (e.g., a suitable bacterial, yeast, fungal, plant or animal host or host cell expression system), or produced recombinantly by cell-free translation or cell-free transcription and translation, or non-biological peptide, polypeptide or protein synthesis. The terms also encompasses proteins, polypeptides or peptides that carry one or more co- or post-expression-type modifications of the polypeptide chain(s), such as, without limitation, glycosylation, lipidation, acetylation, amidation, phosphorylation, sulphonation, methylation, PEGylation (covalent attachment of polyethylene glycol typically to the N-terminus or to the side-chain of one or more Lys residues), ubiquitination, sumoylation, cysteinylation, glutathionylation, oxidation of methionine to methionine sulphoxide or methionine sulphone, signal peptide removal, N-terminal Met removal, conversion of pro-enzymes or pre-hormones into active forms, etc. Such co- or post-expression-type modifications may be introduced *in vivo* by a host cell expressing the proteins, polypeptides or peptides (co- or post-translational protein modification machinery may be native to the host cell and/or the host cell may be genetically engineered to comprise one or more (additional) co- or post-translational protein modification functionalities), or may be introduced *in vitro* by chemical (e.g., PEGylation) and/or biochemical (e.g., enzymatic) modification of the isolated proteins, polypeptides or peptides.

In certain embodiments, the anti-NE ISVD binding agent may comprise a polyethylene glycol moiety. In certain embodiments, the PEG moiety may be connected to, such as particularly covalently bound to, the ISVD, such as particularly to an amino acid of the ISVD. One or more amino acid suitable for connecting the PEG moiety thereto may be provided in the ISVD, such as by genetic engineering.

In a certain embodiment, an amino acid residue such as lysine, histidine, arginine, aspartic acid, glutamic acid, serine, threonine, tyrosine or preferably cysteine can be introduced at the C-terminus or the N-terminus of the anti-NE ISVD through genetic engineering. Such a modification allows for the functionalization of anti-NE ISVD by attaching a PEG moiety, thereby extending its half-life in the lungs. The site-specific PEGylation of anti-NE ISVD via an amino acid residue provides protection against aggregation during nebulization and spray-drying processes, whereas the unmodified anti-NE ISVD is prone to aggregation under similar conditions, as shown in the examples below.

In a preferred embodiment a linker is introduced before the cysteine residue at the C-terminus of the anti-NE ISVD through genetic engineering. This allows for the functionalization of anti-NE ISVD by attaching a polyethylene glycol (PEG) moiety, thereby extending its half-life in the lungs. The site-specific PEGylation of anti-NE ISVD via a linker in proximity of the cysteine residue provides protection against aggregation during nebulization and spray-drying processes, whereas the unmodified anti-NE ISVD is prone to aggregation under similar conditions, as shown in the examples below.

In particular embodiments, any one linker may be a peptide or polypeptide linker of one or more amino acids. In certain embodiments, all linkers in the molecule may be peptide or polypeptide linkers. More particularly, the peptide linker may be 1 to 20 amino acids long, such as preferably 1 to 15 amino acids long, such as more preferably 12 to 14 amino acids long. For example, the linker may be exactly 1, 2, 3, 4, 5, 6, 7, 8, 9, 10, 11, 12, 13, 14 or 15 amino acids long, such as preferably exactly 12, 13 or 14 amino acids long. The nature of amino acids constituting the linker is not of particular relevance so long as the biological activity of the molecule stretches linked thereby is not substantially impaired. Preferred linkers are essentially non-immunogenic and/or not prone to proteolytic cleavage. In certain embodiments, the linker may contain a predicted secondary structure such as an alpha-helical structure. However, linkers predicted to assume flexible, random coil structures are preferred. Linkers having tendency to form beta-strands may be less preferred or may need to be avoided. Cysteine residues may be less preferred or may need to be avoided due to their capacity to form intermolecular disulphide bridges. Basic or acidic amino acid residues, such as arginine, lysine, histidine, aspartic acid and glutamic acid may be less preferred or may need to be avoided due to their capacity for unintended electrostatic interactions. In certain preferred embodiments, the peptide linker may comprise, consist essentially of or consist of amino acids selected from the group consisting of glycine, serine, alanine, phenylalanine, threonine, proline, and combinations thereof, including D-isomers and analogues thereof. In certain preferred embodiments, the peptide linker may comprise, consist essentially of or consist of amino acids selected from the group consisting of glycine, serine, alanine, threonine, proline, and combinations thereof, including D-isomers and analogues thereof. In even more preferred embodiments, the peptide linker may comprise, consist essentially of or consist of amino acids selected from the group consisting of serine, threonine, proline and combinations thereof, including D-isomers and analogues thereof. In certain embodiments, the peptide linker may consist of only serine, threonine and proline residues. In certain embodiments, the peptide linker may consist of only serine or only threonine or only proline or D-isomers or analogues thereof. In certain embodiments, the linker may comprise serine, threonine and proline residues of which proline and serine are present at a ratio between 7:1 and 1:7 (by number), such as about 6:1, about 5:1, about 4: 1, about 3: 1, about 2: 1, about 3:2, about 1:1, about 1:2, about 2:3, about 1:3, about 1:4, about 1:5 or about 1:6 proline : serine. Preferably, proline may be more abundant than serine, e.g., a ratio between 7:4 or 3:2 or 2:1 proline : serine (by number), preferably at a ratio of 3:2 proline : serine (by number). In certain embodiments, the linker may comprise serine, threonine and proline residues of which threonine and proline residues are present at a ratio between 7: 1 and 1:7 (by number), such as about 6:1, about 5:1, about 4: 1, about 3: 1, about 2: 1, about 1:1, about 1:2, about 1:3, about 1:4, about 1:5 or about 1:6 proline : threonine. Preferably, proline may be more abundant than threonine, e.g., a ratio between 7:3 or 3:1 or 2:1 proline : threonine (by number) , preferably at a ratio of 2:1 proline : threonine (by number). In certain embodiments, the linker may comprise serine, threonine and proline residues of which serine and threonine are present at a ratio between 6:1 and 1:6 (by number), such as about 5:1, about 4:1, about 3:1, about 2: 1, about 1:1, about 1:2, about 1:3, about 1:4, about 1:5 or about 1:6 serine : threonine. Preferably, serine may be more abundant than threonine, e.g., a ratio between 2:1 and 4:3 serine : threonine (by number), preferably at a ratio of 4:3 serine : threonine (by number). In a preferred embodiment, the serine, threonine and proline residues may be present at a ratio 4:3:7. In certain embodiments, the N-terminal and C-terminal residues of the linker are both a serine residue; or the N-terminal and C-terminal residues of the linker are both proline residues; or the N-terminal residue is a serine residue and the C-terminal residue is a proline residue; or the N-terminal residue is a proline residue and the C-terminal residue is a threonine residue. In certain embodiments, the peptide linker may consist of only proline residues or D-isomers or analogues thereof, preferably of only proline residues. By means of examples and without limitation, peptide linkers as intended herein may comprise, consist essentially of or consists of an amino acid sequence as defined in SEQ ID NO 16: SPSTPPTPSPSTPP.

Polyethylene glycol (PEG) is a polymer compound that comprises repeating units of ethylene glycol, also known as polyethylene oxide (PEO) or polyoxyethylene (POE). PEGs contemplated for use herein are composed of linear ethylene oxide oligomer or polymer chains, as well as PEGs with branched, Y-shaped, or multi-arm geometries. The PEGs can have a wide variety of average molecular masses, such as for example average molecular mass, in particular number average Mw, in the range 190-210 Da (PEG 200), about 285-315 Da (PEG 300), about 380-420 Da (PEG 400), about 570-630 Da (PEG 600), about 855-900 Da (PEG 900), about 950-1050 Da (PEG 1000), about 1900-2200 Da (PEG 2000), about 2700-3300 Da (PEG 3000), about 3500-4500 Da (PEG 4000), or about 7000-9000 Da (PEG 8000).

PEG can be linked to cysteine by forming thioester bonds. Typically a PEG reactant containing a chemical group, such as maleimide and derivates thereof such as N-ethylmaleimide (NEM) and N-(2-aminoethyl)maleimide (AEM); haloacetamide and vinyl sulfone groups that can react with a thiol-group from cysteine to form a thioesther bond is used. Cysteine is an amino acid that contains a thiol group (-SH) on its side chain. When the PEG reactant containing a chemical group reacts with cysteine, a covalent bond is formed, resulting in the attachment of the PEG molecule to the cysteine residue.

A PEG reactant containing a chemical group, as mentioned above, can have a wide variety of average molecular masses, such as for example with an average molecular mass in the range of 1 to 100 kDa, about 1 to 80 kDA, about 1 to 70 kDa, about 1 to 60 kDa, about 1 to 50 kDA, about 1 to 40 kDA, about 1 to 30 kDA, about 1 to 20 kDa, about 1 to 10 kDA, preferably with an average molecular of 10 or 20 or 40kDa.

Moreover, PEGylation provides protection of the anti-NE ISVD against degradation by proteases. The PEGylated form of the anti-NE ISVD exhibits a significantly increased half-life in the lungs. This prolonged half-life provided by PEGylation allows for a lower frequency of treatment.

An advantage of this is that the anti-NE ISVD is stable at 4°C and even up to 37°C for at least 3 months without experiencing any loss in activity, which makes the agent suitable for storage and shipment without freezing, for example for extended storage with refrigeration (about 4°C) or without refrigeration at ambient temperatures (about 22 to 25°C).

The term "amino acid" encompasses naturally occurring amino acids, naturally encoded amino acids, non-naturally encoded amino acids, non-naturally occurring amino acids, amino acid analogues and amino acid mimetics that function in a manner similar to the naturally occurring amino acids, all in their D- and L-stereoisomers, provided their structure allows such stereoisomeric forms. Amino acids are referred to herein by either their name, their commonly known three letter symbols or by the one-letter symbols recommended by the IUPAC-IUB Biochemical Nomenclature Commission. A "naturally encoded amino acid" refers to an amino acid that is one of the 20 common amino acids or pyrrolysine, pyrroline-carboxy-lysine or selenocysteine. The 20 common amino acids are: Alanine (A or Ala), Cysteine (C or Cys), Aspartic acid (D or Asp), Glutamic acid (E or Glu), Phenylalanine (F or Phe), Glycine (G or Gly), Histidine (H or His), Isoleucine (I or Ile), Lysine (K or Lys), Leucine (L or Leu), Methionine (M or Met), Asparagine (N or Asn), Proline (P or Pro), Glutamine (Q or Gln), Arginine (R or Arg), Serine (S or Ser), Threonine (T or Thr), Valine (V or Val), Tryptophan (W or Trp), and Tyrosine (Y or Tyr). A "non-naturally encoded amino acid" refers to an amino acid that is not one of the 20 common amino acids or pyrrolysine, pyrroline-carboxy-lysine or selenocysteine. The term includes without limitation amino acids that occur by a modification (such as a post-translational modification) of a naturally encoded amino acid, but are not themselves naturally incorporated into a growing polypeptide chain by the translation complex, as exemplified without limitation by N-acetylglucosaminyl-L-serine, N-acetylglucosaminyl-L-threonine, and O-phosphotyrosine. Further examples of non-naturally encoded, un-natural or modified amino acids include 2-Aminoadipic acid, 3-Aminoadipic acid, beta-Alanine, beta-Aminopropionic acid, 2-Aminobutyric acid, 4-Aminobutyric acid, piperidinic acid, 6-Aminocaproic acid, 2-Aminoheptanoic acid, 2-Aminoisobutyric acid, 3-Aminoisobutyric acid, 2-Aminopimelic acid, 2,4 Diaminobutyric acid, Desmosine, 2,2'-Diaminopimelic acid, 2,3-Diaminopropionic acid, N-Ethylglycine, N-Ethylasparagine, homoserine, homocysteine, Hydroxylysine, allo-Hydroxylysine, 3-Hydroxyproline, 4-Hydroxyproline, Isodesmosine, allo-Isoleucine, N-Methylglycine, N-Methylisoleucine, 6-N-Methyllysine, N-Methylvaline, Norvaline, Norleucine, or Ornithine. Also included are amino acid analogues, in which one or more individual atoms have been replaced either with a different atom, an isotope of the same atom, or with a different functional group. Also included are un-natural amino acids and amino acid analogues described in Ellman et al. Methods Enzymol. 1991, vol. 202, 301-36. The incorporation of non-natural amino acids into proteins, polypeptides or peptides may be advantageous in a number of different ways. For example, D-amino acid-containing proteins, polypeptides or peptides exhibit increased stability *in vitro* or *in vivo* compared to L-amino acid-containing counterparts. More specifically, D-amino acid-containing proteins, polypeptides or peptides may be more resistant to endogenous peptidases and proteases, thereby providing improved bioavailability of the molecule and prolonged lifetimes *in vivo.*

The term "sequence identity" with regard to amino acid sequences denotes the extent of overall sequence identity (i.e., including the whole or entire amino acid sequences as recited in the comparison) expressed in % between the amino acid sequences read from N-terminus to C-terminus. Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62 (Henikoff et al., 1992, Proc. Natl. Acad. Sci., 89: 10915-10919), cost to open a gap = 11, cost to extend a gap = 1, expectation value = 10.0, word size = 3).

An example procedure to determine the percent identity between a particular amino acid sequence and a query amino acid sequence (e.g., the sequence of an anti-NE ISVD, or of anti-NE VHH, or of a CDR) will entail aligning the two amino acid sequences each read from N-terminus to C-terminus using the Blast 2 sequences (Bl2seq) algorithm, available as a web application or as a standalone executable programme (BLAST version 2.2.31+) at the NCBI web site (www.ncbi.nlm.nih.gov), using suitable algorithm parameters. An example of suitable algorithm parameters includes: matrix = Blosum62, cost to open a gap = 11, cost to extend a gap = 1, expectation value = 10.0, word size = 3). If the two compared sequences share identity, then the output will present those regions of identity as aligned sequences. If the two compared sequences do not share identity, then the output will not present aligned sequences. Once aligned, the number of matches will be determined by counting the number of positions where an identical amino acid residue is presented in both sequences. The percent identity is determined by dividing the number of matches by the length of the query sequence, followed by multiplying the resulting value by 100. The percent identity value may, but need not, be rounded to the nearest tenth. For example, 78.11, 78.12, 78.13, and 78.14 may be rounded down to 78.1, while 78.15, 78.16, 78.17, 78.18, and 78.19 may be rounded up to 78.2. It is further noted that the detailed view for each segment of alignment as outputted by Bl2seq already conveniently includes the percentage of identities.

Where an amino acid sequence differs, varies or diverges from a certain other amino acid sequence - for example, where the former amino acid sequence is said to display a certain degree or percentage of sequence identity to the latter amino acid sequence, or where the former amino acid sequence is said to differ by a certain number of amino acids from the latter amino acid sequence - such sequence variation may be constituted by one or more amino acid additions (e.g., a single amino acid or a stretch of two or more contiguous amino acids may be added at one position of an amino acid sequence or each independently at two or more positions of an amino acid sequence), deletions (e.g., a single amino acid or a stretch of two or more contiguous amino acids may be deleted at one position of an amino acid sequence or each independently at two or more positions of an amino acid sequence), and/or or substitutions (e.g., a single amino acid or a stretch of two or more contiguous amino acids may substitute a single one or a stretch of two or more contiguous amino acids at one position of an amino acid sequence or each independently at two or more positions of an amino acid sequence).

Preferably, the one or more amino acid substitutions, in particular one or more single amino acid substitutions, may be conservative amino acid substitutions. A conservative amino acid substitution is a substitution of one amino acid for another with similar characteristics. Conservative amino acid substitutions include substitutions within the following groups: valine, alanine and glycine; leucine, valine, and isoleucine; aspartic acid and glutamic acid; asparagine and glutamine; serine, cysteine, and threonine; lysine and arginine; and phenylalanine and tyrosine. The nonpolar hydrophobic amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (i.e., basic) amino acids include arginine, lysine and histidine. The negatively charged (i.e., acidic) amino acids include aspartic acid and glutamic acid. Any substitution of one member of the above-mentioned polar, basic, or acidic groups by another member of the same group can be deemed a conservative substitution. By contrast, a non-conservative substitution is a substitution of one amino acid for another with dissimilar characteristics.

A further aspect provides a nucleic acid molecule comprising a polynucleotide sequence encoding the binding agent as taught herein or a vector comprising the nucleic acid molecule, or the expression cassette or expression vector as defined herein.

A further aspect relates to an expression cassette or an expression vector comprising the nucleic acid molecule as defined herein and a promoter operably linked to the nucleic acid molecule. Preferably, the expression cassette or expression vector may be configured to effect expression of the nucleic acid molecule encoding for the anti-NE ISVD or functionally active variant or fragment thereof in a host cell. For example, in a bacterial cell, a fungal cell, including yeast cells, an animal cell, or a mammalian cell, including human cells and non-human mammalian cells. Preferably, the expression cassette or expression vector is configured to effect expression of the - nucleic acid molecule encoding for the anti-NE ISVD or functionally active variant or fragment thereof.

By "encoding" is particularly meant that a nucleic acid sequence or part(s) thereof corresponds to another nucleic acid sequence in a template - transcription product (e.g., RNA or RNA analogue) relationship, or corresponds, by virtue of the genetic code of an organism in question, to a particular amino acid sequence, e.g., the amino acid sequence of one or more desired proteins or polypeptides.

The terms "expression vector" or "vector" as used herein refers to nucleic acid molecules, typically DNA, to which nucleic acid fragments, preferably the recombinant nucleic acid molecule as defined herein, may be inserted and cloned, i.e., propagated. Hence, a vector will typically contain one or more unique restriction sites, and may be capable of autonomous replication in a defined host cell or vehicle organism such that the cloned sequence is reproducible. A vector may also preferably contain a selection marker, such as, e.g., an antibiotic resistance gene, to allow selection of recipient cells that contain the vector. Vectors may include, without limitation, plasmids, phagemids, bacteriophages, bacteriophage-derived vectors, PAC, BAC, linear nucleic acids, e.g., linear DNA, viral vectors, etc., as appropriate (see, e.g., Sambrook *et al.,* 1989; Ausubel 1992). Expression vectors are generally configured to allow for and/or effect the expression of nucleic acids or ORFs introduced thereto in a desired expression system, e.g., *in vitro,* in a host cell, host organ and/or host organism. For example, expression vectors may advantageously comprise suitable regulatory sequences.

Factors of importance in selecting a particular vector include *inter alia*: choice of recipient host cell, ease with which recipient cells that contain the vector may be recognised and selected from those recipient cells which do not contain the vector; the number of copies of the vector which are desired in particular recipient cells; whether it is desired for the vector to integrate into the chromosome or to remain extra-chromosomal in the recipient cells; and whether it is desirable to be able to "shuttle" the vector between recipient cells of different species.

Expression vectors can be autonomous or integrative. A recombinant nucleic acid can be introduced into the host cell in the form of an expression vector such as a plasmid, phage, transposon, cosmid or virus particle. The recombinant nucleic acid can be maintained extrachromosomally or it can be integrated into the cell chromosomal DNA. Expression vectors can contain selection marker genes encoding proteins required for cell viability under selected conditions (e.g., URA3, which encodes an enzyme necessary for uracil biosynthesis or TRP1, which encodes an enzyme required for tryptophan biosynthesis) to permit detection and/or selection of those cells transformed with the desired nucleic acids. Expression vectors can also include an autonomous replication sequence (ARS).

Integrative vectors generally include a serially arranged sequence of at least a first insertable DNA fragment, a selectable marker gene, and a second insertable DNA fragment. The first and second insertable DNA fragments are each about 200 (e.g., about 250, about 300, about 350, about 400, about 450, about 500, or about 1000 or more) nucleotides in length and have nucleotide sequences which are homologous to portions of the genomic DNA of the host cell species to be transformed. A nucleotide sequence containing a gene of interest for expression is inserted in this vector between the first and second insertable DNA fragments, whether before or after the marker gene. Integrative vectors can be linearized prior to transformation to facilitate the integration of the nucleotide sequence of interest into the host cell genome.

As used herein, the term "promoter" refers to a DNA sequence that enables a gene to be transcribed. A promoter is recognized by RNA polymerase, which then initiates transcription. Thus, a promoter contains a DNA sequence that is either bound directly by, or is involved in the recruitment, of RNA polymerase. A promoter sequence can also include "enhancer regions", which are one or more regions of DNA that can be bound with proteins (namely the trans-acting factors) to enhance transcription levels of genes in a gene-cluster. The enhancer, while typically at the 5' end of a coding region, can also be separate from a promoter sequence, e.g., can be within an intronic region of a gene or 3' to the coding region of the gene. Preferred promoters include, but are not limited to, promoters known *per se* for the expression in bacterial cells. Some preferred, but non-limiting promoters for use with these host cells include, for expression in *E. coli*: lac promoter (and derivatives thereof such as the lacUV5 promoter); arabinose promoter; left- and rightward promoter of phage lambda; promoter of the tryptophan (trp) operon; hybrid lac/trp promoters (tac); T7-promoter and other T-phage promoters; promoter of the Tn10 tetracycline resistance gene; engineered variants of the above promoters.

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence. Hence, "operably linked" may mean incorporated into a genetic construct so that expression control sequences, such as a promoter, effectively control expression of a coding sequence of interest, such as the nucleic acid molecule as defined herein.

The promotor may be a constitutive or inducible (conditional) promoter. A constitutive promoter is understood to be a promoter whose expression is constant under the standard culturing conditions. Inducible promoters are promoters that are responsive to one or more induction cues. For example, an inducible promoter can be chemically regulated (e.g., a promoter whose transcriptional activity is regulated by the presence or absence of a chemical inducing agent such as an alcohol, tetracycline, a steroid, a metal, or other small molecule) or physically regulated (e.g., a promoter whose transcriptional activity is regulated by the presence or absence of a physical inducer such as light or high or low temperatures). An inducible promoter can also be indirectly regulated by one or more transcription factors that are themselves directly regulated by chemical or physical cues.

Prior to introducing the vectors into a target cell of interest, the vectors can be grown (e.g., amplified) in bacterial cells such as *Escherichia coli* (*E. coli*). The vector DNA can be isolated from bacterial cells by any of the methods known in the art which result in the purification of vector DNA from the bacterial milieu. The purified vector DNA can be extracted extensively with phenol, chloroform, and ether, to ensure that no *E. coli* proteins are present in the plasmid DNA preparation, since these proteins can be toxic to mammalian cells.

It is understood that any genetically engineered modification as intended herein can also be conditional. For example, a gene can be conditionally deleted using, e.g., a site-specific DNA recombinase such as the Cre-loxP system (see, e.g., Gossen et al., 2002, Ann. Rev. Genetics, 36:153-173 and U.S. 20060014264).

The expression vector or cassette may further comprise one or more selection markers encoding proteins required for cell viability under selected conditions to permit detection and/or selection of those cells transformed with the desired nucleic acids, including any one or more genes needed for the production of leucine (e.g. LEU2), uracil (e.g. URA3), adenine (e.g. ADE2), Lysine (e.g. LYS5), Arginine, Tryptophan, or for glycerol utilization (Gut), and the hygromycin B phosphotransferase (hph) markers. Some preferred, but non-limiting examples of such selection markers are genes that provide resistance against antibiotics, such as ampicillin, chloramphenicol, tetracycline or kanamycin, genes that provide for temperature resistance, or genes that allow the host cell or host organism to be maintained in the absence of certain factors, compounds and/or (food) components in the medium that are essential for survival of the non-transformed cells or organisms.

The expression vector or expression cassette may be integrated into the genome of the host cell. For example, the expression vector or expression cassette may comprise a zeta element such as a long terminal repeat of a retrotransposon, such as without limitation, a Ylt1 or Tyl6 retrotransposon or others known to those skilled in the art. In one embodiment of the invention, the integration is targeted integration.

Alternatively, the expression vector or expression cassette may be replicative rather than integrated. For example, the replicative expression vector or expression cassette may comprise one or more autosomal replication sequences (ARS). The ARS may comprise a centromere (CEN) and an origin of replication (ORI). For example, the ARS may be ARS18 or ARS68.

The antibodies as taught herein can be formulated into pharmaceutical compositions. Therefore, any reference to the use of the antibodies in therapy (or any variation of such language) also subsumes such uses of pharmaceutical compositions comprising the antibodies.

In certain embodiments, the pharmaceutical composition may comprise the anti-NE ISVD, the nucleic acid molecule or vector encoding for the anti-NE ISVD, or the cell or virus comprising the anti-NE ISVD, and a pharmaceutically acceptable carrier, diluent, and/or excipient. In preferred embodiments the pharmaceutical composition comprises a further inhibitor of the NE.

The terms "pharmaceutical composition" and "pharmaceutical formulation" may be used interchangeably. The pharmaceutical compositions as taught herein may comprise in addition to the one or more actives (such as antibodies), one or more pharmaceutically or acceptable carriers. Suitable pharmaceutical excipients depend on the dosage form and identities of the active ingredients and can be selected by the skilled person (e.g., by reference to the Handbook of Pharmaceutical Excipients 7th Edition 2012, eds. Rowe et al.).

As used herein, the terms "carrier" or "excipient" are used interchangeably and broadly include any and all solvents, diluents, buffers (such as, e.g., neutral buffered saline, phosphate buffered saline, or optionally Tris-HCl, acetate or phosphate buffers), surfactants(such as, e.g., Tween® 80, Polysorbate 80), protein stabilizers (such as, e.g., sugars, polyols, polymers, cyclodextrins), colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives (such as, e.g., Thimerosal™, benzyl alcohol), antioxidants (such as, e.g., ascorbic acid, sodium metabisulfite), tonicity controlling agents, absorption delaying agents, adjuvants, bulking agents (such as, e.g., lactose, mannitol) and the like. The use of such media and agents for the formulation of pharmaceutical compositions is well known in the art. Acceptable diluents, carriers and excipients typically do not adversely affect a recipient's homeostasis (e.g., electrolyte balance). The use of such media and agents for pharmaceutical active substances is well known in the art. Such materials should be non-toxic and should not interfere with the activity of the actives. Acceptable carriers may include biocompatible, inert or bioabsorbable salts, buffering agents, oligo- or polysaccharides, polymers, viscosity-improving agents, preservatives and the like. One exemplary carrier is physiologic saline (0.15 M NaCl, pH 7.0 to 7.4). Another exemplary carrier is 50 mM sodium phosphate, 100 mM sodium chloride.

The precise nature of the carrier or other material will depend on the route of administration. For example, the pharmaceutical composition may be in the form of a parenterally acceptable aqueous solution, which is pyrogen-free and has suitable pH, isotonicity and stability. Preferably, the pH value of the pharmaceutical formulation is in the physiological pH range, such as particularly the pH of the formulation is between about 5 and about 9.5, more preferably between about 6 and about 8.5, even more preferably between about 7 and about 7.5.

Administration of the pharmaceutical composition can be systemic or local. Pharmaceutical compositions can be formulated such that they are suitable for parenteral and/or non-parenteral administration. Specific administration modalities include subcutaneous, intravenous, intramuscular, intraperitoneal, transdermal, intrathecal, oral, rectal, buccal, topical, nasal, ophthalmic, intra-articular, intra-arterial, sub-arachnoid, bronchial, lymphatic, vaginal, and intra-uterine administration.

Administration can be by periodic injections of a bolus of the pharmaceutical composition or can be uninterrupted or continuous by intravenous or intraperitoneal administration from a reservoir which is external (e.g., an IV bag) or internal (e.g., a bioerodable implant, a bioartificial organ, or a colony of implanted host cells). Administration of a pharmaceutical composition can be achieved using suitable delivery means such as: a pump, microencapsulation, continuous release polymer implants, macro encapsulation, injection, either subcutaneously, intravenously, intra-arterially, intramuscularly, or to other suitable site, or oral administration, in capsule, liquid, tablet, pill, or prolonged release formulation.

Examples of parenteral delivery systems include ethylene-vinyl acetate copolymer particles, osmotic pumps, implantable infusion systems, pump delivery, encapsulated cell delivery, liposomal delivery, needle-delivered injection, needle-less injection, nebulizer, aerosolizer, electroporation, and transdermal patch.

In certain embodiments, the pharmaceutical composition as taught herein may thus be configured for parenteral administration. Preferably, the pharmaceutical composition as taught herein may be configured for nasal or pulmonary or administration using suitable device such as: nebulization, inhalation dry powders or metered-dose inhalers.

In particular embodiments, a device comprises the pharmaceutical composition, which device can be a nebulizer or a metered-dose inhaler or a dry powder inhaler.

The quantity and nature of the pharmaceutical composition, as well as the duration of administration for a single dose, depend on the chosen delivery system. Various devices are known in the field for administration of the anti-NE ISVD via inhalation into the lungs of patients requiring such treatment. These devices include metered-dose inhalers, liquid nebulizers, dry powder inhalers, sprayers, thermal vaporizers, and similar devices. When utilizing certain suitable delivery system, such as nebulizers, the frequency of administration and activation duration will vary based on the concentration of the anti-NE ISVD in the aerosol powders. Higher concentrations of inhalation dry powders in the nebulizer solution can allow for shorter administration periods. Similarly, metered dose inhalers can generate higher aerosol concentrations, enabling shorter operation times to achieve the desired powder dosage. On the other hand, devices such as dry powder inhalers can deliver the anti-NE ISVD until a specific amount of the anti-NE ISVD is expelled. In this type of inhaler, the quantity of the anti-NE ISVD in a given quantity of the powder determines the dose delivered in a single administration.

The pharmaceutical composition comprising the anti-NE ISVD may optionally encompass an additive, such as a bulking agent, carrier, or excipient. Additives can be included in the dry powder to dilute the powder as required for delivery from the particular powder inhaler, to facilitate processing of the pharmaceutical composition, to provide advantageous powder properties to the pharmaceutical composition, to facilitate dispersion of the powder from the inhalation device, to stabilize the pharmaceutical composition (e.g., antioxidants or buffers), to provide taste to the pharmaceutical composition, or the like. Typical additives include mono-, di-, and polysaccharides; cellulose, cellulose derivatives, sugar alcohols such as sorbitol, lactose, glucose, raffinose, melezitose, lactitol, maltitol, trehalose, sucrose and mannitol, starch, polyvinylpyrrolidone, lipids and lipid-like excipients, such as diphosphatidyl choline, or lecithin; and amino acids, such as arginine, glycine, and leucine; or the like.

Administration of the present pharmaceutical composition to the respiratory tract by inhalation advantageously allows local delivery of the formulations, thereby maximizing the contribution of the anti-NE ISVD in the respiratory tract. Furthermore, by local administration of the pharmaceutical composition to the respiratory tract by inhalation, the administered dose of the anti-NE ISVD is not diluted in the systemic circulation, thereby significantly enhancing the therapeutic ratio.

One skilled in this art will recognise that the above description is illustrative rather than exhaustive. Indeed, many additional formulations techniques and pharmaceutically-acceptable excipients and carrier solutions are well-known to those skilled in the art, as is the development of suitable dosing and treatment regimens for using the particular compositions described herein in a variety of administration or treatment regimens.

The dosage or amount of the antibodies as taught herein, optionally in combination with one or more other active compounds to be administered, depends on the individual case and is, as is customary, to be adapted to the individual circumstances to achieve an optimum effect. Thus, the unit dose and regimen depend on the nature and the severity of the disorder to be treated, and also on factors such as the species of the subject, the sex, age, body weight, general health, diet, mode and time of administration, immune status, and individual responsiveness of the human or animal to be treated, efficacy, metabolic stability and duration of action of the compounds used, on whether the therapy is acute or chronic or prophylactic, or on whether other active compounds are administered in addition to the agent of the invention. Without limitation, depending on the type and severity of the disease, a typical dosage (e.g., a typical daily dosage or a typical intermittent dosage, e.g., a typical dosage for every two days, every three days, every four days, every five days, every six days, every week, every 1.5 weeks, every two weeks, every three weeks, every month, or other) of the molecules as taught herein may range from about 10 μg/kg to about 100 mg/kg body weight of the subject, per dose, depending on the factors mentioned above, e.g., may range from about 100 μg/kg to about 100 mg/kg body weight of the subject, per dose, or from about 200 μg/kg to about 75 mg/kg body weight of the subject, per dose, or from about 500 μg/kg to about 50 mg/kg body weight of the subject, per dose, or from about 1 mg/kg to about 25 mg/kg body weight of the subject, per dose, or from about 1 mg/kg to about 10 mg/kg body weight of the subject, per dose, e.g., may be about 100 μg/kg, about 200 μg/kg, about 300 μg/kg, about 400 μg/kg, about 500 μg/kg, about 600 μg/kg, about 700 μg/kg, about 800 μg/kg, about 900 μg/kg, about 1.0 mg/kg, about 2.0 mg/kg, about 5.0 mg/kg, about 10 mg/kg, about 15 mg/kg, about 20 mg/kg, about 30 mg/kg, about 40 mg/kg, about 50 mg/kg, about 75 mg/kg, or about 100 mg/kg body weight of the subject, per dose.

For certain pulmonary diseases, such as COPD and CF, several treatment approaches available including antibiotics, bronchodilators and anti-inflammatory medications. In pulmonary diseases associated with NE activity, antibiotics are often used to treat and prevent bacterial infections. Targeting the bacteria can help decrease the release of NE and subsequent lung damage. In CF, a newer class of medications designed to specifically target the defective cystic fibrosis transmembrane conductance regulator (CFTR) protein responsible for CF is also known. These medications operate by correcting the malfunctioning CFTR protein, thereby improving the function of chloride channels and restoring the balance of ion transport in affected cells. Examples of CFTR modulators include ivacaftor, lumacaftor/ivacaftor, tezacaftor/ivacaftor, and elexacaftor/tezacaftor/ivacaftor. Ivacaftor is used to treat certain CFTR gene mutations, while lumacaftor/ivacaftor, tezacaftor/ivacaftor, and elexacaftor/tezacaftor/ivacaftor are used for specific combinations of CFTR gene mutations. In certain embodiments the anti-NE ISVD can be combined with antibiotics, bronchodilators and anti-inflammatory medications as a treatment for pulmonary diseases targeting NE. In a particular embodiment, the anti-NE ISVD combined with CFTR modulators include ivacaftor, lumacaftor/ivacaftor, tezacaftor/ivacaftor, and elexacaftor/tezacaftor/ivacaftor to treat subject with a pulmonary disease such as CF.

A further aspect relates to a kit such as a diagnostic kit comprising the binding agent, the nucleic acid molecule or vector, or the cell or virus.

A further aspect relates to the binding agent, the nucleic acid molecule or vector, the cell or virus, or the pharmaceutical composition as taught herein, for use in medicine.

A certain embodiment relates to the binding agent, the nucleic acid molecule or vector, the cell or virus, or the pharmaceutical composition, for use in the prevention or treatment of an inflammatory disease in a subject.

The term "prevention", or its alternative forms such as "preventing" or "to prevent", commonly means to keep something from occurring, happening or existing, or to delay the occurrence or onset of something, especially by one or more precautionary (preventative) measures. For example, the present uses or methods may be applied to a subject not yet displaying one or more symptoms of an inflammatory disease, and may prevent, such as prevent for a given duration of time (delay), the onset or arrival of said one or more symptoms of an inflammatory disease.

As used throughout this specification, the terms "therapy" or "treatment" refer to the alleviation or measurable lessening of one or more symptoms or measurable markers of a pathological condition such as a disease or disorder. Measurable lessening includes any statistically significant decline in a measurable symptom or marker. Generally, the terms encompass both curative treatments and treatments directed to reduce symptoms and/or slow progression of the disease. The terms encompass both the therapeutic treatment of an already developed pathological condition, as well as prophylactic or preventative measures, wherein the aim is to prevent or lessen the chances of incidence of a pathological condition. Beneficial or desired clinical results include, but are not limited to, prevention of a disease, reduction of the incidence of a disease, alleviation of symptoms associated with a disease, diminishment of extent of a disease, stabilisation of the disease, delay or slowing of the progression of a disease, amelioration or palliation of a disease, or combinations thereof. In certain embodiments, the terms may relate to therapeutic treatments. In certain other embodiments, the terms may relate to preventative treatments. Treatment of a chronic pathological condition during the period of remission may also be deemed to constitute a therapeutic treatment. The term may encompass *ex vivo* or *in vivo* treatments as appropriate in the context of the present invention.

As used herein, the terms "subject" typically and preferably denotes humans, but may also encompass reference to non-human animals, preferably warm-blooded animals, even more preferably mammals, such as, e.g. non-human primates, rodents, canines, felines, equines, ovines, porcines, and the like. The term "non-human animals" includes all vertebrates, e.g., mammals, such as non-human primates (particularly higher primates), sheep, dog, rodent (e.g., mouse or rat), guinea pig, goat, pig, cat, rabbits, cows, and non-mammals such as chicken, amphibians, reptiles, etc. In certain embodiments, the subject is a mammal. In some embodiments, the subject is a non-human mammal. In some preferred embodiments of the methods and uses as taught herein, the subject is a human subject. In other embodiments, the subject is an experimental animal or animal substitute as a disease model. The term does not denote a particular age or sex. Thus, adult and newborn subjects, as well as foetuses, whether male or female, are intended to be covered.

A certain embodiment relates to the binding agent, the nucleic acid molecule, the vector, the cell or virus, or the pharmaceutical composition for use, wherein the inflammatory disease is a pulmonary disease, preferably a disease selected from the group consisting of a muco-obstructive lung disease such as, CF, COPD, bronchiectasis, ciliary dyskinesia and acute respiratory distress syndrome, inflammatory bowel disease.

The term "inflammatory disease" as used herein generally includes all diseases and conditions associated with inflammation, or in other words, which entail an inflammatory component. The inflammatory disease may entail acute inflammation, such as resulting from an injury or infection, or chronic inflammation. By means of example and not limitation, examples of inflammatory diseases are inflammatory lung disease (e.g. muco-obstructive lung disease such as, CF, COPD, emphysema, pulmonary fibrosis, bronchiectasis, ciliary dyskinesia and acute respiratory distress syndrome and non-muco-obstructive lung diseases such as pulmonary arterial hypertension), respiratory allergic diseases (e.g. asthma, allergic rhinitis, hypersensitivity lung diseases), pulmonary autoimmune diseases, tuberculosis, inflammatory nose and sinus diseases (e.g., chronic rhinosinusitis), inflammatory bowel disease, Crohn's disease, ulcerative colitis, inflammatory skin diseases (e.g. hidradenitis suppurativa).

Another aspect of the invention provides the binding agent, the nucleic acid molecule or vector, the cell or virus, or the kit for use in the diagnosis of an inflammatory disease in a subject.

A further aspect relates to a cell or a virus comprising the nucleic acid molecule or vector. optionally wherein the cell is capable of expressing or expresses the binding agent or the virus is configured to induce expression of the binding agent in a recipient cell infected with the virus.

Cells (host cells) that can be used for small or large scale production of the anti-NE ISVD include, without limitation, prokaryotic or eukaryotic cell. For example, a prokaryote such as a bacterial cell may be used. A host may be for example, an actinomycete such as a streptomyces species, e.g. *S. coelicolor* , for example *S. coelicolor* A3 (2), (strain M145 or M600), *S. lividans, S. cinnamoneous ,* or *E. coli* . Other examples may include other species of gram positive bacteria, such as those from the group Actinobacteria, for example, bacteria from the genus Mycobacterium , such as the pathogenic bacteria *Mycobacterium tuberculosis, M. bovis, M. africanum, and M. microti; M. leprae .* A further example of a genus of gram positive bacteria is Clostridium , which includes pathogenic bacteria such as *Clostridium difficile* (a human pathogen), *C. botulinum, C. perfingens and C. tetani* , as well as bacteria of potential industrial use such as *C. acetylbutylictum, C. thermocellum and C. ljungdahlii* . Other genera of gram positive bacteria may include Bacillus, Listeria, Staphylococcus, Clostridium, Corynebacterium, Streptococcus , and Enterococcus . Host cells may also be gram negative bacteria, which includes the genera Enterobacteriaceae which includes human pathogens, such as Salmonella and *Escherichia coli* . Other examples of genera of gram negative bacteria may include Pseudomonas, Bordetella, Borrelia, Brucella, Campylobacter, Francisella, Haemophillus, Klebsiella, Neisseria, Proteobacteria, Rickettsia, Vibrio, Yersina Moraxella, Helicobacter, Stenotrophomonas, Bdellovibrio , acetic acid bacteria, Legionella, the cyanobacteria, spirochaetes, green sulfur and green non-sulfur bacteria and many others. Important gram negative pathogens include the cocci species which cause a sexually transmitted disease (*Neisseria gonorrhoeae* ), a meningitis (*Neisseria meningitidis* ), and respiratory symptoms (*Moraxella catarrhalis* ). Other medically relevant Gram-negative bacilli include a multitude of species. Some of them primarily cause respiratory problems (*Hemophilus influenzae, Klebsiella pneumoniae, Legionella*

*pneumophila, Pseudomonas aeruginosa* ), primarily urinary problems (*Escherichia coli, Proteus mirabilis, Enterobacter cloacae, Serratia marcescens* ), and primarily gastrointestinal problems (*Helicobacter pylori, Salmonella enteritidis, Salmonella typhi* ). Host cells could also include eukaryotes such as yeasts (examples of which may include: those used for industrial production such as the genera Saccharomyces (such as *S. cerevisiae* ), Schizosacharromyces (*S. pombe* ) and the methyltrophic yeast genera Pichia [such as *P. pastoris* ] and Candida, also *Hansenula polymorpha* ; pathogenic yeasts such as the Candida genera [such as *C. albicans* and *C. tropicalis* ], also the Cryptococci genera [such as *C. neoformans* ), fungi (which may include: pathogenic fungi such as the genera Candida, Aspergillus [such as *A. fumigatus* and *A. flavus*], Cryptococcus, Histoplasma [such as *H. capsulatum*], Pneumocystis [such as *P. jirovecii* ] and Stachybotyrus [such as *S. chartarum* ]; fungi used in industrial production such as the genera Aspergillus [in particular *A. niger* and *A. oryzae* ]) and members of the Neurospora genus, such as *N. crassa* , plant cells and mammalian cells, avian cells, either in cell culture or part of a tissue. Bacterial host expression systems, such as *E. coli,* or yeast expression systems, such as *Pichia pastoris,* may be preferred.

As used herein, the term "virus" is interchangeable with terms "host virus" or "genetically engineered host virus" comprises a recombinant nucleic acid molecule or a vector encoding for the anti-NE ISVD according to any one of the different embodiments of the invention. The virus can be introduced into suitable host cells or a host organism which allows for the expression and preferably also the secretion of the anti-NE ISVD.

Another aspect of the invention provides a method for determining the level of active neutrophil elastase in a specimen, comprising contacting the specimen with the binding agent, and detecting at least the neutrophil elastase bound to the binding agent.

The anti-NE ISVD can be used for determining the level of active neutrophil elastase in a specimen. Both AAT and the anti-NE ISVD bind to the active site of NE. The binding of AAT to NE serves a crucial purpose by inhibiting NE's proteolytic activity, preventing the excessive breakdown of proteins. AAT achieves this inhibition by irreversibly binding to NE within its active site, blocking its proteolytic function. Once AAT forms a complex with NE, NE becomes unavailable for further interactions, including binding with anti-NE ISVD.

Consequently, anti-NE ISVD is unable to bind to NE molecules that are already complexed with AAT or optionally with other endogenous inhibitors such as Elafin, secretory leukocyte protease inhibitor (SLPI), $\alpha$1-anti-chymiotrypsin (ACT), $\alpha$2-macroglobulin, or monocyte neutrophil elastase inhibitor (MNEI or SerpinB 1 . Instead, anti-NE ISVD can only bind to active NE molecules that are uncomplexed with AAT or optionally with other endogenous inhibitors. NbE201 recognizes and binds to the unoccupied active sites of NE, regulating NE's activity. Therefore, the binding and inhibition of anti-NE ISVD depend on the availability of active NE molecules, as NE that is complexed with AAT is inaccessible to anti-NE ISVD. So anti-NE ISVD can be used in a method to determine the level of active neutrophil elastase by detecting the level of anti-NE ISVD that is bound to NE, wherein the level of the anti-NE ISVD complexed to NE is indicative of the level of uncomplexed, active NE in the specimen. This could provide information about the level of NE activity in the specimen.

Yet another further aspect relates to a method for the diagnosis, prognosis and/or monitoring of an inflammatory disease, optionally any inflammatory disease as described herein, in a subject, comprising:

- contacting a biological sample obtained from the subject with the binding agent and

- determining the level and/or activity of neutrophil elastase in the sample by detecting at least the neutrophil elastase bound to the binding agent.

As used herein, the term "contacting" also encompasses "exposing", "interacting" and generally refers to the act of bringing a binding agent into physical or functional contact with a sample or test material. When a binding agent is contacted with a specimen, it means that the binding agent is brought into direct or indirect contact with the sample under conditions generally conducive to antibody-antigen binding, for the purpose of detecting, identifying, or binding to specific antigens present in the specimen. More specifically, when a binding agent is contacted with a specimen, it means that the binding agent can contact the active neutrophil elastase for the purpose of detecting, identifying, or binding to the active NE present in the specimen.

In some methods of detection of the level of active NE protein, the binding agent as described herein can be used. Affinity-based assay methods, such as immunological assay methods, include without limitation immunohistochemistry, immunocytochemistry, flow cytometry, mass cytometry, fluorescence activated cell sorting (FACS), fluorescence microscopy, fluorescence based cell sorting using microfluidic systems, (immuno)affinity adsorption based techniques such as affinity chromatography, magnetic particle separation, magnetic activated cell sorting or bead based cell sorting using microfluidic systems, immunoprecipitation, enzyme-linked immunosorbent assay (ELISA) and ELISPOT based techniques, radioimmunoassay (RIA), Western blot, etc.

In further examples, such methods may include mass spectrometry analysis methods. Generally, any mass spectrometric (MS) techniques that are capable of obtaining precise information on the mass of peptides, and preferably also on fragmentation and/or (partial) amino acid sequence of selected peptides (e.g., in tandem mass spectrometry,

EP 4 483 951 A1

MS/MS; or in post source decay, TOF MS), may be useful herein for separation, detection and/or quantification of markers (such as, preferably, peptides, polypeptides, or proteins). Suitable peptide MS and MS/MS techniques and systems are well-known *per se* (see, e.g., Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)$^n$ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI-(MS)$^n$; atmospheric pressure photoionization mass spectrometry (APPI-MS); APPI-MS/MS; and APPI- (MS)$^n$. Peptide ion fragmentation in tandem MS (MS/MS) arrangements may be achieved using manners established in the art, such as, e.g., collision induced dissociation (CID). Detection and quantification of markers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods.

In other examples, such methods may include chromatography methods. The term "chromatography" encompasses methods for separating substances, such as chemical or biological substances, e.g., markers, such as preferably peptides, polypeptides, or proteins, referred to as such and vastly available in the art. In a preferred approach, chromatography refers to a process in which a mixture of substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, etc.

Chromatography may be preferably columnar (i.e., wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immunoaffinity, immobilized metal affinity chromatography, and the like.

Further techniques for separating, detecting and/or quantifying markers, such as preferably peptides, polypeptides, or proteins, may be used, optionally in conjunction with any of the above described analysis methods. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

In some embodiments, reagents such as binding agents (for example oligonucleotide primers) as taught herein may comprise a detectable label. The term "label" refers to any atom, molecule, moiety or biomolecule that may be used to provide a detectable and preferably quantifiable read-out or property, and that may be attached to or made part of an entity of interest, such as a binding agent. Labels may be suitably detectable by for example mass spectrometric, spectroscopic, optical, colorimetric, magnetic, photochemical, biochemical, immunochemical or chemical means. Labels include without limitation dyes; radiolabels such as $^{32}P$, $^{33}P$, $^{35}S$, $^{125}I$, $^{131}I$; electron-dense reagents; enzymes (e.g., horse-radish peroxidase or alkaline phosphatase as commonly used in immunoassays); binding moieties such as biotin-streptavidin; haptens such as digoxigenin; luminogenic, phosphorescent or fluorogenic moieties; mass tags; and fluorescent dyes alone or in combination with moieties that may suppress or shift emission spectra by fluorescence resonance energy transfer (FRET).

In some embodiments, binding agents may be provided with a tag that permits detection with another agent (e.g., with a probe binding partner). Such tags may be, for example, biotin, streptavidin, his-tag, myc tag, maltose, maltose binding protein or any other kind of tag known in the art that has a binding partner. Example of associations which may be utilized in the probe:binding partner arrangement may be any, and includes, for example biotin:streptavidin, his-tag:metal ion (e.g., $Ni^{2+}$), maltose maltose binding protein, etc.

The protein - binding agent conjugate may be associated with or attached to a detection agent to facilitate detection. Examples of detection agents include, but are not limited to, luminescent labels; colorimetric labels, such as dyes; fluorescent labels; or chemical labels, such as electroactive agents (e.g., ferrocyanide); enzymes; radioactive labels; or radiofrequency labels. The detection agent may be a particle. Examples of such particles include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles may be colloidal gold particles.

Similarly, it should be appreciated that in the description of illustrative embodiments of the invention, various features of the invention are sometimes grouped together in a single embodiment, figure, or description thereof for the purpose of streamlining the disclosure and aiding in the understanding of one or more of the various inventive aspects.

The present application also provides aspects and embodiments as set forth in the following Statements. In these statements, the wording "The binding agent according to Statement 1, wherein..." or "The binding agent according to any one of Statements 2 to 6, wherein..." also discloses and may be replaced by the simple wording "In certain embodiments... ".

Statement 1. A binding agent capable of specifically binding to and inhibiting a neutrophil elastase (NE), wherein the binding agent comprises an immunoglobulin single variable domain (ISVD) and the binding agent competes with Elastase Inhibitor 3 (EI3) for binding to NE.

Statement 2. A binding agent according to statement 1, wherein the binding agent comprises an immunoglobulin single variable domain (ISVD) binding to at least one of the residues R36, A60, N61, P96, V97, S195, G218 and G219, preferably two or more of the residues R36, A60, N61, P96, V97, S195, G218 and G219, more preferably three or more of the residues R36, A60, N61, P96, V97, S195, G218 and G219, more preferably four or more of the residues R36, A60, N61, P96, V97, S195, G218 and G219, more preferably five or more of the residues R36, A60, N61, P96, V97, S195, G218 and G219, more preferably six or more of the residues R36, A60, N61, P96, V97, S195, G218 and G219, more preferably seven or more of the residues R36, A60, N61, P96, V97, S195, G218 and G219, even more preferably all of the residues R36, A60, N61, P96, V97, S195, G218 and G219 of the NE.

Statement 3. A binding agent capable of specifically binding to and inhibiting a neutrophil elastase (NE), wherein the binding agent comprises an immunoglobulin single variable domain (ISVD) comprising a complementarity determining region 1 (CDR1) having the sequence set forth in SEQ ID NO: 1 (GRTISLYR), a CDR2 having the sequence set forth in SEQ ID NO:2 (INWSGDMT) and a CDR3 having the sequence set forth in SEQ ID NO:3 (TADPKLLPLADSSYGY).

Statement 4. A binding agent capable of specifically binding to and inhibiting a neutrophil elastase (NE), wherein the binding agent comprises an ISVD comprising a CDR1, CDR2 and CDR3, each as present in SEQ ID NO: 4 wherein the CDR1, CDR2 and CDR3 are annotated according to anyone of IMGT, Kabat, Chlotia, Martin or AHo numbering systems.

Statement 5. The binding agent according to statement 1 to 4, which comprises or consists of a VHH.

Statement 6. The binding agent according to any one of claims 1 to 5, wherein the ISVD comprises an amino acid sequence with at least 90% identity to the amino acid sequence set forth in SEQ ID NO:4

(QVQLQESGGGLVQAGGSLRLSCVVPGRTISLYRMGWFRQAPGKEREFVAGINWSGDMT DYVDSVKGRFTISRDNAKNTVYLEMNSLKPEDTAIYYCTADPKLLPLADSSYGYWGQGTQ VTVSS).

Statement 7. The binding agent according to any one of statement 1 to 6, wherein the ISVD comprises the amino acid sequence set forth in SEQ ID NO:4

(QVQLQESGGGLVQAGGSLRLSCVVPGRTISLYRMGWFRQAPGKEREFVAGINWSGDMT DYVDSVKGRFTISRDNAKNTVYLEMNSLKPEDTAIYYCTADPKLLPLADSSYGYWGQGTQ VTVSS).

Statement 8. The binding agent according to any one of statement 1 to 7, wherein the binding agent binds to at least a portion of the active site of the NE.

Statement 9. A nucleic acid molecule comprising a polynucleotide sequence encoding the binding agent according to any one of statement 1 to 8, or a vector comprising the nucleic acid molecule.

Statement 10. A cell or a virus comprising the nucleic acid molecule or vector according to statement 9, optionally wherein the cell is capable of expressing or expresses the binding agent or the virus is configured to induce expression of the binding agent in a recipient cell infected with the virus.

Statement 11. A pharmaceutical composition comprising the binding agent according to any one of statement 1 to 8, the nucleic acid molecule or vector according to statement 9, or the cell or virus according to statement 10, and a pharmaceutically acceptable carrier, diluent, and/or excipient, optionally wherein the pharmaceutical composition comprises a further inhibitor of the NE.

Statement 12. The pharmaceutical composition according to statement 11, wherein the pharmaceutical composition is suitable for nasal or pulmonary administration to a subject, optionally wherein the pharmaceutical composition is formulated as an inhalable dry powder, optionally wherein the pharmaceutical composition is configured for a nebulizer or a metered-dose inhaler.

Statement 13. Device comprising the pharmaceutical composition according to statement 11 or 12, wherein the device is a nebulizer or a metered-dose inhaler or a dry powder inhaler.

Statement 14. A kit such as a diagnostic kit comprising the binding agent according to any one of statement 1 to 8, the nucleic acid molecule or vector according to statement 9, or the cell or virus according to statement 10.

Statement 15. The binding agent according to any one of statement 1 to 8, the nucleic acid molecule or vector according to statement 9, the cell or virus according to statement 10, or the pharmaceutical composition according to statement 11, for use in medicine.

Statement 16. The binding agent according to any one of statement 1 to 8, the nucleic acid molecule or vector according to claim 9, the cell or virus according to statement 10, or the pharmaceutical composition according to statement 11, for use in the prevention or treatment of an inflammatory disease.

Statement 17. The binding agent, the nucleic acid molecule, the vector, the cell or virus, or the pharmaceutical composition for use according to statement 16, wherein the inflammatory disease is a pulmonary disease, preferably a disease selected from the group consisting of a muco-obstructive lung disease such as cystic fibrosis, chronic obstructive pulmonary disease (COPD), bronchiectasis, ciliary diskinesia and acute respiratory distress syndrome, inflammatory nose and sinus diseases, or the inflammatory disease is inflammatory bowel disease or the inflammatory disease is an inflammatory skin disease.

Statement 18. The binding agent according to any one of statement 1 to 8, the nucleic acid molecule or vector according to statement 9, the cell or virus according to statement 10, or the kit according to statement 14, for use in the diagnosis of an inflammatory disease in a subject.

Statement 19. A method for determining the level of active neutrophil elastase in a specimen, comprising contacting the specimen with the binding agent according to any one of statement 1 to 8, and detecting at least the neutrophil elastase bound to the binding agent.

Statement 20. A method for the diagnosis, prognosis and/or monitoring of an inflammatory disease, optionally any inflammatory disease as defined in statement 17, in a subject, comprising:

- contacting a biological sample obtained from the subject with the binding agent according to any one of statement 1 to 8, and

- determining the level of active neutrophil elastase in the sample by detecting at least the neutrophil elastase bound to the binding agent.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and scope of the appended claims.

The herein disclosed aspects and embodiments of the invention are further supported by the following non-limiting examples.

## EXAMPLES

**Example 1 - Materials and methods used in Example 2**

**1. Selection and *in vitro* characterization of the NbE201.**

**1.1. Animal immunization and construction of the immune libraries.**

An animal immunisation was performed on a lama (*Lama glama*) by six subcutaneous (s.c.) injections of 100 $\mu$g

of human sputum elastase (Elastin Products, United States) and Gerbu adjuvant, with an interval of one week between each injection. Four days after the last immunization, about 80 ml of blood was collected from the jugular vein and mixed with Ethylenediaminetetraacetic acid (EDTA) to prevent coagulation. The blood sample was centrifuged with Lymphoprep centrifugation tubes (Nycomed, Switzerland) to isolate the peripheral blood lymphocytes (PBLs). The immune libraries were constructed according to the protocol described by Pardon *et al* (A general protocol for the generation of Nanobodies for structural biology. Nat. Protoc. (2014) doi:10.1038/nprot.2014.039) and Vincke et al (Generation of single domain antibody fragments derived from camelids and generation of manifold constructs. Methods Mol Biol. 2012;907:145-76. doi: 10.1007/978-1-61779-974-7_8. Briefly, total RNA was isolated from PBLs with the RNeasy Plus Mini Kit (Qiagen, Germany). Then, 29 $\mu$g of total RNA was used as template to synthesize cDNA with Oligo(dT)12-18 Primer (Waltham, Massachusetts, USA). Next, the cDNA was amplified with the primers CALL001 (5'-GTC CTG GCT GCT CTT CTA CAA GG-3' (SEQ ID NO: 5)) and CALL002 (5'-GGT ACG TGC TGT TGA ACT GTT CC-3' (SEQ ID NO: 6)). These primers amplify the heavy chain antibody gene fragments from the variable region to the CH2 region. Amplification of this region results in two bands one of 900 base pairs (bp) that corresponds to the conventional antibodies and another one of 700 bp that corresponds to the variable region of the heavy chain (VHH or Nanobody or single-domain antibody (sdAb)). The variable region of the heavy chain was excised from the gel and DNA was extracted with NucleoSpin Gel and PCR Clean-up mini kit (Macherey-Nagel, Germany). After amplification with primers PMCF (5'-CTA GTG CGG CCG CTGA GGA GAC GGT GAC CTG GGT-3' (SEQ ID NO: 7)) and A6E (5'GAT GTG CAG CTG CAG GAG TCT GGR GGA GG-3' (SEQ ID NO: 8)), the ISVD genes were digested with the restriction enzymes BstEII and PstI and cloned into the pMES3 phagemid vector which contains a pelB signal coding sequence to address the ISVD into the periplasm of *E. coli*.

The library of phagemids was used to transform *Escherichia coli* TG1 electrocompetent cells (Lucigen, United States). The resultant transformants were collected in Luria-Berthani media (LB) supplemented with ampicillin (100$\mu$g/ml) and glycerol 10% (%v/v), and stored at -80°C. This constitutes the bacterial library. The percentage of clone having a phagemid coding for a ISVD in each library was calculated by colony polymerase chain reaction (PCR) carried out with 50 individual transformants randomly selected, with specific primers MP57 (5'- TTA TGC TTC CGG CTC GTA TG-3' (SEQ ID NO: 9)) and GIII (5'-CCA CAG ACA GCC CTC ATA G-3' (SEQ ID NO: 10)).

## 1.2. Selection of ISVDs specific of hNE by a directional sandwich phage display in solution.

*1.2.1. Amplification of phages.* An aliquot of 1 ml of the bacterial library was grown in 2XTY medium supplemented with ampicillin (100$\mu$g/ml) and glucose (0.1%, %w/v, final concentration) until the optical density (OD)$_{600nm}$ reached 0.8. Then, the cells were infected with the M13K07 phage helper (ThermoFisher Scienctific, United States) with a 20X multiplicity of infection as described previously (Pardon et al., Nat. Protoc. 2014, doi:10.1038/nprot.2014.039). The phage virions were recovered by polyethylene glycol-6000 precipitation and resuspended in sterile phosphate-buffered saline (PBS) pH 7.4. This constitutes the phage library. The concentration of phages was estimated by absorbance measurement at 260 nm.

*1.2.2. Directional sandwich phage display panning in solution.* A directional sandwich panning in solution was designed to select ISVDs specifically targeting the active site of hNE. Three rounds of panning were performed against hNE immobilized on the surface of streptavidin-coated magnetic Dynabeads M280 (ThermoFisher Scientific, United States) via a biotinylated capture ISVD, NbE34. NbE34 is an ISVD specific for hNE and does not bind into the active site of the enzyme. It was selected among a set of non-inhibitory ISVDs based of its capacity to expose the active site of hNE to the library of phage-exposed ISVD for a time long enough to carry out the panning procedure and therefore allow the enrichment of the library with inhibitory clones. NbE34 has a sequence set forth in SEQ ID NO: 15 (QVQLQESGGGL VQPGGSLRLSCAASGFTLDYHAIGWFRQAPGKEREGVSCISSSGGRADY ADSVKGRFTISRDNAKNTVYLQMNSLK PEDTAVYYCATRCAVVGGTYYYGMDYWGKGT QVTVSS).

The following sequence for NbE34 was used: QVQLQESGGGLVQPGGSLRLSCAASGFTLDYHAIGWFRQA PGKEREGVSCISSSGGRADY ADSVKGRFTISRDNAKNTVYLQMNSLKPEDTAVYYCATRCAVVGGTYYYGMDYWGK GT QVTVSSAAAYPYDVPDYGS (SEQ ID NO: 21) (this sequence includes the sequence of NbE34 of SEQ ID NO: 15 and HA tag). Hence, an *in vitro* method for panning hNE immobilized to a bead, using NbE34 is herein disclosed. The recovery of the beads after each step was carried out on a magnetic stand for 1.5 ml Eppendorf tubes for two minutes. All the incubation steps took place at room temperature and in a shaker with vertical rotation. Washing steps were carried out with 500 $\mu$l phosphate-buffered saline solution with a detergent solution of 0.01% (PBST, PBS with 0.01%, %v/v, Tween 20). First, 0.04 mg of beads were functionalized with 4 $\mu$g of chemically biotinylated NbE34 in 1 ml of PBS. After 5 washings, the beads were incubated with 5 $\mu$g of hNE in 500 $\mu$L of PBS. A negative control tube with magnetic beads functionalized with NbE34 but without hNE was also prepared. Then, the beads in each tube were incubated in 500 $\mu$l of blocking buffer. The blocking buffer was casein 0.1% (%w/v), bovine serum albumin 0.1% (%w/v) or protein free-blocking buffer (ThermoFisher Scientific, United States) in rounds 1, 2 and 3, respectively. In the next step, $2\times10^{11}$ phages from the library were incubated in the positive and the negative tubes and incubated for 1 hour. The beads were washed at least 10 times with PBST (PBS containing 0.05 %, %v/v, Tween 20), previous to the elution of phages with 200 ul of triethanolamine (TEA) pH 11 for 10 minutes and neutralization with 200 µl of trisaminomethane hydrochloric acid (Tris-HCl) 1M pH8. The resulting sub-repertoire of phages was subjected to reamplification in 15 ml of TG1 cells in the exponential phase of growth in LB medium supplemented with ampicillin (100 µg/ml) and glucose (0.2%, %w/v). The cells were then infected with 10 µl of phages solution (containing $10^7$ phages), incubated for 1 hour without agitation and centrifuged for 15 minutes at 3000xg. The pellet was resuspended in fresh 2xTYmedia supplemented with ampicillin (100 µg/ml) and kanamycin (70 µg/ml) and incubated at 37°C overnight with orbital agitation (250 rpm). Phage precipitation was performed as described above and used for the next round of panning. In each round of panning a subsample of 10 µl of eluted phages was used to infect 100 µl of TG1 cells that were subsequently diluted in series up to $10^7$ times dilution and plated on LB agar media supplemented with ampicillin (100 µg/ml) and glucose (0.2%, %w/v). The enrichment in phage exposing an ISVD specific of hNE was estimated by comparing of the number of colonies at a given dilution in the positive (containing hNE during the panning step) and the negative tubes (i.e., not containing hNE during the panning step).

### 1.3. Enzyme linked immunosorbent assay (ELISA) screening.

Ninety (90) colonies from each round of panning were randomly selected by an EasyPick Microlab STARlet Hamilton workstation (Lincoln, United States) at the Robotein platform at the Centre for Protein Engineering of the Liège University (http://www.robotein.ulg.ac.be). Each colony was inoculated in 100 µl of LB media supplemented with ampicillin (100 µg/ml), glucose (0.2%, %w/v) and glycerol (10%, %v/v) in 96-weels round-bottom plates (Greiner Bio-One, Belgium). 6 wells were filled only with media and were used as negative controls. The plate was incubated overnight at 37°C with agitation (220 rpm). The next day, 20 µl of each well were used to inoculate 1ml of 2xTYmedia containing ampicillin (100µg/mL) and glucose (1%, %w/v) in 96-deep-well plates and incubated for 3 hours under orbital agitation (220 rpm) at 37°C. Next, the production of ISVD was induced by adding isopropyl β-D-1-thiogalactopyranoside (IPTG) at 1 mM final concentration and the plates were incubated for 3 hours under orbital agitation (220 rpm) at 37°C. The plates were then centrifuged for 20 minutes at 3000g and the cytoplasmic extracts (CE) were obtained by a freezing and thawing cycle of the pellet at -80°C, prior to resuspension in 100 µl of sterile PBS and a short centrifugation. The CE of each clone were tested for hNE specificity by CE-ELISA. Briefly, 100 µl of hNE (1 µg/ml) were immobilized by adsorption at 4°C overnight on multisorp 96-well plates (ThermoFisher Scientific, United States). PBS was used as blank for each clone. After 5x washings with PBST 0.001% (%v/v), the wells were blocked with 200 µl of 1% (%w/v) non-fat dried milk blocking solution in PBS for 1hour at room temperature (RT) and incubated with 100 µl of 5-fold diluted CE (in PBS) for 1 hour at RT. The plates were then washed 10 times and bound ISVDs were revealed with a mouse anti-His antibody (BioLegend, United States) and a goat anti-mouse antibody conjugated with alkaline phosphatase (BioLegend, United States). Both antibodies were used diluted 1:2000 fold and incubated for 1 hour at RT. After each antibody, the plates were washed 10 times with PBST 0.001% (%v/v). The revelation was carried out with a solution of 2 mg/ml of p-Nitrophenyl phosphate (pNNP) in alkaline phosphate buffer (100mM Tris, 50 mM $MgCl_2$ $6H_2O$), 100 mM NaCl, pH 9.5). The absorbance at 405 nm was measured using the SpectraMax M2e Microplates Reader from Molecular Devices after 5-10-15-20-40 minutes. Clones were considered positive when the absorbance obtained was at least twice that obtained with their respective blank. Plasmidic DNA was isolated from positive single positive clones and sequenced using specific primer MP57 (5'-TTA TGC TTC CGG CTC GTA TG-3' (SEQ ID NO: 9)). ISVDs with a high similarity in their complementarity-determining regions 3 (CDR3) sequence were grouped into families. One ISVD of each family was selected for production and characterization (i.e. ability to inhibit hNE).

### 1.4. Production and purification of one ISVD of each family.

The phagemid coding for the ISVD of interest was used to transform chemo-competent *E. coli* WK6. The transformed cells were plated on LB-agar containing ampicillin (100 mg/L). One colony was picked to carry out a pre-culture in 100 mL of LB medium supplemented with ampicillin (100 mg·L$^{-1}$), overnight (ON) at 37°C. The preculture (5 mL) was used to seed 500 mL Terrific Broth medium supplemented with glucose (0.1% v/v) and ampicillin (100 mg/L). The bacterial growth was performed at 37°C until the absorbance at $600_{nm}$ ($A_{600nm}$) was comprised between 2 and 3. 1 mM IPTG (final concentration) was then added, and the culture was continued ON at 28°C with orbital agitation. The cells were harvested by centrifugation (5 min at 6000 g) and the periplasmic extract was prepared by an osmotic shock as described previously (Skerra, A. & Plückthun, Science, 1988, doi: 10.1126/science.3285470). The periplasmic extract was then filtered through a 0.45 µm cut-off membrane and loaded in on a 5 mL Immobilized Metal Affinity Chromatography (IMAC) NiPDC resin (Affilland, Liège, Belgium). The resin was washed with 50 mM imidazole in potassium phosphate buffer (KP buffer) 0.5 M pH 8 and then the Nb was eluted using 375 mM imidazole in KP buffer 0.5 M pH 8. The imidazole was removed using G25 Sephadex column (Sigma Aldrich) and sodium buffer (NaP buffer) 0.5 M pH 7 as running buffer. The purity and the integrity of ISVD was verified by Coomassie-stained SDS-PAGE, mass spectroscopy (ESI-Q-TOF) and SEC-MALS. The concentration was determined by UV absorption at 280 nm using the theoretical extinction coefficient calculated from the amino-acid sequence using Expasy prot-parameters.

**1.5. Production of NbE201, NbE201-C terminus and Nb-H7S-Nter-P1 in large amount.**

The gene of NbE201 was cloned into the pHEN6 vector using PstI and BstEII restriction sites. The vector obtained was used to transform chemo-competent *E. coli* WK6. NbE201-C terminus is the NbE201 fused to the following sequence (this sequence includes linker of SEQ ID NO: 16 and a C-terminal cysteine for conjugation to PEG-maleimide): SPSTPPTPSPSTPPC (SEQ ID NO: 22). Nb-H7S-Nter-P1 is a chimeric Nb consisting in the peptide P1 fused at the N-terminus of Nb-H7S which is specific to the β-lactamase BlaP (i.e., Nb-H7S does not bind to NE). P1 is composed by the inhibitory peptide sequence (β-hairpin) plus the β-stranded stalk derived from the bovine Ab BLV1H12 (Liu, T. et la., doi.org/10.1021/ja5130786). The sequence of P1 is: TSVHOETKK<u>MC*TASIPPOC*</u>YYNYEWHVDV (SEQ ID NO: 11), where C*-C* indicates a disulphide bridge; the sequence of the inhibitory peptide, MCTASIPPQCY (SEQ ID NO: 12), is underlined. NbE201-Cter and Nb-H7S-Nter-P1 were produced and purified following the same protocol as that described above using, for each 3L of Terrific Broth (TB) medium.

**1.6. Inhibition of hNE activity**

*1.6.1. Inhibition of the hydrolysis of a chromogenic substrate.* To determine the inhibitory capacity of NbE201, the hNE residual activity in the presence of the Nb at different Nb:hNE ratios was measured. hNE was incubated in the presence of the substrate N-Succinyl-Ala-Ala-Ala-paraNitroAnilide and with different concentrations of ISVD. Briefly, hNE (Elastin products company, EPC, United States) was resuspended at 1 mg·mL$^{-1}$ in 50 mM sodium acetate buffer pH 5 containing 100 mM NaCl and stored at 4°C. The substrate Succinyl-Ala-Ala-Ala-paraNitroAnilide was dissolved at 1 mM in 0.1 M Tris pH 7.5 with NaCl 0.5 M and NaN3 0.01% (%w/v) and stored at 4°C. 230 µl of substrate (1 mM) were mixed with 10 µl of Bovine Serum Albumin 9 µM (BSA, Sigma-Aldrich, Germany) and incubated 20 min at 25°C in a clear flat bottom 96-well plate (Greiner Bio-One, Belgium). Then, the ISVDs were added at a final concentration of 0, 10, 20, 30, 40, 50, 60, 70, 80, 90, 100, 125, 150 and 200 nM. Immediately after, the hNE was added to the mix at a final concentration of 50 nM. The absorbance at 410 nm was measured every min for 60 minutes in a SpectraMax M2e Microplates Reader (Molecular Devices, California, USA). The initial velocity determined in the presence of the ISVD at different Nb:hNE ratios was compared to that measured in the absence of the ISVD, to determine the residual activity. The delay between the addition of hNE and the first measurement was about 100 seconds. All the experiments were performed in triplicate for three independent lots of NbE201. In some experiments, the ISVD was preincubated for 15 minutes with the hNE and the complex was added to the substrate.

The same set-up was used to determine the inhibitory capacity of three other inhibitors of hNE to compare them with NbE201: alpha-1-anti-trypsin (AAT, Sigma-Aldrich, Germany), Elastase inhibitor 3 (EI3, or MeOSuc-Ala-Ala-Pro-Val-CMK, Calbiochem, Sigma-Aldrich, Germany) and Sivelestat (R&D systems, United Kingdom). Briefly, the substrate used was Succinyl-Ala-Ala-Ala-paraNitroAnilide (1 mM) in 0.1 M Tris pH 7.5 with NaCl 0.5 M and NaN3 0.01% (%w/v). hNE concentration used was 50 nM and different concentrations of the inhibitors were used to have different inhibitor:hNE molar ratios. The absorbance at 410 nm was measured every min for 60 minutes. The initial velocity determined in the presence of the inhibitor at different inhibitor:hNE ratios was compared to that measured in the absence of the inhibitor, to determine the residual activity. The deadline between the addition of hNE and the first measurement was about 100 seconds. Some hNE activity measurements in the absence and the presence of NbE201 were also carried out in quartz microcuvette. The substrate Succinyl-Ala-Ala-Ala-paraNitroAnilide 1 mM was incubated at 25°C for 20 minutes. Then, NbE201 was added at two different concentrations ($C_f$ = 40 nM and 75 nM), as well as hNE ($C_f$ = 50 nM) and mixed. The hNE activity was monitored at 410 nm for 10 minutes using the spectrophotometer Specord 50 Plus (Analytik Jena) and data were collected with the WinAspectPlus software. Measures were done in the presence of BSA ($C_f$ = 346 nM). The deadline between the addition of hNE and the first measurement was about 14 seconds.

The $K_m$ of hNE for Succinyl-Ala-Ala-Ala-paraNitroAnilide was determined by incubating hNE 50 nM with different substrate concentrations (12, 24, 48, 96, 192, 391, 586, 781, 1562, 2344 and 3125 mM) in the presence of BSA 346 nM. The reaction was monitored as described above. The initial rates of hydrolysis ($v_0$) were plotted as a function of [S] and linearized with the Hanes-Woolf method in order to derive the Km value.

*1.6.2. Inhibition of the hydrolysis of elastin, a natural substrate of hNE.* The hNE residual activity was measured in the presence of Elastin-CongoRed (Sigma Aldrich, Germany). hNE (132 nM) was incubated with a solution of Elastin-CongoRed (10 mg·mL$^{-1}$ in 0.1 M Tris pH 7.5, NaCl 0.5 M and NaN3 0.01%, %w/v) and with different concentrations of NbE201 or of other inhibitors (e.g. AAT, EI3 and Sivelestat). The samples were incubated for 16 hours at 37°C with orbital shaking (900 rpm). The samples were then centrifuged at 90'000 g for 30 minutes at 4°C to separate the insoluble Elastin from the digested and soluble molecule fragments. The soluble fraction was transferred into a 384-wells clear plate and the absorbance of Congo-red was measured at 495 nm in a SpectraMax M2e Microplates Reader (Molecular Devices, California, USA). The absorbance of the samples at different inhibitor:hNE molar ratios was compared to that measured in the absence of inhibitor, to determine the residual hNE activity. A sample of Elastin-Congo red without elastase was used as blank.

**1.7. Specificity of NbE201 for hNE.**

To determine whether NbE201 was specific for hNE, we first investigated its ability to inhibit several serine proteases: pancreatic elastase from pork (pPE, 39.2% sequence identity, from Sigma), mice neutrophil elastase (mNE, 74.9% sequence identity, from EPC) and with two other human serine proteases present in the lung: proteinase 3 (hPR3 from EPC, 54.7% sequence identity) and cathepsin G (hCG from Calbiochem, 36.7% sequence identity). The substrate used were Succinyl-Ala-Ala-Ala-paraNitroAnilide (1 mM in 0.1 M Tris pH 7.5 with NaCl 0.5 M and NaN3 0.01%, %w/v from Sigma-Aldrich) for pPE, MeOSuc-Ala-Ala-Pro-Val-AMC (100 $\mu$M in Tris 50 mM, NaCl 1 M, 0.05% (%w/v) Brij-35, pH 7.5) for mNE, N-Succinyl-Ala-Ala-Pro-Phe p-nitroanilide (0.45 mM in 100 mM HEPES pH 7.5, from Sigma Aldrich) for hCG and Boc-Ala-Ala-Nva-SBzl (0.22 mM in in 100 mM MOPS pH 5.0 0.5 M NaCl, 100 $\mu$M DTNB, DMSO 10%, from EPC) for PR3. mNE (rmELA2 from R&D systems, United Kingdom) was first activated according to the supplier information. In brief, mNE was incubated with recombinant mouse active cathepsin C/DPPIy (rmCathepsin C from R&D systems) in MES 50 mM, NaCl 50 mM, pH 5.5 for 2 h at 37°C. The enzyme concentration in the assay was 6 nM, 14.7 nM, 25 nM, 5 nM for PPE, mNE, hCG and hPR3, respectively. Different molar ratios of NbE201: Enzyme were tested. The hydrolysis of the substrate and the determination of the residual activity were carried out as explained above for the inhibition of hNE. The cross reactivity was also investigated by Biolayer Interferometry (BLI).

**1.8. Determination of the affinity of NbE201 for hNE and mNE by BLI experiments**

The affinity of NbE201 for hNE and mNE was measured by bio-layer interferometry (BLI) with the Octet HTX machine (Sartorius, Germany) of the Robotein platform of the Center of Protein Engineering (CIP) using various settings. For hNE, the biotinylated NbE201 was immobilized on streptavidin biosensors (SA, Sartorius) following the provider's indications. Then, the biosensors were immersed in a solution of hNE at different concentrations (40 - 20 - 10 - 5 - 2.5 nM). Association and dissociation kinetics were measured for 90 s and 600 s respectively, in kinetic buffer (KB buffer, Sartorius). All the experiments were carried out at 30°C in 384-wells black-bottom microplates in polypropylene (Greiner Bio-One, Belgium) and with orbital agitation (1500 rpm). The sensorgrams obtained were fitted to a 1:1 model to derive the rate constants for association ($k_a$) and dissociation ($k_d$) and the equilibrium dissociation constant ($K_D = k_d/k_a$). The measure was carried out in triplicate. For mNE, the NbE201 was immobilized on HIS 1K biosensors (Sartorius) via their His-tag following the provider's indications. Then, the biosensors were immersed in a solution of mNE at different concentrations (100 - 70 - 50 - 30 - 10 nM). Association and dissociation kinetics were measured for 300 s and 900 s respectively. All the experiments were carried out at 30°C in 384-wells black-bottom microplates in polypropylene (Greiner Bio-One, Belgium) and with orbital agitation (1000 rpm). The sensorgrams obtained were fitted to a 1:1 model to derive the rate constants for association ($k_a$) and dissociation ($k_d$) and the equilibrium dissociation constant ($K_D = k_d/k_a$). The measure was carried out with five independent replicates for hNE and three independent triplicates for mNE.

**1.9. Binding of NbE201 to other serine proteases.**

The specificity of NbE201 for hNE was also investigated by BLI. Biotinylated NbE201 (10 $\mu$g/mL) was immobilized on streptavidin biosensors (SA, Sartorius) following provider's indications. The biosensors were then immersed in a 50 nM solution of a serine protease. The proteases tested were human cathepsin G (hCG), human proteinase 3 (hPR3), mouse pancreatic elastase (mPE), mouse neutrophil elastase (mNE) and porcine pancreatic elastase (pPE). hNE was used a positive control. The association and dissociation were measured for 70 s and 320 s respectively, in kinetic buffer (KB, Sartorius). The experiments were carried out at 30°C in 384-wells black-bottom microplates in polypropylene (Greiner Bio-One, Belgium) and with orbital agitation (1000 rpm).

**1.10. Competitive binding of NbE201 with alpha1-antitrypsin (AAT), elastase inhibitor III**

**(EI3) and Nb-H7S-Nter-P1.** Competitive binding assays between NbE201 and hNE in complex with either (i) AAT, (ii) EI3 and (iii) Nb-H7S-Nter-P1 were also carried out by bio-layer interferometry (BLI) with the Octet HTX equipment. The biotinylated NbE201 (10 $\mu$g/ml in KB buffer) was immobilized on streptavidin biosensors (SA, Sartorius) following the provider's indications. A biosensor quenching step with biocytin 1 $\mu$g/mL was performed. Then, the biosensors were immersed in a hNE solution at 250 nM pre-incubated with or without saturating concentrations of one active site competitors (AAT 1.5 $\mu$M, EI3 15 $\mu$M or cAb-H7S-Nter-P1 1.5 $\mu$M). The association was measured for 280 s in kinetic buffer (KB buffer, Sartorius). The experiments were carried out at 30°C in 384-wells black-bottom microplates in polypropylene (Greiner Bio-One, Belgium) and with agitation (1000 rpm).

## 1.11. Thermodynamic stability of NbE201

*1.11.1. Preparation of the samples.* Samples of NbE201 were incubated overnight at 25°C in the presence of increasing concentrations of urea (Sigma-Aldrich) in 0.05 M NaP buffer pH 7. The protein concentration was 0.2 mg·mL$^{-1}$. The denaturant concentration in each sample was determined from refractive index measurements (Pace, C. N., 1986, doi.org/10.1016/0076-6879(86)31045-0), using a R5000 hand refractometer from Atago (Tokyo, Japan). The same samples were used to measure both the intrinsic fluorescence and far ultraviolet circular dichroism (UV-CD) spectra. Blank samples (without the Nb) every 1 M urea were prepared to make a reference curve that was used to correct the contribution of (buffer + urea) for fluorescence and circular dichroism (CD) signal at each urea concentration.

*1.11.2. Measurements of fluorescence and far-UV-CD signals spectra.* Intrinsic fluorescence spectra to monitor changes in the tertiary structure were recorded at 25°C from 305 nm to 440 nm after excitation at 280 nm with 5 nm excitation and emission slits using a Cary Eclipse Fluorimeter (Agilent, Santa Clara, USA) equipped with a thermostatically controlled multi-cuvette holder and a 10*0.4 mm pathlength quartz micro cuvette. The voltage applied at the detector was 710 V. Data acquisition was made every 0.5 nm with a scan rate of 30 nm·min$^{-1}$.

Far UV-CD spectrum of the native and denatured states (i.e. in 9 M urea) were recorded from 190 nm to 260 nm in a 0.2 mm pathlength quartz cuvette at 25°C. 606 data points were recorded with a 0.1 nm data interval, a data pitch of 0.1 nm, 2 s integration time, a bandwidth of 1 nm and a scanning speed of 50 nm/min. The unfolding transitions were monitored by far UV-CD at 207 nm in a 0.2 mm pathlength quartz cuvette. At each denaturant concentration, 37 data points were acquired with a 1 nm bandwidth, a reading frequency of 5 s and a 4 s integration time, and averaged. The measurements were carried out with Jasco J-810 spectropolarimeter, (JASCO, Lisses, France) equipped with a Peltier cell holder.

*1.11.3. Data analysis.* The intrinsic fluorescence spectra were fitted using a five parameter Weibull function (from software SigmaPlot 5.0) to determine the wavelength corresponding to the highest fluorescence intensity ($\lambda_{max}$). The changes in fluorescence intensity at 337 nm were also used to monitor the unfolding transition. The transition curves obtained were analyzed based on a two-state (N $\rightleftharpoons$ U) model according to Dumoulin et al. (doi: 10.1110/ps.34602) and equation 1:

$$y_{obs} = \{(Y_N + p\bullet[x]) + (Y_U + q \bullet [x]) \bullet \exp(-a)\} / (1 + \exp(-a)) \qquad (1)$$

where x is the concentration in denaturant and a = ($\Delta G°_{NU<H2O}$) - m * [D])/RT, and $y_{obs}$ is the monitored signal (i.e. $\lambda_{max}$, $F_{337nm}$ or $CD_{207nm}$) at a given denaturant concentration, and $Y_N$ and $Y_U$ are the values of these signals for the native and denatured states, respectively in the absence of denaturant. $\Delta G°_{NU(H2O)}$ is the difference in free energy between the folded and unfolded states at 25°C; m is a measure of the dependence of the free energy on the denaturant concentration, and [D] is the denaturant concentration. p and q are the slopes of the pre- and post-unfolding baselines, respectively, R is the gas constant, and T is the absolute temperature. $C_m$, the denaturant concentration at the midpoint of the denaturation curve ([U]/[N] = 1) is defined as $\Delta G°_{NU(H2O)}$/ m.

The transitions were normalized using the equation (2):

$$y = \{y_{obs} - (Y_N + p\bullet[x])\} / \{(Y_U + q \bullet [x]) - (Y_N + p\bullet[x])\} \quad (2)$$

## 1.12. Thermal stability of NbE201.

*1.12.1. Preparation of the samples and measurements.* Samples of NbE201 in 0.05 M NaP buffer pH 7 were prepared at a protein concentration of 0.2 mg·mL$^{-1}$. All the experiments were carried out in 1 mm quartz cell and a JASCO J-810 CD spectrophotometer equipped with a Peltier cell-holder. Mineral oil was added on the top of the sample to avoid sample evaporation. The CD spectrum of the native and denatured states of the protein were recorded from 190 nm to 260 nm at 25°C and at 97°C. 61 data points were recorded with a 1 nm data interval, a data pitch of 0.1 nm, 2 s integration time, a bandwidth of 1 nm and a scanning speed of 50 nm/min. Heat-induced unfolding was monitored by far UV-CD at 206 nm. The temperature was increased linearly from 25 °C to 97°C at a heating rate of 0.5 °C·min$^{-1}$, and data points were acquired every 0.3°C. The reversibility of the phenomenon was assayed by cooling the sample from 97°C to 25°C at the same rate. Data were acquired with a reading frequency of 0.05 s$^{-1}$ using a 4 s integration time and a 1 nm bandwidth. The temperature in the cuvette was monitor with a thermocouple (PT200 differential thermometer, IMPOT Electronics, Albertslund, Denmark).

*1.12.2. Data analysis.* The transition curves obtained were analyzed based on a two-state (N $\rightleftharpoons$ U) model and Equation 1 where x is the temperature during the analysis and a = -($\Delta H_m$ (1-x/$T_m$))/RT (doi: 10.1016/j.jmb.2008.11.046), and $y_{obs}$ is the CD signal at 206 nm at a given temperature (x), and $Y_N$ and $Y_U$ are the values of these signals for the native and denatured states, respectively at 273K. p and q are the slopes of the pre- and post-transition baselines, respectively, R is the gas constant, and T is the absolute temperature. $T_m$ is the temperature of mid-transition and $\Delta H_m$ is the enthalpy value at $T_m$. Since the transitions were not fully reversible, only the Tm which is referred to as apparent $T_m$ ($T_m{}^{app}$), was considered. The transitions were normalized using the equation 2.

**1.13. Stability of NbE201 towards hNE.**

NbE201 (26.4 $\mu$M) was incubated at 37°C alone or in the presence of hNE ([NbE201]: [hNE]=4:1). Aliquots (10 $\mu$L) taken at different time intervals were mixed with 4 $\mu$L of sample loading buffer (Tris-HCl (pH 6.8), SDS denaturant (4%, %w/v), $\beta$-mercaptoethanol (5%, %v/v), bromophenol blue (0.01%, %w/v), glycerol (20%, %v/v), H2O)) and then stored at -20°C until analysis by SDS-PAGE using 4-20% Mini-PROTEAN TGX Protein Gel, 10 wells, 50 $\mu$L (Bio-Rad, Germany). Then, the gel was stained by Coomassie blue, destained in water and imaged using a Gel Doc EZ Imager (Biorad). The relative amount of intact protein was quantified by densitometry analysis with a Gel Doc EZ Imager program using the intensity of the band at time zero as the relative standard. hNE alone was incubated on similar conditions and an aliquot was taken at different time points to measure its activity using N-Succinyl-Ala-Ala-Ala-paraNitroAnilide as substrate as describe above.

**1.14. Crystallographic structure of NbE201 alone and in complex with hNE.**

*1.14.1. Conditions of crystallization of the complex:* The crystals were grown at 20°C using the sitting drop vapor diffusion method. The drop contained 0.2 $\mu$L of hNE in complex with NbE201 at a concentration of 15 mg·mL$^{-1}$ and 0.2 $\mu$L of 0.1M HEPES pH7 buffer with 10% polyethylene glycol 5000 monomethyl ether and 5% tacsimate. The crystals were transferred to a cryoprotectant solution containing 33% (%v/v) polyethylene glycol 6000 and 33% (%v/v) glycerol before being frozen in liquid nitrogen.

*1.14.2. Conditions of crystallization of NbE20 1 alone:* The crystals were grown at 20°C using the sitting drop vapor diffusion method. The drop contained 0.2 $\mu$L of NbE201 at a concentration of 15 mg·mL$^{-1}$ and 0.2 $\mu$L of PBS buffer pH 7.4, 10 mM Na$_2$HPO$_4$, 1.8 mM KH$_2$PO$_4$, 137 mM NaCl, 2.7 mM KCl, in a cryo-protectant solution of 0.2 M Ca Acetate, 0. 1M Na Cacodylate pH 6.5 with 18% (%v/v) polyethylene glycol 8000 et 33% (%v/v) glycerol.

*1.14.3. Data collection, phasing, model construction and refinement for both structures.* Diffraction data were collected with the Proxima 1 beamline of the Soleil synchrotron (Saint Aubin, France). Indexing, integration and scaling of the data were carried out with the XDS Program Package (Kabsch W., 2010, doi.org/10.1107/S0907444909047337). The structure of hNE alone (PDB 3Q76) and/or the structure of the ISVD specific of the mouse Vsig4 protein (PDB 5IMO) were used to obtain the initial phases by molecular replacement using the Phaser Crystallographic program (McCoy AJ, et al., 2007, doi.org/10.1107/S0021889807021206). The structures were built using the Coot program (Crystallographic Object-Oriented Toolkit) (Emsley P. et al., 2010, doi.org/10.1107/S0907444910007493) and refined with BUSTER -TNT (Blanc E. et al., 2004, doi: 10.1107/S0907444904016427). Figures were prepared using PyMOL (The PyMOL Molecular Graphics System, Version 2.4.1 Enhanced for Mac OS X, Schrödinger, LLC.).

**2. PEGylation of NbE201, stability towards different stresses, Inhibition of hNE in sputa of CF and COPD patients and pharmaco-kinetics.**

**2.1. PEGylation of NbE201 and purification**

A 0.063 mM solution of NbE201-Cter was exposed to 1mM of tris(2-carboxyethyl)phosphine (TCEP) linked to agarose beads (Merck, Kenilworth, USA), and 0.252 mM linear 10 or 20 kDa polyethylene glycol (PEG)-malemimide or branched 40 kDa PEG-maleimide (Nof Corporation, Tokyo, Japan) at RT on an agitating plate at 150 rpm in a 2 mM EDTA (Merck), 50 mM sodium phosphate buffer at pH 7 during 2h30. The solution was then centrifuged for 1 min at 1000 rpm to precipitate the agarose beads and the supernatant was harvested. The PEGylated products were purified via Fast Protein Liquid Chromatography (FPLC) (Akta purifier 10, Cytiva, Chicago, USA) by using a cation exchange column (Resource S, 1ml, Cytiva). The primary buffer was a 20 mM succinic acid, 5 mM NaCl solution at a pH of 4.6 and the elution buffer was a 20 mM succinic acid, 350 mM NaCl solution at a pH of 4.6. The solution was dialyzed to the primary buffer (3kDa MWCO membrane, Carl Roth, Karlsruhe, Germany) and was filtered on a 0.2 $\mu$m polyvinylidene difluoride (PVDF) filter (Carl Roth). The solution was injected in the column and eluted with a 20 ml gradient from 0 to 100% of the elution buffer (3ml/min). The purified PEGylation products were harvested via a Frac-920 system (Cytiva). Samples were then transferred to their initial sodium phosphate buffer (50mM, pH7) and concentrated to a desired concentration by the use of ultrafiltration tubes (Vivaspin Turbo 3 kDa, Sartorius).

**2.2 hNE enzymatic activity assessment and inhibition.**

*2.2.1 Evaluation of the proteolytic activity inhibition on purified hNE.* hNE activity was assessed by following the degradation of a chromogenic *hNE substrate,* N-succinyl-alanine-alanine-proline-valine-p-nitroanilide (suc-*Ala-Ala*-Pro-Val-pNA, Elastin products, Owensville, Missouri, USA), measuring the absorbance at 410 nm with a spectrophotometer (Spectramax ID5, Molecular Devices, San Jose, USA). The substrate was diluted to a concentration of 1.5 mM in a tris buffer (0.1M tris, 0.5M NaCl, 0.01% NaN3, pH 7.5) and 200 $\mu$l of this solution were added to the wells of a 96-well plate and heated at 37°C. Purified hNE (Elastin products) was reconstituted on ice in a 0.05M Sodium acetate (NaOAc) , 0.1M NaCl buffer at pH5 to a concentration of 0.1 mg/ml. 10 $\mu$l of this hNE solution were added per well and the substrate degradation was followed during 20 min to verify the hNE activity in the wells. ISVDswere then added to the wells at the desired hNE: ISVD ratio, and the substrate degradation was then followed during 50 min. Slopes were calculated from the regression line drawn from the absorbance measurements and were expressed as a percentage of the hNE activity without ISVDs.

*2.2.2 Evaluation of the proteolytic activity inhibition in CF, COPD and ciliary diskenisia sputa.* Sputa from patients with cystic fibrosis, COPD or ciliary diskenisia above 18 years old were collected during a monthly kinesitherapy consultation and did not require specific induction. Sputa collection procedures were evaluated by the local ethic committee (comité d'Ethique Hospitalo-Facultaire des Cliniques universitaires Saint-Luc) and approved under the Belgian registration number B4032021000071.

Since hNE induces damages in different lung pathologies characterized with chronic inflammation, other diseases such as COPD, AAT deficiency, severe asthma, bronchiectasis and infiltrative pulmonary diseases could benefit from a treatment with NbE201. Therefore, NbE201(-PEG10/40) inhibitory capacity was assessed in CF, ciliary diskenisia and COPD patient sputa.

Sputum proteolytic activity was assessed by following with a spectrophotometer the absorbance at 410 nm of suc-*Ala-Ala*-Pro-Val-pNA degradation, the chromogenic *hNE substrate.* Sputa were classified as mucoid, muco-purulent or purulent (Serisier et al., 2009, Respir Res, 10(1):63. doi: 10.1186/1465-9921-10-6). According to the experiment, 10 to 20 mg of sputum were weighted in a 96-well plate. ISVDs were then added to the sputum samples and pre-incubated at 37°C during different periods of time. The hNE substrate was diluted to a concentration of 1 mM in a tris buffer (0.1M tris, 0.5M NaCl, 0.01% Sodium azide (NaN$_3$), pH 7.5) and 50 $\mu$l of this solution were added to the wells. The substrate degradation was followed during 50 min. To estimate the elastase activity in each sputum, various amounts of sputum ranging from 1 to 5 mg were weighted in a 96-well plate and their activity was compared to the activity of an hNE standard curve, where increasing quantities from 2 to 20 $\mu$l of the hNE solution described in 2.2.1 were added to the substrate int the wells without Nbs. Slopes were then calculated from the regression line drawn from the absorbance measurements and were expressed as a percentage of the sputum proteolytic activity without ISVDs.

From these inhibition experiments, IC$_{50}$ values after exposure of the NbE201 to the sputa at the different time points studied (0, 1, 4 and 24h) will be obtained and compared to the ones obtained after exposure to NbE201-PEG10, NbE201-PEG40 and AAT (Bio-Techne, Minneapolis, USA).

**2.3 Stability of NbE201 and its PEGylated counterparts towards physical stress application.**

A 0.25 mg/ml solution of NbE201, NbE201-PEG10 or NbE201-PEG40 was prepared and divided in order to apply the following conditions: control, 1 or 4h of agitation with a magnetic bar, nebulization, 5 or 10 freeze/thaw cycles.

*2.3.1 Stability towards agitation.* 100 $\mu$l of a 0.25mg/ml ISVDs solution were agitated in a 1ml glass vial with a 2x5mm magnetic bar (VWR, Radnor, USA) at 200 rpm during 1 or 4 hours.

*2.3.2 Stability towards Nebulization.* 300 $\mu$l of a 0.25mg/ml ISVDs solution were nebulized with a MeSH nebulizer (InnoSpire Go, Phillips, Amsterdam, The Netherlands).

*2.3.3 Stability towards freezing/thawing.* 100 $\mu$l of a 0.25mg/ml ISVDs solution underwent 5 or 10 freeze/thaw (F/T) cycles. Freezing was performed in a -20°C freezer and thawing was performed at RT.

*2.3.4 Concentration measurements.* Concentrations and UV spectra were measured by spectrophotometry (NanoDrop 2000/2000c spectrophotometer, ThermoFisher). Concentrations were determined based on the absorbance at 280nm. NbE201 and NbE201-Cter mass extinction coefficients applied to the measurements were 1.888 and 1.716 (mg/mL)$^{-1}$ cm$^{-1}$ respectively. Regarding the ISVDs exposed to physical stresses, the concentration was read before and after centrifugation at 10 000g at 4°C for 10 min.

*2.3.5 Aggregates Detection.* Protein aggregates were visualized by Nanoparticle Tracking Analysis (NTA) (ZetaView, 220 Twin Laser, Particle Metrix, Ammersee, Germany), a technique used for measuring single particle size and concentration. NbE201, NbE201-PEG10 and NbE201-PEG40 solutions (0.25mg/ml) were analysed before and after nebulization. 11 positions were analysed for which following parameters were used:

- Laser wavelength: 488 nm

- Scatter, no fluorescence

- Sensitivity: 80

- Shutter: 100

### 2.4 Dry powders formulation by spray drying

Dry powders for inhalation were formulated with the NbE201 and the NbE201-PEG40. The spray dryer used was the PROCEPT's modular 4M8-Trix. The composition of the powders was 65% of trehalose dihydrate, 15% trileucine, 9.8% phosphate buffer, 0.2% rhodamine b, 10% NbE201 or 37% of trehalose dihydrate, 15% trileucine, 9.8% phosphate buffer, 0.2% rhodamine b, 38% NbE201-PEG40 (10% NbE201 moiety and 28% PEG moiety). The following spray drying conditions were used:

- Flow rate: 10 ml/min

- Compressed air: 1.1 bar

- Inlet temperature: 145°C

- Outlet temperature: 51.1°C

- Relative humidity: 32%

- Nozzle size: 1.2 mm

### 2.5 Pharmacokinetic study.

*2.5.1. Instillation of mice and collection of BAL.* One nanomole of NbE201, NbE201-PEG40 or AAT (Bio-Techne) was intratracheally instilled in a final volume of 25µl in transgenic Tg mice and in β-ENaC over-expressing Tg mice. Broncho-alveolar lavages (BAL), nasal lavages (NAL), blood samples, and lungs were collected 0, 4 or 24h after instillation. BAL and NAL were collected with 2 ml and 1 ml of Hanks' Balanced Salt Solution (HBSS) buffer respectively. Cells were discarded from BAL and NAL samples via a 5 min centrifugation at 300 g and supernatants were stored at -20°C. Lungs were collected in 0.5 ml of HBSS buffer, ground (Precellys evolution, Bertin Technologies, Montigny-le-Bretonneux, France) and centrifuged at 300g for 15 min. Supernatants were stored at - 20°C. Blood was collected by intra-cardiac puncture and stored in anti-coagulant tubes (Microvette® 500, K3 EDTA, VWR) at -20°C. All samples were treated with an anti-protease cocktail (Roche).

The β-ENaC murine model mimics the cystic fibrosis lung pathology, and is the most representative murine model available since cystic fibrosis transmembrane conductance regulator gene (cftr) (-/-) knockout mice do not develop the lung disease associated with cystic fibrosis (Clarke et al., 1992, Sciences, 257(5073):1125-8. doi: 10.1126/science.257.5073.1125). It will allow the assessment of the effects of a local chronic inflammation and of the presence of a thick mucus in the respiratory tract on the studied compounds pharmacokinetics.

*2.5.2. ELISA analysis.* ISVD and AAT content in the BALs collected *in vivo* were measured by a sandwich ELISA method. An ELISA conversion pack from Meso scale Discovery (Rockville, USA) was used for ISVD analysis, according to the manufacturer instructions. Uncoated Mesoscale compatible plates were coated with an anti-histag antibody (BioLegend, San Diego, USA). ISVDs were then detected with an anti-VHH antibody (Jackson ImmunoResearch, Ely, UK) mixed with an anti-goat Sulfo-TAG labelled antibody (Meso scale Discovery) and the signal was read with an MESO QuickPlex SQ 120MM (Meso scale Discovery). AAT content in the samples was measured by a commercial ELISA kit (Human Serpin A1 DuoSet ELISA, R&D system, Mineapolis, USA) according to manufacturer instructions.

### 3. Protection activity of NbE201 against development of hNE-induced acute lung injury syndrome in mice.

### 3.1. Animal protocol

The *in vivo* efficacy of NbE201 was determined using a mouse model of acute lung injury induced by nasal instillation of HNE ( L agente et al., 2009, American Thoracic Society International Conference Abstracts. (American Thoracic Society), A5668. doi: 10.1164/ajrccm-conference. 2009.179.1_MeetingAbstracts.A5668). To this goal, female mice C3H x C57BL/6 (wild-type littermates of the Cystic Fibrosis (CF) like mouse model overexpressing the β-subunit of the ENaC protein (Mall et al., 2004, Nat. Med. 10, 487-493. doi:10.1038/nm1028) were anesthetized with xylazine/ketamine (10/100 mg/kg, 0.1 ml/10g) by intraperitoneal injection and were nasally instilled with 15 $\mu$L of hNE solution (Elastin Products Company,USA; 25 $\mu$g/mouse = 0.8 nmol/mouse) or with 15 $\mu$L of saline solution (NaCl 0.9%). Fifteen minutes prior hNE treatment, a nasal instillation (30 $\mu$L) of Nb (molar ratio Nb/HNE 4/1 nmol) or NaCl 0.9% was performed. Four hours after hNE instillation, mice were sacrificed by intraperitoneal injection of pentobarbital solution (200 mg/kg, 0.1 mL/10g).

### 3.2. Bronchoalveolar lavage (BAL)

The trachea was exposed and clamped. The left lung was also clamped to allow simultaneous pathological analysis of a non-lavaged lung. BAL fluid was collected by washing three times the lung with 1 mL of sterile saline solution (NaCl 0.9%). After centrifugation (10 min at 1500 rpm, 4°C), total cells were resuspended in 200 $\mu$L of saline and counted with Turk solution in Neubauer chamber. Cytospin (5 min at 900 rpm) from BAL cells were made and cells were stained with Diff-Quick Kit. The percentages of macrophages, eosinophils, lymphocytes, and neutrophils in the BAL fluid were obtained by counting 200 cells with regard to their morphological characteristics. The supernatants of BAL fluid were aliquoted and frozen at -80 °C for further analysis.

### 3.3. Morphological analysis

Non-lavaged lungs were fixed in 4% paraformaldehyde and embedded in paraffin. Sections were cut at a thickness of 5 $\mu$m using a microtome, and deparaffinized tissue sections were stained with hematoxylin and eosin using standard histological techniques. Pathology studies were used to characterize the status of inflammatory responses and the degree of tissue damage. The intensities of cell infiltrate and tissue damage were scored to allow between-group comparisons and to quantify the beneficial, protective effect of the NbE201 treatment.

### 3.4. Pro-inflammatory cytokines and chemokines

The concentrations of C-C motif chemokine ligand 2 (CCL2), tumor necrosis factor-alpha (TNF-$\alpha$), interleukin 1 beta (IL-1$\beta$), interleukin 6 (IL-6) and keratinocytes-derived chemokine (KC) in BAL fluids were measured using an ELISA kit (R&D Systems). Analyses were performed according to manufacturer's instructions. Between-group data comparisons allowed evaluating the degree of antiinflammatory protection brought by the pretreatment of animals with NbE201.

### 3.5 BAL hemoglobin concentration

It was determined with the hemoglobin assay kit (Sigma) and performed according to the manufacturer's instructions. This kit is based on the Triton/NaOH method, which consists of the conversion of hemoglobin into hematin complexed with Triton. A measurement of the optical density at 400 nm allowed the quantification of this stable complex. Between-group data comparisons allowed evaluating the magnitude of release of hemoglobin in the bronchoalveolar lumen induced by instillation of hNE and the protective effect of the pretreatment of mice with NbE201.

### 3.6 Alveolar-capillary damage quantification

Histological sections of the lung were digitized using a panoramic scan 2 (3DHISTECH). An image analysis was then performed with the Visiopharm® software (v2022.01.3). This semi-automated analysis made it possible to quantify the damaged alveolar surface. These lesions are characterized by the presence of red blood cells in the alveolar spaces. To this goal, several steps were used (**Figure 3A**). The percentage of injured surface was determined by the ratio: % injured area = (injured area / alveolar area) x 100

### 3.7 Statistical analyses

Data were expressed as means $\pm$ standard deviation (SD) (GraphPad Prism, v9). Statistical comparisons were performed using a Kruskal-Wallis test and Dunns' post-test for multiple comparison. A p value of less than 0.05 was considered significant.

### Example 1 - Selection of NbE201

The size of the immune library constructed from the blood of the lama immunized with hNE was $4.6 \times 10^7$ and the percentage of clone containing a plasmid with a Nb gene was 75%. The gene of 91 positive clones from the CE-ELISA were sequenced. Based on the sequence of their CDR3, the ISVD with unique sequence could be classified in 9 families, as shown in Table 1.

Table 1: Families of ISVD selected by directional sandwich panning in solution and name of ISVD belonging to those families. Within a family, all the ISVD have the same CDR3 but their sequence differs in the region outside this CDR. Meaning that these ISVD are expected to bind to the same epitope albeit with a different affinity.

| Families | ISVD |
|---|---|
| Family 1 | E139, E202, E216, E128, E225, E130, E195, E105 |
| Family 2 | E214 |
| Family 3 | E144, E219, E97, E132, E196, E205, E217, E54 |
| Family 4 | E61 |
| Family 5 | E229 |
| Family 6 | E121 |
| Family 7 | E201 |
| Family 8 | E200 |
| Family 9 | E74 |

One ISVD of each family was produced in *E. coli* WK6 and purified. The ability of the pure ISVDs to inhibit the activity of hNE was evaluated at a ISVD:hNE: 4:1. Only NbE201 inhibited hNE (remaining activity $3 \pm 0.03$ %). The NbE201 gene was transformed into expression vector pHEN6 to produce it in larger quantity in *E. coli* WK6 cells.

The amino acid sequence encoded by NbE201 is as follows:

QVQLQESGGGLVQAGGSLRLSCVVP**GRTISLYR**MGWFRQAPGKEREFVAG**INWSGDMT**

DYVDSVKGRFTISRDNAKNTVYLEMNSLKPEDTAIYYC**TADPKLLPLADSSYGY**WGQGT

QVTVSS (SEQ ID NO: 4).

The sequences of the 3 CDRs are:

- GRTISLYR (SEQ ID NO: 1)

- INWSGDMT (SEQ ID NO: 2)

- TADPKLLPLADSSYGY (SEQ ID NO: 3)

## Example 2 - Production and purification of NbE201

Following its expression into the periplasm of *E. coli* WK6, several independent batches of NbE201 were purified to homogeneity. In each batch, its purity was higher than 95% as judged by SDS-PAGE analysis and the integrity of the protein was confirmed by mass spectrometry (data not shown).

## Example 3 - NbE201 is a tight inhibitor of hNE

*3.1 Inhibition of the hydrolysis of a small synthetic substrate.* The substrate (N-Succinyl-Ala-Ala-Ala-paraNitroAnilide) Km was calculated by incubation of hNE with different substrate concentrations. The initial velocity of hydrolysis of hNE vo were plotted with [S] with the Hanes-Woolf linearization method: [S]/Vo vs [S]. The derived $K_m$ was equal to 1.4 mM.

The residual activity of hNE in the presence of different concentrations of NbE201 was determined from the initial rate of substrate hydrolysis (**Figure 4**). The activity of hNE is significantly inhibited in the presence of a NbE201 concentration similar (i.e. within a factor 10) to the total enzyme concentration (50 nM). This observation suggests that NbE201 acts as a tight binding inhibitor. The calculated $IC_{50}$ for the inhibition of the hydrolysis of N-Succinyl-Ala-Ala-Ala-paraNitroAnilide by hNE is of $38.3 \pm 3.3$ nM (hNE concentration: 50 nM). The $K_i$ was calculated averaging three $K_i$ of three independent curves, each curve representing experiment carried out with a different lot of production of the Nb. From each

curve, the $K_i$ was estimated by plotting $v_0/v_i$ vs [NbE201] and by using the following equation:

$$\frac{K_m}{K_m + [NbE201]} \cdot \frac{1}{K_i} = slope$$

considering $K_m$ = 1.4 mM and [S] = 1 mM. The $K_i$ was estimated to be 19 $\pm$ 3 nM.

This confirms that NbE201 is a tight-binding inhibitor. Similar inhibition was observed when NbE201 was preincubated with the hNE for 15 min before addition of the substrate. Moreover, the experiment was carried out in both a microplate format (deadtime of the experiment of 100 s) or in a 1.5 mL cell (deadtime of the experiment of 14 s). In both cases the graph shows no curvature at the initial time points, indicating that the equilibrium between the enzyme, substrate and NbE201 is reached within seconds.

As comparison, the hNE inhibition for the hydrolysis of N-Succinyl-Ala-Ala-Ala-paraNitroAnilide, by three other inhibitors: AAT ($\alpha$-1-anti-trypsin, Sigma-Aldrich, Germany, covalent natural inhibitor), Elastase Inhibitor 3 (EI3, Calbiochem, MerckMillipore, Darmstadt, Germany, small molecule, covalent inhibitor) and Sivelestat (R&D systems, United Kingdom, small molecule, non-covalent inhibitor). The measured IC50 are, respectively: 28 nM, 349 $\pm$ 70 nM and 77$\pm$1 nM. The IC50 for NbE201 is in the same range as that of AAT and better than the only clinically used small non-competitive inhibitor Sivelestat.

*3.2. Inhibition of the hydrolysis of elastin, a natural substrate.* Measurements of hNE activity inhibition of the hydrolysis of the physiological substrate, elastin, were carried out with NbE201 and with other hNE inhibitors (i.e., $\alpha$-1-anti-trypsin (AAT, Sigma-Aldrich, Germany), Elastase Inhibitor 3 (EI3, Calbiochem, MerckMillipore, Darmstadt, Germany) and Sivelestat (R&D systems, United Kingdom). To determine the residual activity of hNE, the absorbance of samples at different inhibitor:hNE ratios was compared to the one measured in the absence of inhibitor, as shown in **Figure 5**. The activity of hNE is inhibited by 80% in the presence of a ratio of [NbE201]: [hNE] equal to 15. AAT shows a higher inhibitory activity on hNE than NbE201 (85% inhibition at a ratio [AAT]:[hNE]=1.45). The activity of hNE is inhibited by 95% in the presence of a ratio of [EI3]:[hNE] of 15. These differences could be explained by the modality of hNE inhibition by the inhibitors tested. AAT and EI3 are covalent inhibitors, while NbE201 inhibits hNE non-covalently. Sivelestat is a non-covalent inhibitor of hNE and at [Sivelestat]: [hNE] =50, inhibition is only of 40%, whereas with NbE201 at the same ratio, hNE residual activity is almost zero. The IC50 for NbE201, AAT, EI3 and Sivelestat are respectively: 487.75 $\pm$ 70nM, 120 $\pm$ 4 nM, 271 $\pm$ 28 nM, 7760 $\pm$ 255 nM. Thus, NbE201 inhibits better the hydrolysis of physiological substrate than the only clinically used small inhibitor Sivelestat.

**Example 4 - Determination of the affinity of NbE201 for hNE.**

Quantitative binding measurements were performed in order to measure the kinetic ($k_{on}$, $k_{off}$) and equilibrium ($K_D$) constants. The sensorgrams obtained are shown in **Figure 6**. The global fit of the kinetics obtained with 5 different concentrations of hNE can be carried out with the 1:1 ligand model. The kinetics and equilibrium constant characterizing the binding are the following:

$$K_D = 2.18 \pm 1.60 \text{ nM } (k_{on} = 2.55 \cdot 10^5 \pm 1.40 \cdot 10^5 \text{ M}^{-1}\text{s}^{-1}, k_{off} = 4.30 \cdot 10^{-4} \pm 2.42 \cdot 10^{-4} \text{ s}^{-1})$$

**Example 5 - NbE201 is specific for NE.**

The ability of NbE201 to inhibit other serine proteases was verified by using chromogenic substrates (Figure 7A). This data suggests that NbE201 does not inhibit human cathepsin G (CG, 36.7 % identity with hNE), and porcine pancreatic elastase (pPE, 39.2 % identity with hNE), whereas NbE201 can inhibit human proteinase 3 (PR3, 54.7 % identity with hNE), however with a very low efficiency (20% of inhibition at a NbE201:enzyme ratio of 60). More importantly, **Figure 7B** demonstrates that NbE201 significantly inhibits murine elastase (mNE, 74.9 % of sequence identity with hNE). The IC$_{50}$ is 49.4 $\pm$ 6.6 nM.

To confirm that NbE201 is specific for hNE and that it does not bind to other serine proteases, a BLI assay was performed in the presence of five different proteases: hNE, hCG, hPR3, mice pancreatic elastase (mPE) and Porcine Pancreatic Elastase (pPE). **Figure 8** shows that there is only an association signal when NbE201 is in contact with hNE, meaning that NbE201 does not bind to the other proteases. This confirms the high specificity for NE.

Furthermore, the affinity between NbE201 and mNE was measured by BLI, in **Figure 9.** As for hNE, the sensorgrams were fitted using the 1:1 ligand model. The kinetics and equilibrium binding parameters are as follows:

$$K_D = 2.10 \pm 1.34 \text{ nM } (k_{on} = 2.34 \cdot 10^5 \pm 1.15 \cdot 10^5 \text{ M}^{-1}\text{s}^{-1}, k_{off} = 4.99 \cdot 10^{-4} \pm 3.21 \cdot 10^{-4} \text{ s}^{-1})$$

This demonstrates that NbE201 binds to mNE with a similar affinity as to hNE.

**Example 6 - NbE201 is a competitive inhibitor of NE.**

To determine if NbE201 binds into or near the active site of the elastase, competitive binding experiments were performed with molecules that are known to bind elastase into its active site. The first competitive molecule to be tested was AAT which is the natural inhibitor of hNE. **Figure 10** (right panel) shows that hNE in complex with AAT does not bind to NbE201, suggesting that NbE201 binds into the active site of the hNE. AAT forms a covalent complex with hNE and upon the formation of the complex both AAT and hNE undergo a conformational change. Thus, the absence of binding of the hNE-AAT preformed complex to NbE201 could result from the fact that the NbE201 epitope is not present anymore on the surface of hNE due to this conformational reorganization. To confirm this, two other inhibitory molecules known to bind hNE active site were tested. These molecules are: (i) MeOSuc-AAPV-CMK also referred to as Elastase Inhibitor 3 (EI3) and as methyl 4-[[(2S)-1-[[(2S)-1-[(2S)-2-[[(3S)-1-chloro-4-methyl-2-oxopentan-3-yl]carbamoyl]pyrrolidin-1-yl]-1-oxopropan-2-yl]amino]-1-oxopropan-2-yl]amino]-4-oxobutanoate, which is a small molecule inhibitor binding into hNE active site covalently but not inducing a conformational change into hNE, and (ii) cAb-H7S-Nter-P1, an ISVD which does not bind to hNE fused to an inhibitory peptide (P1) that binds the hNE active site non-covalently. **Figure 10** shows that when hNE is pre-incubated with saturating concentrations of these two inhibitors, it is unable to bind to NbE201. While the competition between NbE201 and AAT or Nb-H7S-Nter-P1 could results from steric hindrance effect (i.e. NbE201 binds close to the active site presenting the binding of the active-site targeting molecule), the competition with the small hNE competitive inhibitor (MeOSuc-AAPV-CMK) strongly suggests that NbE201 binds into the active site of hNE.

**Example 7 - Stability of NbE201 towards urea-induced unfolding.**

The thermodynamic stability of NbE201 was investigated by urea-induced unfolding monitored by intrinsic fluorescence ($\lambda_{max}$ and $F_{337nm}$) and far UV-CD (207 nm). In both cases, a single transition was obtained suggesting that both the tertiary and secondary structures unfold cooperatively following a two-state model (N $\rightleftharpoons$ U) **(Figure 11)**. The reversibility of the denaturation, after dilution of the urea concentration was established by intrinsic fluorescence. The coincidence of the transition curves obtained by intrinsic fluorescence and far UV-CD measurements after normalization demonstrates that secondary and tertiary structures are unfolded concomitantly (**Figure 12**). Thus, the denaturation of the protein can be described by an apparent cooperative two-state model (N $\rightleftharpoons$ U), where only the native and unfolded states are significantly populated. Therefore, values of the thermodynamic parameters were computed from the transition monitored by the three parameters assuming a simple two-state model and equation 1, as shown in **Table 2**. The value of $\Delta G^{\circ}_{NU(H2O)}$ is in the upper range of that observed for other Nbs (Dumoulin et al., 2002, doi: 10.1 1 10/ps.34602).

Table 2: Thermodynamic parameters of unfolding of NbE201 at pH 7, 25°C, as obtained from the analysis of the urea-induced equilibrium transitions.

| | CD | F ($\lambda$max) | F (337 nm) | Mean $\pm$ SD |
|---|---|---|---|---|
| $\Delta G^{\circ}_{NU(H2O)}$ (kJ·mole$^{-1}$) | 44.3 | 39.1 | 39.0 | 40.8 $\pm$ 2.5 |
| m (kJ·mol$^{-1}$ M$^{-1}$) | 7.7 | 6.5 | 6.7 | 7.0 $\pm$ 0.5 |
| $C_m$(M) | 5.8 | 6.0 | 5.8 | 5.9 $\pm$ 0.1 |

**Example 8 - Stability of NbE201 towards heat-induced denaturation.**

The stability of the NbE201 towards heat-induced unfolding was assessed by heating the protein sample from 25 to 97°C and then cooling it down to 25°C and by measuring the signal at 206 nm by far UV-CD (**Figure 13**). This experiment was carried out with three NbE201 samples from three independent lots (**Figure 14**). A single transition was obtained suggesting that the secondary structures unfold cooperatively following a two-state model (N--U). Upon cooling down the temperature, the refolding was only partial although very significant, thus only the apparent temperature of unfolding ($T_m^{app}$, temperature at which 50 % of the molecules are unfolded while the remaining are still in the native state) was considered. The $T_m$ as calculated for the transition obtained for the three different batches of ISVDs and then averaged, is 70.7 $\pm$ 1.5 °C, which is in the higher range of the $T_m$s observed for ISVDs (Dumoulin et al., 2002, doi: 10.1110/ps.34602).

**Example 9 - Stability of NbE201 towards human neutrophil elastase.**

The stability of NbE201 towards hNE proteolytic activity was tested for 14 days. The stability of the complex was assessed by SDS-PAGE. In **Figure 15**, the percentage of remaining complex over time is shown (upper curve). Over time, the complex remains around 100%, meaning that the NbE201 is stable and is not digested in presence of hNE for two weeks.

To be sure that the stability of the ISVD is not due to the complete loss of activity of the enzyme, hNE activity was also measured during 14 days. In **Figure 15**, the curve relative to the percentage of hNE remaining activity is shown (lower curve). Over time, hNE does loose some of its activity. Anyway, its loss of activity cannot explain the complex stability at around 100%. In conclusion, these data show that NbE201 is stable for 14 days in presence of hNE.

**Example 10 - Crystal structure of NbE201 and of the complex NbE201-hNE.**

NbE201 in complex with hNE was crystallized in the $P2_12_12_1$ group and the final resolution was 2.3Å. The asymmetric unit contains a single NbE201-hNE complex. The structure shows a well-defined density for amino acids I1 to Q218 of hNE, with the exception of amino acids R147 and N148 according to the chymotrypsinogen numbering scheme, and amino acids Q1 to S123 of NbE201 according to the IMGT numbering scheme. The refinement statistics are shown in **Table 3.** At positions N109 and N204, according to the chymotrypsinogen numbering scheme we found glycans in the NAG-NAG-FUC conformation.

Table 3 : Data collection and refinement statistics for the X-ray structure of NbE201 in complex with hNE and alone.

| | hNE-NbE201 | NbE201 |
|---|---|---|
| PDB code | | |
| **Data Collection:** | | |
| Wavelength | 0.9786 | 0.98011 |
| Space group | $P2_12_12_1$ | $P2_12_12_1$ |
| a, b, c (Å) | 65.06 73.36 85.32 | 61.68 88.68 86.56 |
| $\alpha$, $\beta$, $\gamma$ (°) | 90, 90, 90 | 90,90,90 |
| Resolution range (Å)[a] | 48.5 - 2.3 (2.42 - 2.3) | 44.35-1.8 (1.85-1.8) |
| Rmerge (%)[a] | 12.8 (191.4) | 16.8 (208) |
| $<I>/<\sigma I>$[a] | 11.6 (1.0) | 8.24 (1.3) |
| Completeness (%)[a] | 99.7 (98.1) | 97.9 (99) |
| Redundancy[a] | 13.2 (12.6) | 9.5 (9.5) |
| CC 1/2[a] | 0.999 (0.886) | 99.4 (62.6) |
| | | |
| **Refinement:** | | |
| No. of unique reflections | 18530 | 43692 |
| R work (%) | 22.4 | 21.69 |
| R free (%) | 27.1 | 25.43 |
| RMS deviations from ideal stereochemistry | | |
| Bond lengths (Å) | 0.01 | 6 |
| Bond angles (°) | 1.13 | 884 |
| Mean B factor (Å$^2$) | 85.0 | 32.7 (protein) 35.1 (solvent) |
| Ramachandran plot: | | |
| Favoured region (%) | 97.0 | 98.55 |
| Allowed regions (%) | 2.7 | 1.45 |
| Outlier regions (%) | 0.3 | 0 |
| [a] Numbers in parenthesis refer to the highest resolution shell. | | |

The interaction between the CDR3 of NbE201 and hNE is essentially mediated by polar interactions between amino acids P100, K101, D107, Y110 and Y112 (at the boundary between CDR3 and framework) of NbE201 and amino acids S195 A60, R36, N61 and P96 of hNE, respectively **(Figure 16A)**. At the level of CDR1, there is only one interaction between S30 of NbE201 and G218 and G219 of hNE. Furthermore, the hydrophobic interactions are limited to V2 of NbE201 and V97 of hNE **(Figure 16B)**. The proline P100, belonging to the CDR3, establishes a polar interaction with the hNE active site S195 through a water molecule **(Figure 17A)**. On the other hand, the amino acids H57 and D102 of the catalytic triad of the enzyme do not bind to NbE201 **(Figure 17A)**. Therefore, it is likely that the inhibition exerted by Nb E201 on hNE activity is due to the interaction with serine S195 via a water molecule interaction. The structure of the ISVD alone was solved at a resolution of 1.8 Å **(Figure 16C)**. The structure of the NbE201 alone was superimposed to the one of the Nb in complex with hNE and no structural differences were seen.

## Example 11 - NbE201 PEGylation does not affect its inhibitory capacity.

NbE201 was PEGylated according to the previously described protocol with polyethylene glycol (PEG) linked to a maleimide function able to react with the added cysteine in C-terminal. Three PEGs were separately used: linear PEG of 10 or 20 kDa, or a branched 40 kDa PEG. PEGylation products were purified via FPLC with a cation exchange column and their purity was verified by electrophoresis gel staining. Their inhibitory capacity was then assessed on purified hNE by measuring the lysis of a chromogenic hNE substrate in presence or absence of Nbs by spectrophotometry (**Figure 18**). Inhibition curves were obtained for the wild-type NbE201 and its PEGylated forms by exposing hNE to the ISVD with a molar hNE: NbE201 ratio ranging from 1:0.25 to 1:5 in an *in vitro* enzymatic assay. No significant difference was observed between the inhibitory capacity of NbE201 and its PEGylated counterparts.

## Example 12 - PEGylation protects the Nbs from damages induced by nebulization, agitation and FIT cycles

To assess whether PEGylation increases or decreases NbE201 stability, the resistance of the native and PEGylated ISVD towards different stresses was studied and compared. Therefore, wild type and PEGylated ISVD were exposed to nebulization, agitation with a magnetic bar, FIT cycles and will be exposed to chemical denaturant and heat. So far, wild type NbE201 and NbE201 PEGylated with a PEG10 or 40 were exposed to a MeSH nebulization, 1 or 4h of agitation and 5 or 10 FAT cycles. Their concentration before and after centrifugation at 10 000 g was measured in order to highlight the presence of insoluble aggregates. Regarding the non-PEGylated ISVD, a decrease in concentration is already observed before centrifugation in agitated and nebulized samples (**Table 4**). This decrease is further pronounced in centrifuged samples. A decrease in concentration after centrifugation is also observed in samples exposed to FIT cycles. However, no such concentration loss is observed in NbE201-PEG40 samples exposed to agitation or FIT cycles (**Table 4**). A decrease in NbE201-PEG40 concentration is observed in nebulized samples though, but no difference was detected after centrifugation, suggesting that the decrease in concentration might not necessarily come from protein aggregation but might come from a loss of protein during the nebulization process and the sampling process. Similar results showing a protective effect of the PEGylation were obtained with the NbE201-PEG10.

Table 4. Concentration percentage of preserved ISVD viaA280nm absorbance measurements before and after centrifugation of the sample at 10 000 g during 10 min. Percentage of the wild type and PEGylated (liner PEG10 or branched PEG40) NbE201 initial concentration after exposure to nebulization, one or four hours of magnetic agitation or 5 or 10 freeze/thaw (F/T) cylces. N=3, n=3 (NbE201 wt and NbE201-PEG40). N=2, n=3 (NbE201-PEG10). N=number of experiments, n= technical replicates. Means ± SD. Statistical test (NbE201 wt and NbE201-PEG40): one-way ANOVA followed by a Dunnett post-hoc test, compared to the respective control group. *P value <0.05, **p value<0.01, ***p value<0.001, ****p value <0.0001.

| | NbE201 wt | | NbE201-PEG40 | | NbE201-PEG 10 | |
|---|---|---|---|---|---|---|
| | Initial concentration percentage before centrifugation | Initial concentration percentage after centrifugation | Initial concentration percentage before centrifugation | Initial concentration percentage after centrifugation | Initial concentration percentage before centrifugation | Initial concentration percentage after centrifugation |
| Control | 100±0% | 100±0% | 100±0% | 99±1% | 100±0% | 94±2% |
| Nebulization | 73%±3%(***) | 31±2% (****) | 88±3% (****) | 88±3% (****) | 85±2% (***) | 85±3% |

(continued)

| | NbE201 wt | | NbE201-PEG40 | | NbE201-PEG 10 | |
|---|---|---|---|---|---|---|
| | Initial concentration percentage before centrifugation | Initial concentration percentage after centrifugation | Initial concentration percentage before centrifugation | Initial concentration percentage after centrifugation | Initial concentration percentage before centrifugation | Initial concentration percentage after centrifugation |
| Agitatio n (1h) | 93±4% | 54±12% (****) | 99±1% | 98±1% | 98±1% | 97±2% |
| Agitatio n (4h) | 77±6%(**) | 27±19% (****) | 98±1% | 99±1% | 100±1% | 93±2% |
| F/T 5x | 96±8% | 85±10% (*) | 97±2% | 99±1% | 99±1% | 99±1% |
| F/T 10x | 103±5% | 75±9 % (**) | 97±2% | 97±3% | 98±2% | 98±1% |

The inhibitory capacity of the samples exposed to nebulization, agitation or FIT cycles was then compared to the inhibitory capacity of the unexposed ISVD (**Table 4 and Figure 19**). The observed loss of inhibitory capacity induced on the wild type NbE201 are in line with the concentration loss described above, since the greatest losses were observed with nebulization and 4h of agitation. However, the smaller loss of concentration described above did not correlate with a decreased inhibitory capacity in the samples exposed to FIT cycles. The NbE201-PEG40 samples did not display any significant loss of inhibitory activity, suggesting an increased stability of the ISVD towards the applied stresses thanks to PEGylation. Similar results were obtained with the NbE201-PEG10. To our knowledge, PEGylation has not yet been described to protect from nebulization.

Nebulized samples and their control were also analyzed by NTA to confirm the presence of aggregates. Particles size and count were measured for the NbE201, NbE201-PEG10 and NbE201-PEG40 before and after nebulization. An average minimum of 50 counted particles per frame is required for the measure to be reliable. The average counted particles were far below that threshold in the blank solution and all the measured solutions, except for the nebulized NbE201, where an average counted particles per frame of 228 was measured. Regarding the sizes of the counted particles, the percentile 10, 50 and 90 respectively were 22.3 ± 12, 138 ± 8 and 342.8 ± 26 nm. Thus, particles were tracked in nebulized samples from the native NbE201 and not in nebulized samples of PEGylated NbE201 with this method.

## Example 13 - Dry powder formulation.

Since the NbE201 aims at treating lung diseases, inhalation dry powders were produced with the NbE201 and the NbE201-PEG40 and then characterized. Two protein stabilizers were added to the composition of the powder: Trehalose (a non-reducing sugar) and trileucine.

The integrity of the ISVD within the spray-dried powders was then evaluated after solubilizing the powders into water followed by a dialysis into phosphate buffer (50 mM, pH7). Protein concentration was measured by reading the absorbance at 280nm before and after centrifugation at 10 000g for ten minutes in order to detect the presence of insoluble aggregates. Regarding the NbE201, the concentration read after centrifugation was 53 ± 3% of the concentration read before centrifugation, indicating that almost half of the Nb aggregated during spray-drying. On the contrary, the NbE201-PEG40 concentration after centrifugation was 97 ± 2 % of the initial concentration, indicating that the PEGylation protects against aggregation during the spray drying.

The inhibitory activity was also assessed after solubilizing the powders. The inhibitory capacity of the Nbs were assessed in an *in vitro* assay at a [hNE]:[ISVD] ratio of 1:1. The inhibition capacity of the solubilized powders was compared to the inhibition capacity of their respective controls (eg. untreated NbE201 or NbE201-PEG40). While NbE201 showed a decrease in inhibition capacity at the studied ratio due to the spray drying, it was not the case for NbE201-PEG40 (**Table 5**). Moreover, the inhibition capacity of the Spray-dried ISVD was also assessed after centrifugation to determine whether the remaining Nbs (without aggregates) displayed a decreased inhibition capacity. The protein concentration used to calculate the hNE: ISVD ratio was the concentration read after centrifugation. No decrease of inhibition was observed for both ISVD in these conditions, suggesting that the soluble ISVD have kept their inhibitory capacity.

Table 5: hNE Inhibition percentage by untreated (control) NbE201 or NbE201-PEG40 or spray dried NbE201 or NbE201-PEG40, before or after removing the aggregates by centrifugation at 10 000g. N=1, n=3.

| Inhibition percentage | NbE201-WT | NbE201-PEG40 |
|---|---|---|
| hNE alone | $0 \pm 1\%$ | $0 \pm 1\%$ |
| hNE + Control sdAb | $76 \pm 1\%$ | $70 \pm 2\%$ |
| hNE + Solubilized powder; with aggregates | $48 \pm 5\%$ | $67 \pm 2\%$ |
| hNE + Solubilized powder; without aggregates | $78 \pm 2\%$ | $72 \pm 1\%$ |

**Example 14 - NbE201 is able to inhibit proteolytic activity in cystic fibrosis patient sputa and PEGylation prolongs its activity**

The inhibitory capacity of NbE201 was then studied on sputa from patients with cystic fibrosis. First, inhibition curves of the ISVD alone were obtained from the exposure of 20mg of sputum to NbE201 quantities ranging from 0 to 60µg. The quantity of NbE201 necessary to totally inhibit the sputum proteolytic activity varied according to the visual classification of the sputum (mucoid, purulent or muco-purulent) and to a previous proteolytic activity estimation based on a standard curve with different quantities of purified hNE. The required amount to inhibit proteolytic activity in purulent samples was higher than in muco-purulent samples (**Figure 20**).

The inhibitory capacity of the NbE201 in sputa was then compared to the inhibitory capacity of the PEGylated ISVD and of AAT. Inhibition curves were obtained by exposing 15mg of sputum to a concentration of 0 to 15µmol/ml ofNbE201, NbE201-PEG10, NbE201-PEG40 or AAT (**Figure 21**). Although it only requires an AAThNE ratio of 1:1 to fully inhibit the elastase in aqueous assays, the AAT inhibitory capacity in sputa was lower than that of the PEGylated and native ISVD.

Non-linear regression curves were fitted via the use of GraphPad Prism 9.1.2 to interpolate concentrations of Nb/AAT to inhibit 50% of the activity of the sputa. The elastase activity of each sputa was then estimated based on standard curves made with increasing concentrations of purified hNE. Nanobody/AAT: hNE molar ratio to inhibit 50% of the sputum proteolytic activity were then calculated based on the estimated hNE activity in the sputa (**Figure 22**). Although AAT only needs a 1:1 AAT:hNE molar ratio *in vitro* to achieve total elastase inhibition while it requires a 3:1 NbE201:hNE ratio, AAT:hNE ratio to inhibit 50% of a given sputum quantity is significantly higher than NbE201:hNE ratio. No significant differences were observed between native and PEGylated ISVD.

The inhibition capacity of the non-PEGylated and PEGylated Nbs was studied over time. To this end, the inhibition capacities were assessed after different exposure times ranging from 0 to 24h. No significant difference was observed between the inhibition induced by the NbE201 and the one induced by NbE201-PEG10 or NbE201-PEG40 at time 0. However, a loss of inhibitory capacity over time was observed for the NbE201, and this loss was reduced for the PEGylated ISVD (**Figure 23**).

The results presented here suggest that the NbE201 is able to inhibit the proteolytic activity in sputum samples *ex vivo* and that the inhibition capacity might be prolonged by PEGylation.

The same experiment was then repeated to compare the inhibition capacity of the ISVD over time to the inhibition capacity of AAT. Although the AAT inhibition capacity is lower at time 0, it did not show any significant decrease over time (**Figure 23**).

**Example 15 - Non-PEGylated and PEGylated NbE201 are able to inhibit proteolytic activity in ciliary dyskinesia and COPD patient sputa.**

The inhibitory capacity of the NbE201(-PEG40) or AAT was then studied on a sputum from a COPD patient in exacerbation and a sputum from a ciliary dyskinesia (CD) patient.

Inhibition curves were obtained from the exposure of 15 mg (COPD) or 10 mg (CD) of sputum to increasing concentrations of NbE201, NbE201-PEG40 or AAT. No apparent differences in inhibitory capacity were observed between the PEGylated and native ISVD and AAT (**Figure 25 and 26**).

**Example 16 - PEGylation increases NbE201 residence time in β-ENaC and wild type Swiss mice.**

A pharmacokinetic study was performed in wild type Swiss mice and in a β-ENaC mouse model mimicking the lung pathophysiology of cystic fibrosis. The NbE201, NbE201-PEG40 and AAT were instilled into the trachea and BALs were collected after 0, 4 or 24h. The ISVD and AAT were then quantified by ELISA and it was observed that the PEGylation with a PEG40 increased the residence time of the NbE201. While only 3.3 and 4.4 % of the NbE201 dose collected at time 0 were quantified after 24h in β-ENaC and Swiss mice respectively, 56 % and 65 % of the NbE201-PEG40 dose collected at time 0 were quantified after 24h in β-ENaC and Swiss mice respectively. AAT quantification in the BALs collected after 24h was intermediate as 25 % and 33 % of the time 0h dose were recovered in β-ENaC and Swiss mice respectively. No significant difference was observed between the two mice models (**Figure 27**).

**Example 17 - Protective activity of NbE201 against the development of hNE-induced acute lung injury syndrome in mouse model.**

The presence of hemoglobin reflects the development of alveolar-capillary lesions as a result of hNE instillation (**Figures 3B** and **3C**). Histologically, "hemorrhagic lesions" defined by areas rich in red blood cells are observed in the unwashed lungs (**Figure 3D**). We have developed and characterized a relevant mouse (nasal instillation of hNE 25 μg) model allowing us to assess the effect of the selected NbE201 by analyzing alveolar-capillary damage. As AAT, the NbE201 protects the lungs from developing alveolar-capillary damage in the presence of hNE (**Figure 3D**).

## Claims

1. A binding agent capable of specifically binding to and inhibiting a neutrophil elastase (NE), wherein the binding agent comprises an immunoglobulin single variable domain (ISVD) and the binding agent competes with Elastase Inhibitor 3 (EI3) for binding to NE.

2. A binding agent according to claim 1, wherein the binding agent comprises an immunoglobulin single variable domain (ISVD) binding to at least one of the residues R36, A60, N61, P96, V97, S195, G218 and G219, preferably two or more of the residues R36, A60, N61, P96, V97, S195, G218 and G219, more preferably three or more of the residues R36, A60, N61, P96, V97, S195, G218 and G219, more preferably all of the residues R36, A60, N61, P96, V97, S195, G218 and G219 of the NE, such as to at least the residues R36, A60, N61, P96, V97 and S195 of the NE.

3. The binding agent according to claim 1 or 2, wherein the binding agent comprises an immunoglobulin single variable domain (ISVD) comprising a complementarity determining region 1 (CDR1) having the sequence set forth in SEQ ID NO:1 (GRTISLYR), a CDR2 having the sequence set forth in SEQ ID NO:2 (INWSGDMT) and a CDR3 having the sequence set forth in SEQ ID NO:3 (TADPKLLPLADS SYGY).

4. The binding agent according to claim 1 or 2,
   wherein the binding agent comprises an ISVD comprising a CDR1, CDR2 and CDR3, each as present in SEQ ID NO: 4 wherein the CDR1, CDR2 and CDR3 are annotated according to anyone of IMGT, Kabat, Chlotia, Martin or AHo numbering systems.

5. The binding agent according to claim 1 to 4, which comprises or consists of a VHH.

6. The binding agent according to any one of claims 1 to 5, wherein the ISVD comprises an amino acid sequence with at least 90% identity to the amino acid sequence set forth in SEQ ID NO:4

   (QVQLQESGGGGLVQAGGSLRLSCVVPGRTISLYRMGWFRQAPGKEREFVAGINWSGDMT DYVDSVKGRFTISRDNAKNTVYLEMNSLKPEDTAIYYCTADPKLLPLADSSYGYWGQGTQ VTVSS).

7. The binding agent according to any one of claims 1 to 6, wherein the ISVD comprises the amino acid sequence set forth in SEQ ID NO:4

   (QVQLQESGGGGLVQAGGSLRLSCVVPGRTISLYRMGWFRQAPGKEREFVAGINWSGDMT DYVDSVKGRFTISRDNAKNTVYLEMNSLKPEDTAIYYCTADPKLLPLADSSYGYWGQGTQ VTVSS).

8. The binding agent according to any one of claims 1 to 7, wherein the binding agent binds to at least a portion of the active site of the NE.

9. A nucleic acid molecule comprising a polynucleotide sequence encoding the binding agent according to any one of claims 1 to 8, or a vector comprising the nucleic acid molecule.

10. A cell or a virus comprising the nucleic acid molecule or vector according to claim 9, optionally wherein the cell is capable of expressing or expresses the binding agent or the virus is configured to induce expression of the binding agent in a recipient cell infected with the virus.

11. A pharmaceutical composition comprising the binding agent according to any one of claims 1 to 8, the nucleic acid molecule or vector according to claim 9, or the cell or virus according to claim 10, and a pharmaceutically acceptable carrier, diluent, and/or excipient, optionally wherein the pharmaceutical composition comprises a further inhibitor of the NE.

12. The pharmaceutical composition according to claim 11, wherein the pharmaceutical composition is suitable for nasal or pulmonary administration to a subject, optionally wherein the pharmaceutical composition is formulated as an inhalable dry powder, optionally wherein the pharmaceutical composition is configured for a nebulizer or a metered-dose inhaler.

13. Device comprising the pharmaceutical composition according to claim 11 or 12, wherein the device is a nebulizer or a metered-dose inhaler or a dry powder inhaler.

14. A kit such as a diagnostic kit comprising the binding agent according to any one of claims 1 to 8, the nucleic acid molecule or vector according to claim 9, or the cell or virus according to claim 10.

15. The binding agent according to any one of claims 1 to 8, the nucleic acid molecule or vector according to claim 9, the cell or virus according to claim 10, or the pharmaceutical composition according to claim 11, for use in medicine.

16. The binding agent according to any one of claims 1 to 8, the nucleic acid molecule or vector according to claim 9, the cell or virus according to claim 10, or the pharmaceutical composition according to claim 11, for use in the prevention or treatment of an inflammatory disease.

17. The binding agent, the nucleic acid molecule, the vector, the cell or virus, or the pharmaceutical composition for use according to claim 16, wherein the inflammatory disease is a pulmonary disease, preferably a disease selected from the group consisting of a muco-obstructive lung disease such as cystic fibrosis, chronic obstructive pulmonary disease (COPD), bronchiectasis, ciliary diskinesia and acute respiratory distress syndrome, inflammatory nose and sinus diseases, or the inflammatory disease is inflammatory bowel disease or the inflammatory disease is an inflammatory skin disease.

18. The binding agent according to any one of claims 1 to 8, the nucleic acid molecule or vector according to claim 9, the cell or virus according to claim 10, or the kit according to claim 14, for use in the diagnosis of an inflammatory disease in a subject.

19. A method for determining the level of active neutrophil elastase in a specimen, comprising contacting the specimen with the binding agent according to any one of claims 1 to 8, and detecting at least the neutrophil elastase bound to the binding agent.

20. A method for the diagnosis, prognosis and/or monitoring of an inflammatory disease, optionally any inflammatory disease as defined in claim 17, in a subject, comprising:

- contacting a biological sample obtained from the subject with the binding agent according to any one of claims 1 to 8, and
- determining the level of active neutrophil elastase in the sample by detecting at least the neutrophil elastase bound to the binding agent.

## Fig.1

| FASTA numbering | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | | 24 |
|---|---|---|---|---|---|---|---|---|---|---|---|---|---|---|---|---|---|---|---|---|---|---|---|---|---|
| | I | V | G | G | R | R | A | R | P | H | A | W | P | F | M | V | S | L | Q | L | R# | G | G | - | H |
| Chymotrypsinogen numbering scheme | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 |

| FASTA numbering | 25 | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 |
|---|---|---|---|---|---|---|---|---|---|---|---|---|---|---|---|---|---|---|---|---|---|---|---|---|---|
| | F | C | G | A | T | L | I | A | P | N | F | V | M | S | A | A | H | C | V | A# | N# | V | N | V | R |
| Chymotrypsinogen numbering scheme | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 |

| FASTA numbering | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 |
|---|---|---|---|---|---|---|---|---|---|---|---|---|---|---|---|---|---|---|---|---|---|---|---|---|---|
| | A | V | R | V | V | L | G | A | H | N | L | S | R | R | E | P | T | R | Q | V | F | A | V | Q | R |
| Chymotrypsinogen numbering scheme | 66 | 67 | - | 68 | 69 | 70 | 71 | 72 | 73 | 74 | 75 | 76 | 77 | 78 | 79 | 78 | 80 | 81 | 82 | 83 | 84 | 85 | 86 | 87 | 88 |

| FASTA numbering | 75 | 76 | 77 | 78 | 79 | - | 80 | 81 | 82 | 83 | 84 | 85 | 86 | 87 | 88 | 89 | 90 | 91 | 92 | 93 | 94 | 95 | 96 | 97 | 98 |
|---|---|---|---|---|---|---|---|---|---|---|---|---|---|---|---|---|---|---|---|---|---|---|---|---|---|
| | I | F | E | N | G | - | Y | D | P# | V# | N | L | L | N | D | I | V | I | L | Q | L | N* | G | S | A |
| Chymotrypsinogen numbering scheme | 89 | 90 | 91 | 92 | 93 | 94 | 94 | 95 | 96 | 97 | 98 | 99 | 100 | 101 | 102 | 103 | 104 | 105 | 106 | 107 | 108 | 109 | 110 | 111 | 112 |

| FASTA numbering | 99 | 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 |
|---|---|---|---|---|---|---|---|---|---|---|---|---|---|---|---|---|---|---|---|---|---|---|---|---|---|
| | T | I | N | A | N | V | Q | V | A | Q | L | P | A | Q | G | R | R | L | G | N | G | V | Q | C | L |
| Chymotrypsinogen numbering scheme | 113 | 114 | 115 | 116 | 117 | 118 | 119 | 120 | 121 | 122 | 123 | 124 | 125 | 126 | 127 | 128 | 129 | 130 | 131 | 132 | 133 | 134 | 135 | 136 | 137 |

| FASTA numbering | 124 | 125 | 126 | 127 | 128 | 129 | 130 | 131 | - | 132 | 133 | 134 | 135 | 136 | 137 | 138 | 139 | 140 | 141 | 142 | 14 3 | 144 | 145 | 146 | 147 |
|---|---|---|---|---|---|---|---|---|---|---|---|---|---|---|---|---|---|---|---|---|---|---|---|---|---|
| | A | M | G | W | G | L | L | G | - | R | N | R | G | I | A | S | V | L | Q | E | L | N | V | T | V |
| Chymotrypsinogen numbering scheme | 138 | 139 | 140 | 141 | 142 | 143 | 144 | 145 | 146 | 147 | 148 | 149 | 150 | 151 | 152 | 153 | 154 | 155 | 156 | 157 | 158 | 159 | 160 | 161 | 162 |

| FASTA numbering | 148 | 149 | 150 | 151 | - | 152 | 153 | 154 | - | - | - | - | - | - | - | - | 155 | 156 | 157 | 158 | 159 | 160 | 161 | 162 | 163 |
|---|---|---|---|---|---|---|---|---|---|---|---|---|---|---|---|---|---|---|---|---|---|---|---|---|---|
| | V | T | S | L | - | C | R | R | - | - | - | - | - | - | - | - | S | N | V | C | T | L | V | R | G |
| Chymotrypsinogen numbering scheme | 163 | 164 | 165 | 166 | 167 | 168 | 169 | 170 | 171 | 172 | 173 | 174 | 175 | 176 | 177 | 178 | 179 | 180 | 181 | 182 | 183 | 184 | 185 | 186 | 187 |

| FASTA numbering | 164 | 165 | 166 | 167 | 168 | 169 | 170 | 171 | 172 | 173 | 174 | 175 | 176 | 177 | 178 | 179 | - | - | 180 | 181 | 182 | - | - | 183 | 184 |
|---|---|---|---|---|---|---|---|---|---|---|---|---|---|---|---|---|---|---|---|---|---|---|---|---|---|
| | R | Q | A | G | V | C | F | G | D | S# | G | S | P | L | V | C | - | - | N* | G | L | - | - | I | H |
| Chymotrypsinogen numbering scheme | 188 | 189 | 190 | - | - | 191 | 192 | 193 | 194 | 195 | 196 | 197 | 198 | 199 | 200 | 201 | 202 | 203 | 204 | 205 | 206 | 207 | 208 | 209 | 210 |

| FASTA numbering | 185 | 186 | 187 | 188 | 189 | 190 | 191 | 192 | 193 | 194 | 195 | 196 | 197 | 198 | 199 | 200 | 201 | 202 | 203 | 204 | 205 | 206 | 207 | 208 | 209 |
|---|---|---|---|---|---|---|---|---|---|---|---|---|---|---|---|---|---|---|---|---|---|---|---|---|---|
| | G | I | A | S | F | V | R | G# | G# | C | A | S | G | L | Y | P | D | A | F | A | P | V | A | Q | F |
| Chymotrypsinogen numbering scheme | 211 | 212 | 213 | 214 | 215 | 216 | 217 | 218 | 219 | 220 | 221 | 222 | 223 | 224 | - | 225 | 226 | 227 | 228 | 229 | 230 | 231 | 232 | 233 | 234 |

| FASTA numbering | 210 | 211 | 212 | 213 | 214 | 215 | 216 | 217 | - | - | 218 |
|---|---|---|---|---|---|---|---|---|---|---|---|
| | V | N | W | I | D | S | I | I | | | Q |
| Chymotrypsinogen numbering scheme | 235 | 236 | 237 | 238 | 239 | 240 | 241 | 242 | 243 | 244 | 245 |

EP 4 483 951 A1

Fig. 2

**Fig. 3**

Fig. 4

Fig. 5

**Fig. 6**

**Fig. 7**

**Fig. 8**

**Fig. 9**

**Fig. 10**

Fig. 11

**Fig. 12**

**Fig. 13**

**Fig. 14**

**Fig. 15**

Fig. 16

A

B

C

Fig. 17

A

B

Fig. 18

Fig. 19

Fig. 20

Fig. 21

Fig. 22

Fig. 23

Fig. 24

Fig. 25

Fig. 26

Fig. 27

Europäisches
Patentamt

European
Patent Office

Office européen
des brevets

# EUROPEAN SEARCH REPORT

**Application Number**

EP 23 18 2874

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHU XIAOJIE ET AL: "Human Antibody Domains and Fragments Targeting Neutrophil Elastase as Candidate Therapeutics for Cancer and Inflammation-Related Diseases", INTERNATIONAL JOURNAL OF MOLECULAR SCIENCES, vol. 22, no. 20, 15 October 2021 (2021-10-15), page 11136, XP093103574, DOI: 10.3390/ijms222011136 | 1,5 | INV. A61P11/00 C07K16/40 |
| Y | * abstract * * page 3, paragraph 2. Results; figures 3,6 * * page 9, paragraph 4 * ----- | 8-20 | |
| A | DAVIES P L ET AL: "Monoclonal anti-neutrophil elastase antibody characterisation: Ability to block function, detect free versus serpin-complexed enzyme and stain intracellular granules", JOURNAL OF IMMUNOLOGICAL METHODS, ELSEVIER SCIENCE PUBLISHERS B.V.,AMSTERDAM, NL, vol. 336, no. 2, 31 July 2008 (2008-07-31), pages 175-182, XP022822449, ISSN: 0022-1759, DOI: 10.1016/J.JIM.2008.04.010 [retrieved on 2008-05-09] * abstract * * paragraph [2.1Antibodies] * * paragraph [2.2Functionalassay] * * paragraph [3.2Monoclonalantibodyinhibition] * ----- | 1-20 | TECHNICAL FIELDS SEARCHED (IPC) A61P C07K A61K |
| Y | EP 0 345 906 A2 (MERCK & CO INC [US]) 13 December 1989 (1989-12-13) * page 12, line 32 – page 13, line 30 * ----- | 8-20 | |
| | -/-- | | |

The present search report has been drawn up for all claims

| Place of search | Date of completion of the search | Examiner |
|---|---|---|
| Munich | 5 December 2023 | Page, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

Application Number

EP 23 18 2874

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2013/003641 A2 (INHIBRX LLC [US]; ECKELMAN BRENDAN P [US] ET AL.) 3 January 2013 (2013-01-03) * paragraphs [0071] – [0098], [0121] * ----- | 8-20 | |
| A | WO 2013/128031 A1 (ABLYNX NV [BE]) 6 September 2013 (2013-09-06) * page 26, line 29 – page 27, line 25 * * page 43, line 26 – page 47, line 5 * * page 96, line 9 – line 26 * * page 94, line 21 – page 96, line 19 * ----- | 1-20 | |

TECHNICAL FIELDS
SEARCHED      (IPC)

The present search report has been drawn up for all claims

| Place of search | Date of completion of the search | Examiner |
|---|---|---|
| Munich | 5 December 2023 | Page, Michael |

EPO FORM 1503 03.82 (P04C01)

## ANNEX TO THE EUROPEAN SEARCH REPORT
## ON EUROPEAN PATENT APPLICATION NO.                    EP 23 18 2874

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2023

| Patent document cited in search report | | | Publication date | Patent family member(s) | | | Publication date |
|---|---|---|---|---|---|---|---|
| EP | 0345906 | A2 | 13-12-1989 | DE | 68925923 | T2 | 14-11-1996 |
| | | | | EP | 0345906 | A2 | 13-12-1989 |
| | | | | JP | H02117699 | A | 02-05-1990 |
| WO | 2013003641 | A2 | 03-01-2013 | AU | 2012275287 | A1 | 16-01-2014 |
| | | | | AU | 2017279724 | A1 | 25-01-2018 |
| | | | | AU | 2019202904 | A1 | 16-05-2019 |
| | | | | AU | 2021202131 | A1 | 06-05-2021 |
| | | | | BR | 112013033799 | A2 | 14-02-2017 |
| | | | | CA | 2839619 | A1 | 03-01-2013 |
| | | | | CA | 3132298 | A1 | 03-01-2013 |
| | | | | CN | 103917563 | A | 09-07-2014 |
| | | | | CN | 110066340 | A | 30-07-2019 |
| | | | | CN | 110551223 | A | 10-12-2019 |
| | | | | DK | 2726092 | T3 | 30-09-2019 |
| | | | | EP | 2726092 | A2 | 07-05-2014 |
| | | | | EP | 3569243 | A1 | 20-11-2019 |
| | | | | ES | 2746052 | T3 | 04-03-2020 |
| | | | | HR | P20191652 | T1 | 13-12-2019 |
| | | | | HU | E046156 | T2 | 28-02-2020 |
| | | | | IL | 276534 | A | 30-09-2020 |
| | | | | JP | 6674604 | B2 | 01-04-2020 |
| | | | | JP | 2014523900 | A | 18-09-2014 |
| | | | | JP | 2018008956 | A | 18-01-2018 |
| | | | | JP | 2020078325 | A | 28-05-2020 |
| | | | | JP | 2022109968 | A | 28-07-2022 |
| | | | | KR | 20140054000 | A | 08-05-2014 |
| | | | | KR | 20200027041 | A | 11-03-2020 |
| | | | | KR | 20210032558 | A | 24-03-2021 |
| | | | | KR | 20220003656 | A | 10-01-2022 |
| | | | | KR | 20230114318 | A | 01-08-2023 |
| | | | | LT | 2726092 | T | 10-10-2019 |
| | | | | ME | 03473 | B | 20-01-2020 |
| | | | | MX | 356517 | B | 01-06-2018 |
| | | | | NZ | 619023 | A | 31-07-2015 |
| | | | | NZ | 744257 | A | 28-10-2022 |
| | | | | PL | 2726092 | T3 | 29-11-2019 |
| | | | | PT | 2726092 | T | 08-10-2019 |
| | | | | RU | 2727452 | C1 | 21-07-2020 |
| | | | | RU | 2728861 | C1 | 31-07-2020 |
| | | | | RU | 2014102583 | A | 10-08-2015 |
| | | | | RU | 2017145308 | A | 18-02-2019 |
| | | | | RU | 2020124172 | A | 21-01-2022 |
| | | | | SG | 10201601621P | A | 28-04-2016 |
| | | | | SG | 10201811256Q | A | 30-01-2019 |
| | | | | SI | 2726092 | T1 | 29-11-2019 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 18 2874

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2023

| Patent document cited in search report | Publication date | Patent family member(s) | | Publication date |
|---|---|---|---|---|
| | | US | 2013011398 A1 | 10-01-2013 |
| | | US | 2013330769 A1 | 12-12-2013 |
| | | US | 2015147325 A1 | 28-05-2015 |
| | | US | 2018179264 A1 | 28-06-2018 |
| | | US | 2020102371 A1 | 02-04-2020 |
| | | US | 2021002352 A1 | 07-01-2021 |
| | | US | 2021024613 A1 | 28-01-2021 |
| | | WO | 2013003641 A2 | 03-01-2013 |
| WO 2013128031 A1 | 06-09-2013 | AU | 2013224851 A1 | 28-08-2014 |
| | | CA | 2863468 A1 | 06-09-2013 |
| | | CN | 104144945 A | 12-11-2014 |
| | | EP | 2820043 A1 | 07-01-2015 |
| | | JP | 6415987 B2 | 31-10-2018 |
| | | JP | 7007250 B2 | 10-02-2022 |
| | | JP | 2015509723 A | 02-04-2015 |
| | | JP | 2019013239 A | 31-01-2019 |
| | | KR | 20140132748 A | 18-11-2014 |
| | | PH | 12014501818 A1 | 17-11-2014 |
| | | RU | 2014139911 A | 20-04-2016 |
| | | SG | 11201404532X A | 28-08-2014 |
| | | US | 2015044215 A1 | 12-02-2015 |
| | | US | 2019055322 A1 | 21-02-2019 |
| | | WO | 2013128031 A1 | 06-09-2013 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0518629 A
- WO 9404678 A
- WO 9504079 A
- WO 9634103 A
- WO 9425591 A
- WO 9937681 A
- WO 0040968 A
- WO 0043507 A
- WO 0065057 A
- WO 0140310 A
- WO 0144301 A
- EP 1134231 A
- WO 0248193 A
- WO 9749805 A
- WO 0121817 A
- WO 03035694 A
- WO 03054016 A
- WO 03055527 A

- WO 03050531 A, ofAlgonomics N.V. and Ablynx N.V
- WO 0190190 A
- WO 03025020 A
- EP 1433793 A
- WO 04041867 A
- WO 04041862 A
- WO 04041865 A
- WO 04041863 A
- WO 04062551 A
- WO 05044858 A
- WO 0640153 A
- WO 06079372 A
- WO 06122786 A
- WO 06122787 A
- WO 06122825 A
- US 20060014264 A

**Non-patent literature cited in the description**

- **AMIN** ; **RATJEN**. *Expert Opin Emerg Drugs*, 2014, vol. 19 (1), 143-55
- **SUTER et al.** *Eur Respir J*, 1991, vol. 4 (1), 40-9
- **HAMERS-CASTERMAN et al.** Naturally occurring antibodies devoid of light chains. *Nature*, 1993, vol. 363, 446-448
- **DESMYTER et al.** Crystal structure of a camel single-domain VH antibody fragment in complex with lysozyme. *Nat Struct Biol*, 1996, vol. 3, 803-811
- **MUYLDERMANS**. *Reviews in Molecular Biotechnology*, 2001, vol. 74, 277-302
- **ELLMAN et al.** *Methods Enzymol*, 1991, vol. 202, 301-36
- **ALTSCHUL et al.** *J Mol Biol*, 1990, vol. 215, 403-10
- **TATUSOVA** ; **MADDEN**. *FEMS Microbiol Lett*, 1999, vol. 174, 247-250
- **HENIKOFF et al.** *Proc. Natl. Acad. Sci.*, 1992, vol. 89, 10915-10919
- **GOSSEN et al.** *Ann. Rev. Genetics*, 2002, vol. 36, 153-173
- Handbook of Pharmaceutical Excipients. 2012

- Mass Spectrometry of Proteins and Peptides. Methods in Molecular Biology. Humana Press, 2000, vol. 146
- **BIEMANN**. *Methods Enzymol*, 1990, vol. 193, 455-79
- Biological Mass Spectrometry. Methods in Enzymology. Academic Press, 2005, vol. 402
- **KUHN et al.** *Proteomics*, 2004, vol. 4, 1175-86
- **BIDLINGMEYER, B. A.** Practical HPLC Methodology and Applications. John Wiley & Sons Inc., 1993
- **VINCKE et al.** Generation of single domain antibody fragments derived from camelids and generation of manifold constructs.. *Methods Mol Biol.*, 2012, vol. 907, 145-76
- **PARDON et al.** *Nat. Protoc*, 2014
- **SKERRA, A** ; **PLÜCKTHUN**. *Science*, 1988
- **SERISIER et al.** *Respir Res*, 2009, vol. 10 (1), 63
- **CLARKE et al.** *Sciences*, 1992, vol. 257 (5073), 1125-8
- **L AGENTE et al.** American Thoracic Society International Conference Abstracts. American Thoracic Society, 2009, vol. A5668
- **MALL et al.** *Nat. Med*, 2004, vol. 10, 487-493